# EUROPEAN PATENT APPLICATION

(11) **EP 4 467 547 A1**
(43) Date of publication of application: **27.11.2024**
(21) Application number: 23740222.7
(22) Date of filing: 06.01.2023
(51) Int. Cl.: C07D 497/04, C09K 9/02, G02C 7/10, C07D 311/60, C07D 311/78

(54) **PHOTOCHROMIC COMPOUND, NAPHTHOL DERIVATIVE, CURABLE COMPOSITION, OPTICAL ARTICLE, LENS, AND EYEGLASSES**

(30) Priority: 17.01.2022 JP 2022005343
(71) Applicant: TOKUYAMA CORPORATION, Yamaguchi 745-8648 (JP)
(72) Inventor: MIYAZAKI, Masayuki, Shunan-shi, Yamaguchi 745-8648 (JP); VENU, Srinivas, Shunan-shi, Yamaguchi 745-8648 (JP); MORI, Katsuhiro, Shunan-shi, Yamaguchi 745-8648 (JP)
(74) Representative: Papula Oy
(86) International application number: PCT/JP2023/000163
(87) International publication number: WO 2023/136213

(57) **Abstract**

The purpose of the present invention is to provide: a photochromic compound that has excellent temperature dependence, decoloration speed, and durability; a naphthol derivative that can be an intermediate of the photochromic compound; a naphthol derivative that can be an intermediate of the photochromic compound; a curable composition that contains the photochromic compound; an optical article; a lens; and eyeglasses.An embodiment of the present invention provides a photochromic compound having a skeleton represented by formula (1). In formula (1), M is C, Si, or Ge. R¹ is a group represented by formula (2). R² is an alkyl group that may contain a halogen atom in a substituent, or a group represented by formula (2).

## Description

### TECHNICAL FIELD

The present invention relates to a photochromic compound, a naphthol derivative, a curable composition, an optical article, a lens, and eyeglasses.

### BACKGROUND ART

Photochromic compounds are compounds that can reversibly take two isomeric forms with different absorption spectra upon irradiation with light including ultraviolet light such as sunlight or light from a mercury vapor lamp. In general, a compound in a colorless or decolored state quickly changes in color and is isomerized (chromogenic reaction) to a colored or color-developed state by irradiation with ultraviolet light. The photochromic compounds have been studied and developed as materials for photochromic lenses.

In applications of such photochromic lenses, the photochromic compounds may require the following properties:
(I) a degree of coloration in a visible light range before irradiation with ultraviolet light (hereinafter referred to as initial coloration) is low;
(II) a color developing density (hereinafter referred to as color developing density) quickly reaches a saturation level from the beginning of irradiation with ultraviolet light;
(III) a color developing density quickly reaches a saturation level from the beginning of irradiation with ultraviolet light (hereinafter also referred to as high color developing sensitivity);
(IV) a color quickly returns to its original state after irradiation with ultraviolet light is stopped (hereinafter referred to as color fading rate);
(V) repetition durability of the above-mentioned reversible action is high; and
(VI) solubility in a material that makes up a matrix of a lens is high and dispersibility within a cured product is high.

A number of chromene compounds have been studied as photochromic compounds that satisfy these properties. For example, a chromene compound represented by Formula (A) below (Patent Document 1), a chromene compound represented by Formula (B) below (Patent Document 2), and a chromene compound represented by Formula (C) below (Patent Document 3) have been known.

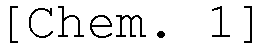

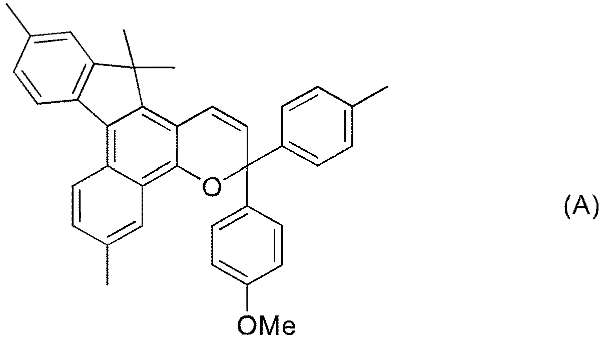

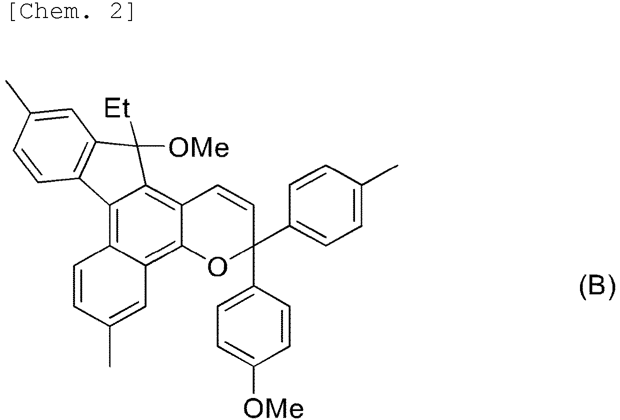

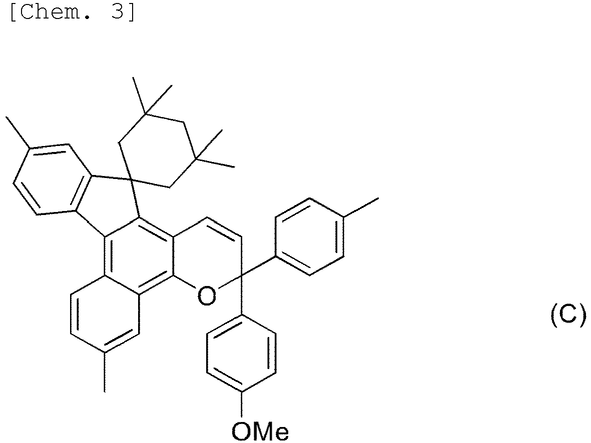

T-type photochromic compounds are photochromic compounds that exhibit a color fading reaction not only by light at a specific wavelength but also by heat when being isomerized from a color-developed state to a decolored state (fading reaction).

For the T-type photochromic compounds, in addition to the above-mentioned properties (I) to (VI), there may be a need for performance that exhibits a high color developing density even under a high temperature such as in summer. Hereinafter, the property of having a high color developing density even under a high temperature may also be referred to as "low temperature dependence".

### Citation List

### Patent Document

Patent Document 1: PCT International Publication No. WO1996/014596
Patent Document 2: PCT International Publication No. WO2004/085568
Patent Document 3: PCT International Publication No. WO2001/060811
Patent Document 4: PCT International Publication No. WO2015/035325
Patent Document 5: PCT International Publication No. WO2018/235771
Patent Document 6: PCT International Publication No. WO2012/121414
Patent Document 7: PCT International Publication No. WO2006/110221
Patent Document 8: PCT International Publication No. WO2012/121414
Patent Document 9: PCT International Publication No. WO2000/015630
Patent Document 10: PCT International Publication No. WO2011/053615
Patent Document 11: PCT International Publication No. WO2006/110219

### Non-Patent Document

Non-Patent Document 1: Journal of Organic Chemistry 69(10) 3282-3293; 2004
Non-Patent Document 2: Synthetic Communications 23(16) 2241-2249 (1993)
Non-Patent Document 3: J. Am. Chem. Soc. 132(41) 14324-14326 (2010)
Non-Patent Document 4: Org. Lett. 16, 6492-6495 (2014)

### DISCLOSURE OF THE INVENTION

### Problems to be Solved by the Invention

An object of the present invention is to provide a photochromic compound with excellent temperature dependence, color fading rate, and durability; a naphthol derivative that can be an intermediate of the photochromic compound; and a curable composition, an optical article, a lens, and eyeglasses including the photochromic compound.

### Means for Solving the Problems

According to an embodiment, there is provided a photochromic compound having a backbone represented by Formula (1) below:

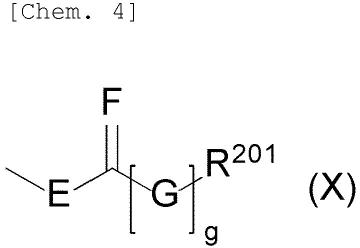

In Formula (1), M is C, Si, or Ge. R¹ is a group represented by Formula (2) below. R² is a group represented by Formula (2) or an alkyl group optionally including a halogen atom as a substituent. Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. Ring A may not be present. Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings.

-Q¹-(X¹Q²)a-X²Q³ (2)

In Formula (2), Q¹ is an alkylene group optionally including a halogen atom as a substituent. Q² is an alkylene group optionally including a halogen atom as a substituent. Q³ is an alkyl group optionally including a halogen atom as a substituent. X¹ and X² are each independently O, S, NR⁷⁰⁰, PR⁷⁰¹, or P(=O). R⁷⁰⁰ is a hydrogen atom, an optionally substituted alkyl group, an optionally substituted cycloalkyl group, an optionally substituted aryl group, or an optionally substituted heteroaryl group. R⁷⁰¹ is a hydrogen atom, an optionally substituted alkyl group, an optionally substituted cycloalkyl group, an optionally substituted aryl group, or an optionally substituted heteroaryl group.
a is 0, or 1 or more and 10 or less.

According to another embodiment, a curable composition is provided. The curable composition includes a photochromic compound according to the embodiment; and at least one selected from the group consisting of a radical polymerizable monomer, a cationic polymerizable monomer, a compound having a polymerization reactive group, and a (thio)urethane(urea) polymer.

According to another embodiment, an optical article is provided. The optical article includes a cured product of the curable composition.

According to another embodiment, a lens is provided. The lens includes a photochromic compound according to the embodiment.

According to another embodiment, eyeglasses are provided. The eyeglasses include the lens according to the embodiment.

According to another embodiment, a naphthol derivative is provided. The naphthol derivative has a backbone represented by Formula (6a) below:

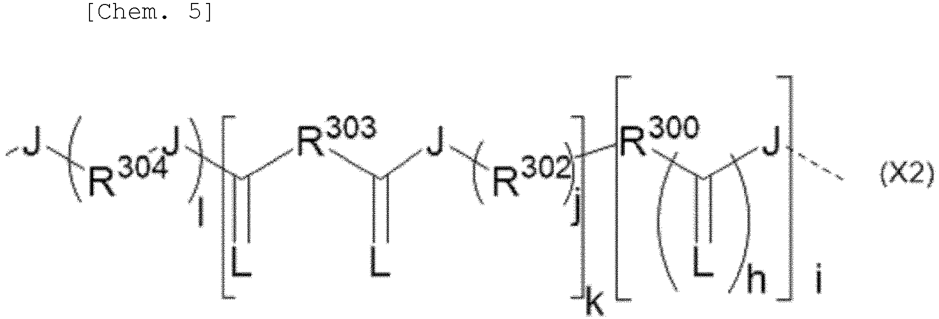

In Formula (6a), M, R¹, and R² are each independently the same as in Formula (1) above.

### Effects of the Invention

According to the present invention, there is provided a photochromic compound that can combine both a color developing density under a high temperature and a fast color fading rate and also has excellent durability; a naphthol derivative that can be an intermediate of the photochromic compound; and a curable composition, an optical article, a lens, and eyeglasses including the photochromic compound.

### BRIEF DESCRIPTION OF THE DRAWINGS

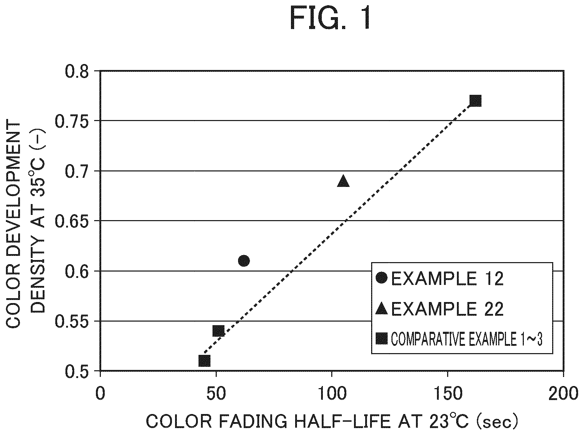

FIG. 1 is a graph showing an example of a relationship between a color fading half-life at 23°C and a color developing density at 35°C of photochromic laminates according to Examples and Comparative Examples.

### PREFERRED MODE FOR CARRYING OUT THE INVENTION

### [Photochromic compound]

According to an embodiment, there is provided a photochromic compound having a backbone represented by Formula (1) below:

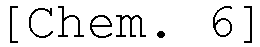

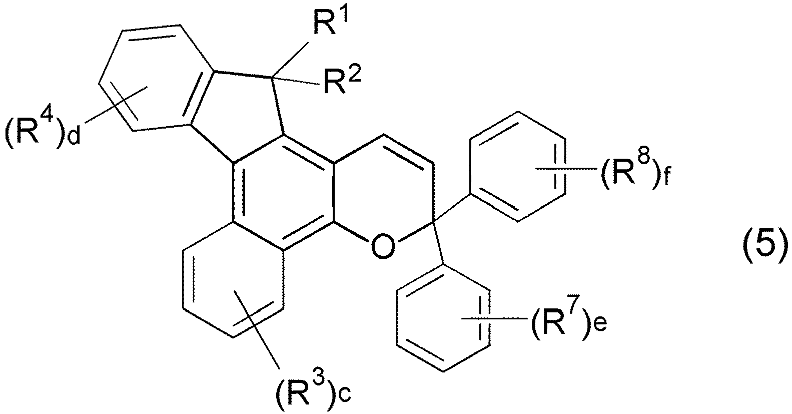

In Formula (1), M is C, Si, or Ge. R¹ is a group represented by Formula (2) below. R² is a group represented by Formula (2) or an alkyl group optionally including a halogen atom as a substituent. Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. Ring A may not be present. Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings.

-Q¹-(X²Q²)a-X²Q³ (2)

In Formula (2), Q¹ is an alkylene group optionally including a halogen atom as a substituent. Q² is an alkylene group optionally including a halogen atom as a substituent. Q³ is an alkyl group optionally including a halogen atom as a substituent. X¹ and X² are each independently O, S, NR⁷⁰⁰, PR⁷⁰¹, or P(=O). R⁷⁰⁰ is a hydrogen atom, an optionally substituted alkyl group, an optionally substituted cycloalkyl group, an optionally substituted aryl group, or an optionally substituted heteroaryl group. R⁷⁰¹ is a hydrogen atom, an optionally substituted alkyl group, an optionally substituted cycloalkyl group, an optionally substituted aryl group, or an optionally substituted heteroaryl group.
a is 0, or 1 or more and 10 or less.

This photochromic compound has excellent temperature dependence, color fading rate, and durability. The reasons for this are believed to be as follows. First, the inventors have found that a substituent attached to an atom at position 13 in a photochromic compound having a backbone represented by Formula (1) has a significant effect on the temperature dependence and the color fading rate. It is R¹ and R² that are attached to an atom M at position 13 of a photochromic compound according to the embodiment. R¹ is a group represented by Formula (2). R² is a group represented by Formula (2) or an alkyl group optionally including a halogen atom as a substituent;
In other words, in the photochromic compound according to the embodiment, R¹ and R² are each a group represented by Formula (2), or a combination of a group represented by Formula (2) and an alkyl group.

It is believed that, when R¹ and R² each have structures, electron repulsion is caused between them to increase a color fading rate of the photochromic compound. In other words, the group represented by Formula (2) includes an atom having one or more unshared electron pairs (X¹ and optionally X²). When R¹ and R² both have the structure of Formula (2), the atoms having an unshared electron pair included in the groups represented by Formula (2) are considered to repel each other. The atoms having an unshared electron pair may also repel a substituent constituting another backbone such as a substituent on a carbon atom at position 3. Therefore, a photochromic compound having a group represented by Formula (2) according to the embodiment is more susceptible to steric hindrance and structural change to a colorless isomer due to electron repulsion between the atoms having an unshared electron pair compared to a photochromic compound in which R¹ and R² are each an alkyl group
Thus, the photochromic compound according to the embodiment can exhibit a high color fading rate while achieving low temperature dependence.

Furthermore, as a result of extensive studies, the present inventors have also found that durability of a photochromic compound is increased by introducing substituents R¹ and R² into an atom M at position 13 without an intervening oxygen atom (O). In other words, in a photochromic compound according to the embodiment, an alkylene group Q¹ or an alkyl group R² is directly attached to an atom M at position 13, and no oxygen atom is attached thereto. Therefore, the photochromic compound according to the embodiment is more durable compared to a photochromic compound having a substituent introduced via an oxygen atom such as an alkoxy group.

In light of the above, the photochromic compound according to the embodiments can be used to achieve a cured product with excellent temperature dependence, color fading rate, and durability. Therefore, such a photochromic compound is suitable for an optical article to be used in an environment with a large temperature variation such as sunglasses.

A photochromic compound having a backbone represented by Formula (1) will be described in detail.

### <M>

In Formula (1), M is C, Si, or Ge. M is preferably C.

### <R2>

-Q¹-(X²Q²)a-X²Q³ (2)

In Formula (2), Q¹ is an alkylene group optionally including a halogen atom as a substituent. The alkylene group has preferably 1 or more and 20 or less carbon atoms, more preferably 1 or more and 12 or less carbon atoms, further preferably 1 or more and 7 or less carbon atoms, and most preferably 2 or more and 6 or less carbon atoms. An alkylene group having a small number of carbon atoms tends to increase a color developing density under a high temperature. An alkylene group having a large number of carbon atoms tends to increase a color fading rate. Q¹ may be a linear alkylene group or a branched alkylene group. Q¹ is preferably a linear alkylene group. At least one halogen atom selected from the group consisting of I, Cl, Br, and F may be used. The halogen atom is preferably at least one of C1 or F and more preferably F. Q¹ binds to an atom M at position 13 in Formula (1).

Q² is an alkylene group optionally including a halogen atom as a substituent. A preferred aspect of a number of carbon atoms in the alkylene group and the halogen atom are the same as for Q¹. The number of carbon atoms in the alkylene group for Q² may be the same as or different from that of the alkylene group for Q¹.

Q³ is an alkyl group optionally including a halogen atom as a substituent. The alkyl group has preferably 1 or more and 20 or less carbon atoms, more preferably 1 or more and 12 or less carbon atoms, and most preferably 1 or more and 7 or less carbon atoms. Q³ may be a linear alkyl group or a branched alkyl group. Q³ is preferably a linear alkyl group.

X¹ and X² are each independently O, S, NR⁷⁰⁰, PR⁷⁰¹, or P (=O). X¹ and X² are each preferably O, S, or NR⁷⁰⁰, more preferably O or S, and most preferably O. R⁷⁰⁰ is a hydrogen atom, an optionally substituted alkyl group, an optionally substituted cycloalkyl group, an optionally substituted aryl group, or an optionally substituted heteroaryl group. R⁷⁰⁰ is preferably a hydrogen atom, an optionally substituted alkyl group, or an optionally substituted aryl group. The alkyl group is preferably a methyl group, an ethyl group, a propyl group, a butyl group, or a pentyl group. The aryl group is preferably a phenyl group or a naphthyl group. R⁷⁰¹ is a hydrogen atom, an optionally substituted alkyl group, an optionally substituted cycloalkyl group, an optionally substituted aryl group, or an optionally substituted heteroaryl group. R⁷⁰¹ is preferably a hydrogen atom, an optionally substituted alkyl group, or an optionally substituted aryl group. The alkyl group is preferably a methyl group, an ethyl group, a propyl group, a butyl group, or a pentyl group. The aryl group is preferably a phenyl group or a naphthyl group.

Formula (2) is preferably an alkylenealkoxy group or an alkyleneoxyalkylenealkoxy group.

Specific examples of Formula (2) include -CH₂OCH₃, - CH₂SCH₃, -CH₂CH₂OCH₃, -CH₂CH₂OCH₂CH₃, -CH₂CH₂SCH₃, -CH₂CH₂CH₂OCH₃, - CH₂CH₂CH₂SCH₃, -CH₂CH₂OCH₂CH₂OCH₃, or -CH₂CH₂OCH₂CH₂OCH₂CH₃.

R¹ may be a group represented by Formula (2a) below:

-Q¹¹-OQ¹³ (2a)

In Formula (2a), Q¹¹ is a linear alkylene group having 1 or more and 10 or less carbon atoms. Q¹¹ has preferably 2 or more and 5 or less carbon atoms. Having the number of carbon atoms falling within this range tends to decrease temperature dependence and increase a color fading rate. Q¹³ is a linear alkyl group having 1 or more and 15 or less carbon atoms. Q¹³ has preferably 2 or more and 12 or less carbon atoms. Having the number of carbon atoms falling within this range tends to decrease temperature dependence and increase a color fading rate.

R¹ may be a group represented by Formula (2b) below:

-Q²¹-(OCH₂CH₂)ₐ₁-OQ²³ (2b)

In Formula (2b), Q²¹ is a linear alkylene group having 1 or more and 5 or less carbon atoms. Q²¹ has preferably 2 or more and 5 or less carbon atoms. Having the number of carbon atoms falling within this range tends to decrease temperature dependence and increase a color fading rate. Q²³ is a linear alkyl group having 1 or more and 5 or less carbon atoms. Q²³ may be a linear alkyl group having 2 or more and 4 or less carbon atoms.
a1 means a number of repeats of an ethylene oxide group.
a1 is 1 or more and 10 or less.
a1 may be 2 or more, 3 or more, or 5 or more. Having a large number of repeats of an ethylene oxide group tends to decrease temperature dependence and increase a color fading rate.

### <R2>

R² is a group represented by Formula (2) or an alkyl group optionally including a halogen atom as a substituent; R² is preferably a group represented by Formula (2). When R² is a group represented by Formula (2), steric hindrance easily occurs and a color fading rate tends to increase. When R² is a group represented by Formula (2), it may have a structure that is the same as or different from that for R¹. R² may be a group represented by Formula (2a) or a group represented by Formula (2b).

The alkyl group optionally including a halogen atom may have preferably 1 or more and 20 or less carbon atoms, more preferably 1 or more and 12 or less carbon atoms, and most preferably 1 or more and 7 or less carbon atoms.

### <Ring A>

In Formula (1), Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. Furthermore, Ring A is not essential and may not be present.

Examples of the aromatic hydrocarbon ring include a benzene ring or a cyclotetradecaheptaene ring.

Examples of the aromatic heterocyclic ring include a furan ring, a thiophene ring, or a pyridine ring.

Examples of the fused polycyclic ring include a naphthalene ring, a fluorene ring, an anthracene ring, a phenanthrene ring, a tetracene ring, a pentacene ring, a benzopyrene ring, a chrysene ring, a pyrene ring, a triphenylene ring, a perylene ring, a benzofuran ring, a benzothiophene ring, a quinoline ring, an isoquinoline ring, an indole ring, a pyrimidine ring, a quinazoline ring, a pyridazine ring, a cinnoline ring, a phthalazine ring, a 1,2,3-, 1,2,4-, or 1,3,5-triazine ring, a carbazole ring, a benzoxazole ring, or an isothiazole ring.

Among them, a benzene ring, a naphthalene ring, a fluorene ring, a phenanthrene ring, a pyrene ring, a furan ring, a thiophene ring, or a pyridine ring is preferred and a benzene ring, a naphthalene ring, a fluorene ring, or a phenanthrene ring is most preferred.

### <Ring B>

In Formula (1), Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings. With regard to a suitable ring B, the same applies as for Ring A.

### <Compound represented by Formula (3)>

A photochromic compound according to the embodiment preferably has a naphthopyran backbone represented by Formula (3) below. In Formula (3) below, R¹, R², and M are each the same as in Formula (1).

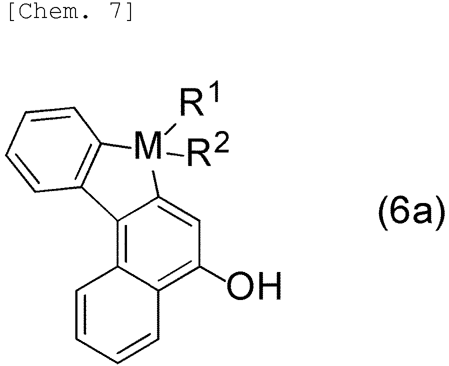

### <Compound represented by Formula (4)>

A photochromic compound according to the embodiment is preferably a compound represented by Formula (4) below. In Formula (4) below, R¹, R², and M are each the same as in Formula (1).

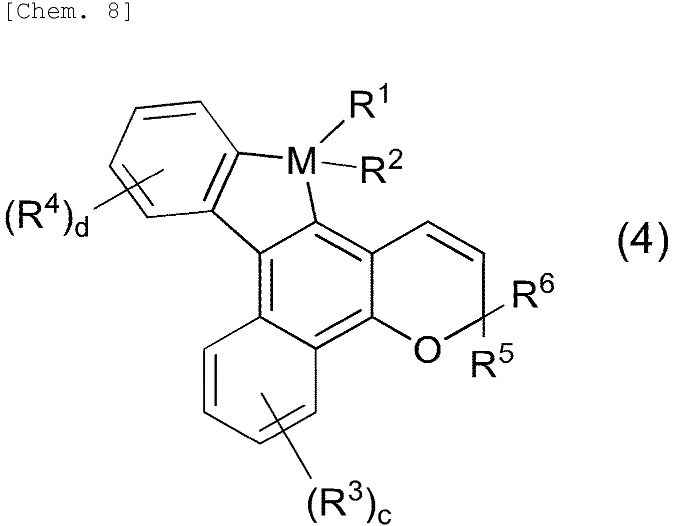

### <R³ and R⁴>

R³ and R⁴ are each independently a group represented by Formula (2), a hydroxyl group, an alkyl group, a haloalkyl group, an optionally substituted cycloalkyl group, an alkoxy group, an amino group, a substituted amino group, an optionally substituted heterocyclic group, a cyano group, a halogen atom, an alkylthio group, an optionally substituted arylthio group, a nitro group, a formyl group, a hydroxycarbonyl group, an alkylcarbonyl group, an alkoxycarbonyl group, an optionally substituted aralkyl group, an optionally substituted aralkoxy group, an optionally substituted aryloxy group, an optionally substituted aryl group, an optionally substituted heteroaryl group, a thiol group, an alkoxyalkylthio group, a haloalkylthio group, or an optionally substituted cycloalkylthio group, an optionally substituted silyl group, an optionally substituted oxysilyl group, a group represented by Formula (X) below, or a group represented by L¹-R⁴⁰⁰ below.

c is an integer of 0 to 4.

d is an integer of 0 to 4.

When c is 2 to 4, a plurality of R³s may be the same as or different from each other. When c is 2 to 4 and adjacent R³s are present, two adjacent R³s may be taken together with a carbon atom to which R³s are attached to form a ring optionally including at least one atom selected from the group consisting of an oxygen atom, a carbon atom, a sulfur atom, and a nitrogen atom and the ring is optionally substituted. Examples of a combination of the adjacent R³s include R³s at positions 5 and 6, positions 6 and 7, or positions 7 and 8 of the chromene compound.

When d is 2 to 4, a plurality of R⁴s may be the same as or different from each other. When d is 2 to 4 and adjacent R⁴s are present, two adjacent R⁴s may be taken together with a carbon atom to which R⁴s are attached to form a ring optionally including at least one atom selected from the group consisting of an oxygen atom, a carbon atom, a sulfur atom, and a nitrogen atom and the ring is optionally substituted. Examples of a combination of the adjacent R⁴s include R⁴s at positions 9 and 10, positions 10 and 11, or positions 11 and 12 of the chromene compound.

A ring having 5 to 8 atoms including a carbon atom to which R³ and R⁴ are attached is preferably formed. The ring may have a substituent and the substituent may be a substituent selected from a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 carbon atoms, a cyano group, a nitro group, or a halogen atom. Specific examples of the substituent will be described below.

A suitable ring includes a ring represented by Formula (X4) below.

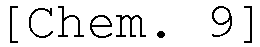

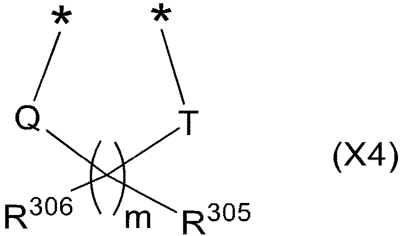

In Formula (X4), Q and T are each independently a sulfur atom, a substituted methylene group, an oxygen atom, or a group represented by NR³⁰⁷. R³⁰⁷ is a hydrogen atom, a hydroxyl group, an alkyl group, a haloalkyl group, a cycloalkyl group, an alkoxy group, an optionally substituted aryl group, an optionally substituted heteroaryl group, or a group represented by Formula (2).

R³⁰⁵ and R³⁰⁶ are each independently preferably a hydroxy group, an alkyl group, a haloalkyl group, a cycloalkyl group, an alkoxy group, an amino group, a substituted amino group, an optionally substituted heterocyclic group, a cyano group, a nitro group, a formyl group, a hydroxycarbonyl group, an alkylcarbonyl group, an alkoxycarbonyl group, a halogen atom, an optionally substituted aralkyl group, an optionally substituted aralkoxy group, an optionally substituted aryl group, a thiol group, an alkylthio group, an alkoxyalkylthio group, a haloalkylthio group, a cycloalkylthio group, or an optionally substituted arylthio group.

In addition, R³⁰⁵ and R³⁰⁶ may be taken together with a carbon atom to which they are attached to form an aliphatic ring that may have a substituent. Specific examples of the aliphatic ring include a cyclopentane ring, a cyclohexane ring, or the like. The substituent on the aliphatic ring is not particularly limited, but examples include a substituent selected from a hydroxyl group, an alkyl group, a haloalkyl group, a cycloalkyl group, an alkoxy group, an amino group, a substituted amino group, a heterocyclic group, a cyano group, a nitro group, or a halogen atom, in which 1 to 8 hydrogen atoms, especially preferably 1 to 4 hydrogen atoms in a group forming the ring are substituted with the substituent. Specific examples of the substituent will be described below.

In the formula above, m is an integer of 1 to 4.

### <Group represented by Formula (X)>

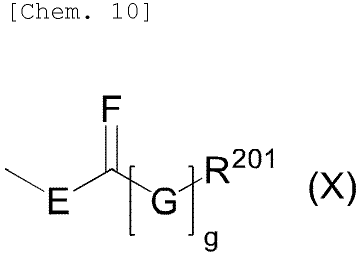

In Formula (X), E is an oxygen atom or NR¹⁰¹. R¹⁰¹ is a hydrogen atom or an alkyl group. E is NR¹⁰¹ in which R¹⁰¹ is preferably a hydrogen atom or an alkyl group having 1 to 6 carbon atoms.

F is an oxygen atom or a sulfur atom. F is preferably an oxygen atom.

G is an oxygen atom, a sulfur atom, or NR²⁰². R²⁰² is a hydrogen atom, an alkyl group, a cycloalkyl group, an aryl group, or a heteroaryl group. G is preferably NH.

g is 0 or 1.

R²⁰¹ is a hydrogen atom, an alkyl group, a cycloalkyl group, an aryl group, or a heteroaryl group. When G is an oxygen atom or a sulfur atom, R²⁰¹ is a group other than a hydrogen atom. R²⁰¹ is preferably an alkyl group having 1 to 6 carbon atoms, an alkoxy group having 1 to 6 carbon atoms, or an aryl group having 6 to 12 carbon atoms.

A suitable group represented by Formula (X) is as follows:

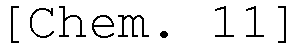

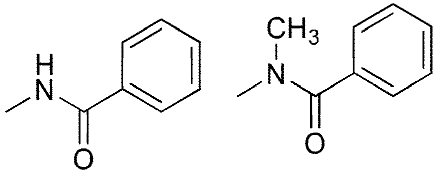

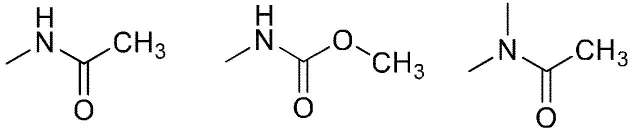

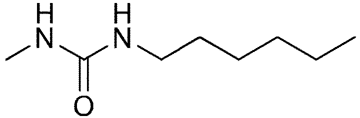

### <Group represented by L¹-R⁴⁰⁰>

In L¹-R⁴⁰⁰, R⁴⁰⁰ is a hydrogen atom, an alkyl group, an aryl group, a polymerizable group, a photochromic group, or a silyl group having an alkyl group, an alkoxyl group, or an aryl group as a substituent.

L¹ is a group represented by Formula (X2) below:

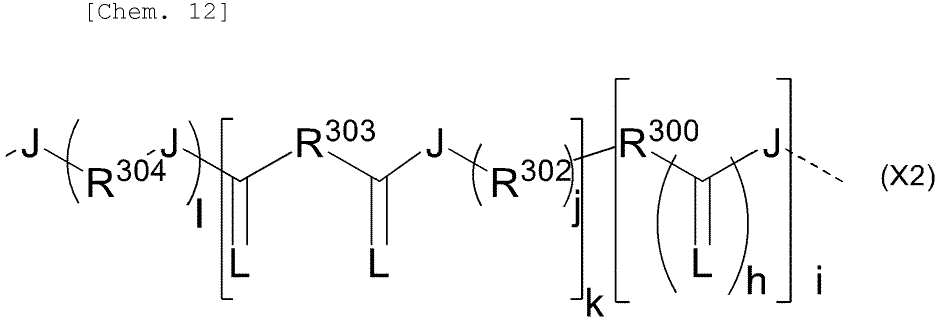

In Formula (X2), J is a divalent group. A plurality of Js are each independently directly attached, a substituted methylene group, an oxygen atom, a sulfur atom, or NR³⁰¹. R is a hydrogen atom or an alkyl group. R³⁰¹ is preferably an alkyl group having 1 to 20 carbon atoms. The alkyl group preferably has a silyl group having an alkyl group having 1 to 10 carbon atoms, a polymerizable group, or a photochromic group as a substituent.

Examples of the polymerizable group include a radical polymerizable group such as a vinyl group, a 1-chlorovinyl group, an allyl group, a styryl group, a (meth)acryl group, a 2-(methacryloxy)ethylcarbamyl group, a 2-(methacryloxy)ethoxycarbonyl group, or a crotyl group. Other than the above-mentioned groups, an epoxy group, an episulfide group, a thietanyl group, an OH group, an SH group, an NH₂ group, a COOH group, an NCO group, or an NCS group may also be used. The polymerizable group is preferably at least one selected from the group consisting of a (meth)acrylic group, a 2-(methacryloxy)ethylcarbamyl group, a 2-(methacryloxy)ethoxycarbonyl group, an epoxy group, an OH group, an SH group, an NH₂ group, and a COOH group.

As a representative example, the photochromic group is exemplified by naphthopyran, spirooxazine, spiropyran, fulgide, fulgimide, or diarylethene. Indenonaphthopyran is preferred from the viewpoint of its ability to develop an excellent photochromic property, and indeno[2,1-f]naphtho[1,2-b]pyran is particularly preferred.

Indeno[2,1-f]naphtho[1,2-b]pyran is preferably a group represented by Formula (X3) below:

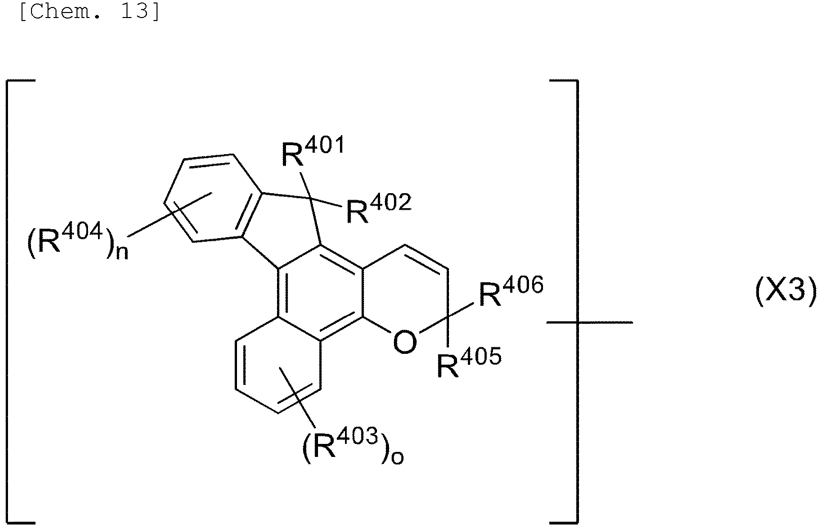

In Formula (X3), R⁴⁰¹ and R⁴⁰² are each independently of L¹, a hydrogen atom, a hydroxyl group, an alkyl group, a haloalkyl group, a cycloalkyl group, an alkoxy group, an alkoxyalkyl group, a formyl group, a hydroxycarbonyl group, an alkylcarbonyl group, an alkoxycarbonyl group, a halogen atom, an optionally substituted aralkyl group, an optionally substituted aralkoxy group, an optionally substituted aryloxy group, an optionally substituted aryl group, or a group represented by Formula (2).

R⁴⁰¹ and R⁴⁰² may be taken together with a carbon atom at position 13 to which they are attached to form an aliphatic ring having 3 to 20 ring member carbon atoms, a fused polycyclic ring in which an aromatic ring or an aromatic heterocyclic ring is fused to the above-mentioned aliphatic ring, a heterocyclic ring having 3 to 20 ring member atoms, or a fused polycyclic ring in which an aromatic ring or aromatic heterocyclic ring is fused to the above-mentioned heterocyclic ring. These rings may each have a substituent.

R⁴⁰³ and R⁴⁰⁴ are each independently L¹, a hydroxyl group, an alkyl group, a haloalkyl group, an optionally substituted cycloalkyl group, an alkoxy group, an amino group (including a primary or secondary amine group), a (optionally substituted) heterocyclic group having a nitrogen atom as a ring member and attached via the nitrogen atom to a carbon atom in an indenonaphthopyran backbone, a cyano group, a nitro group, a formyl group, a hydroxycarbonyl group, an alkylcarbonyl group, an alkoxycarbonyl group, a halogen atom, an optionally substituted aralkyl group, an optionally substituted aralkoxy group, an optionally substituted aryloxy group, an optionally substituted aryl group, an alkylthio group, a cycloalkylthio group, an optionally substituted arylthio group, or the group represented by Formula (2) above.

For R⁴⁰³ and R⁴⁰⁴, each independently, adjacent two may be taken together to form an (optionally substituted) aliphatic ring optionally including at least one selected from the group consisting of an oxygen atom, a nitrogen atom, and a sulfur atom.

R⁴⁰⁵ and R⁴⁰⁶ are each independently an optionally substituted aryl group or an optionally substituted heteroaryl group.

o is an integer of 0 to 4.

n is an integer of 0 to 4.

When o is 2 to 4, a plurality of R⁴⁰³s may be the same as or different from each other.

When n is 2 to 4, a plurality of R⁴⁰⁴s may be the same as or different from each other.

At least one of substituents on the aryl group or the heteroaryl group for R⁴⁰¹, R⁴⁰², R⁴⁰³, R⁴⁰⁴, and R⁴⁰⁵ is attached to L¹.

A particularly suitable group represented by Formula (X2) is represented by any of the following formulae:

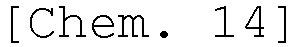

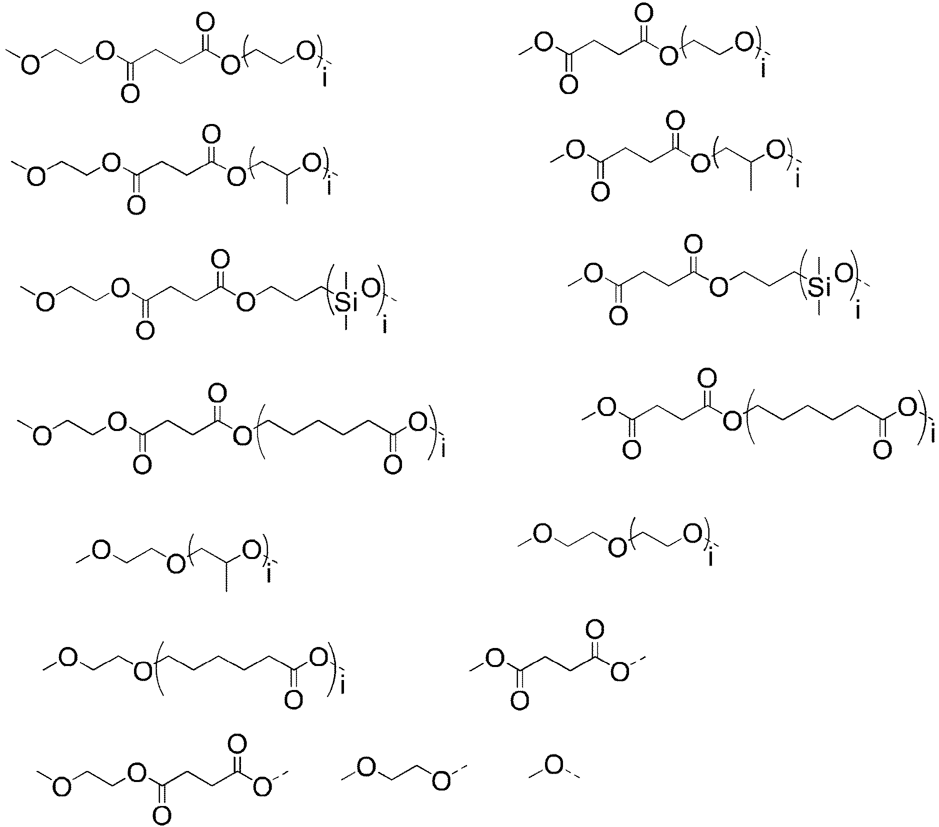

In Formula (X2), L is an oxygen atom or a sulfur atom.

R³⁰⁰ is an alkylene group, or a silylene group having an alkyl group or an aryl group as a substituent. R³⁰⁰ is preferably an alkylene group having 1 to 6 carbon atoms or a silylene group having an alkyl group having 1 to 6 carbon atoms as a substituent.

R³⁰², R³⁰³, and R³⁰⁴ are each independently an alkylene group. R³⁰² is preferably an alkylene group having 1 to 6 carbon atoms. R³⁰³ is preferably an alkylene group having 1 to 6 carbon atoms. R³⁰⁴ is preferably an alkylene group having 1 to 6 carbon atoms.

h, j, k, and l are each independently an integer of 0 or 1.

i is an integer of 1 to 200. A plurality of units i may be the same as or different from each other.
i is preferably 5 to 100, more preferably 8 to 75, and most preferably 10 to 70.

A dashed line represents an attachment to R⁴⁰⁰.

### <R⁵ and R⁶>

In Formula (4), R⁵ and R⁶ are each independently an optionally substituted aryl group or an optionally substituted heteroaryl group.

R⁵ and R⁶ are each independently an optionally substituted phenyl group, an optionally substituted 1-naphthyl group, an optionally substituted 2-naphthyl group, an optionally substituted thienyl group, an optionally substituted furyl group, an optionally substituted pyrrolinyl group, an optionally substituted pyridyl group, an optionally substituted benzothienyl group, an optionally substituted benzofuranyl group, or an optionally substituted benzopyrrolinyl group. Furthermore, it is preferred that at least one of R⁵ or R⁶ be an optionally substituted phenyl group, and it is more preferred that R⁵ and R⁶ both be substituted phenyl groups.

A substituent that the phenyl group has is preferably a group represented by Formula (2), a hydroxyl group, an alkyl group, a haloalkyl group, an optionally substituted cycloalkyl group, an alkoxy group, an amino group, a substituted amino group, an optionally substituted heterocyclic group, a cyano group, a halogen atom, an alkylthio group, an optionally substituted arylthio group, a nitro group, a formyl group, a hydroxycarbonyl group, an alkylcarbonyl group, an alkoxycarbonyl group, an optionally substituted aralkyl group, an optionally substituted aralkoxy group, an optionally substituted aryloxy group, an optionally substituted aryl group, an optionally substituted heteroaryl group, a thiol group, an alkoxyalkylthio group, a haloalkylthio group, or an optionally substituted cycloalkylthio group, an optionally substituted silyl group, an optionally substituted oxysilyl group, the group represented by Formula (X), or the group represented by L¹-R⁴⁰⁰. The substituent is more preferably an alkyl group, a haloalkyl group, an alkoxy group, an amino group, a substituted amino group, an optionally substituted heterocyclic group, a cyano group, a halogen atom, an alkylthio group, an optionally substituted arylthio group, an optionally substituted aryloxy group, an optionally substituted aryl group, an optionally substituted heteroaryl group, an alkoxyalkylthio group, a haloalkylthio group, an optionally substituted silyl group, an optionally substituted oxysilyl group, or the group represented by L¹-R⁴⁰⁰.

### <Detailed description of R³, R⁴, R⁵, R⁶, and substituents>

R³, R⁴, R⁵, R⁶, and substituents that they may include will be described in detail below. These substituents can be used as substituents that can be included in R⁷⁰⁰ or R⁷⁰¹.

An alkyl group is not particularly limited, but is preferably an alkyl group having 1 to 6 carbon atoms. Suitable examples of the alkyl group having 1 to 6 carbon atoms include a methyl group, an ethyl group, an n-propyl group, an isopropyl group, an n-butyl group, a sec-butyl group, a tert-butyl group, a pentyl group, or a hexyl group.

A haloalkyl group is not particularly limited, but is preferably a haloalkyl group having 1 to 6 carbon atoms. The haloalkyl group having 1 to 6 carbon atoms is preferably an alkyl group substituted with a fluorine atom, a chlorine atom, or a bromine atom. Suitable examples of the haloalkyl group may include a trifluoromethyl group, a tetrafluoroethyl group, a chloromethyl group, a 2-chloroethyl group, or a bromomethyl group.

A cycloalkyl group is not particularly limited, but is preferably a cycloalkyl group having 3 to 8 carbon atoms (cycloalkyl group in which 3 to 8 carbon atoms form a ring). Examples of the cycloalkyl group having 3 to 8 carbon atoms may include a cyclopropyl group, a cyclobutyl group, a cyclopentyl group, or a cyclohexyl group. Note that, the cycloalkyl group may have a substituent, but a number of carbon atoms (3 to 8 carbon atoms) shall not include a number of carbon atoms on the substituent.

An alkoxy group is not particularly limited, but is preferably an alkoxy group having 1 to 6 carbon atoms. Suitable examples of the alkoxy group having 1 to 6 carbon atoms include a methoxy group, an ethoxy group, an n-propoxy group, an isopropoxy group, an n-butoxy group, a sec-butoxy group, or a tert-butoxy group.

An amino group is a primary amino group (-NH₂). A substituted amino group is a secondary or tertiary amino group with one or two hydrogen atoms being substituted. A substituent that the substituted amino group has is not particularly limited, but include an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, or a heteroaryl group having 4 to 14 carbon atoms. Suitable examples of the amino group include an amino group, a methylamino group, a dimethylamino group, an ethylamino group, a diethylamino group, a phenylamino group, or a diphenylamino group.

A heterocyclic group preferably has 3 to 10 atoms. Specifically, for example, an aliphatic heterocyclic group such as a morpholino group, a piperidino group, a pyrrolidinyl group, a piperazino group, or an N-methylpiperazino group; or an aromatic heterocyclic group such as an indolinyl group can be used. Furthermore, the heterocyclic ring may have a substituent. The substituent is preferably an alkyl group having 1 to 6 carbon atoms. Suitable examples of a heterocyclic group having a substituent include a 2,6-dimethylmorpholino group, a 2,6-dimethylpiperidino group, or a 2,2,6,6-tetramethylpiperidino group.

Examples of a halogen atom include a fluorine atom, a chlorine atom, a bromine atom, or an iodine atom.

An alkylthio group is not particularly limited, but is preferably an alkylthio group having 1 to 6 carbon atoms. The alkylthio group having 1 to 6 carbon atoms may include a methylthio group, an ethylthio group, an n-propylthio group, an isopropylthio group, an n-butylthio group, a sec-butylthio group, or a t-butylthio group.

An arylthio group is not particularly limited, but is preferably an arylthio group having 6 to 10 carbon atoms. The arylthio group having 6 to 10 carbon atoms may include a phenylthio group, a 1-naphthylthio group, or a 2-naphthylthio group.

An alkylcarbonyl group is not particularly limited, but is preferably an alkylcarbonyl group having 2 to 7 carbon atoms. The alkylcarbonyl group having 2 to 7 carbon atoms may include an acetyl group or an ethylcarbonyl group.

An alkoxycarbonyl group is not particularly limited, but is preferably an alkoxycarbonyl group having 2 to 7 carbon atoms. The alkoxycarbonyl group having 2 to 7 carbon atoms may include a methoxycarbonyl group or an ethoxycarbonyl group.

An aralkyl group is not particularly limited, but is preferably an aralkyl group having 7 to 11 carbon atoms. The aralkyl group having 7 to 11 carbon atoms may include a benzyl group, a phenylethyl group, a phenylpropyl group, a phenylbutyl group, or a naphthylmethyl group.

An aralkoxy group is not particularly limited, but is preferably an aralkoxy group having 7 to 11 carbon atoms. The aralkoxy group having 7 to 11 carbon atoms may include a benzyloxy group or a naphthylmethoxy group.

An aryloxy group is not particularly limited, but is preferably an aryloxy group having 6 to 12 carbon atoms. The aryloxy group having 6 to 12 carbon atoms may include a phenyloxy group or a naphthyloxy group.

An aryl group is not particularly limited, but is preferably an aryl group having 6 to 12 carbon atoms. The aryl group having 6 to 12 carbon atoms may include a phenyl group, a 1-naphthyl group, or a 2-naphthyl group.

A heteroaryl group is not particularly limited, but is preferably a heteroaryl group having 3 to 12 carbon atoms. The heteroaryl group having 3 to 12 carbon atoms may include a thienyl group, a furyl group, a pyrrolinyl group, a pyridyl group, a benzothienyl group, a benzofuranyl group, or a benzopyrrolinyl group.

An alkoxyalkylthio group is not particularly limited, but is preferably an alkoxyalkylthio group having 2 to 9 carbon atoms. The alkoxyalkylthio group having 2 to 9 carbon atoms may include a methoxymethylthio group, a methoxyethylthio group, a methoxy n-propylthio group, a methoxy n-butylthio group, an ethoxytehylthio group, or an n-propoxypropylthio group.

A haloalkylthio group is not particularly limited, but is preferably a haloalkylthio group having 1 to 6 carbon atoms. The haloalkylthio group having 1 to 6 carbon atoms may include a trifluoromethylthio group, a tetrafluoroethylthio group, a chloromethylthio group, a 2-chloroethylthio group, or a bromomethylthio group.

A cycloalkylthio group is not particularly limited, but is preferably a cycloalkylthio group having 3 to 8 carbon atoms. The cycloalkylthio group having 3 to 8 carbon atoms may include a cyclopropylthio group, a cyclobutylthio group, a cyclopentylthio group, or a cyclohexylthio group. Note that, the cycloalkylthio group may have a substituent, but a number of carbon atoms (3 to 8 carbon atoms) shall not include a number of carbon atoms on the substituent.

A silyl group may have a substituent. A substituent that a substituted silyl group has is not particularly limited, but includes an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, or a heteroaryl group having 4 to 14 carbon atoms.

An oxysilyl group may have a substituent. A substituent that a substituted oxysilyl group has is not particularly limited, but includes an alkyl group having 1 to 6 carbon atoms, a haloalkyl group having 1 to 6 carbon atoms, an alkoxy group having 1 to 6 carbon atoms, a cycloalkyl group having 3 to 7 carbon atoms, an aryl group having 6 to 14 carbon atoms, or a heteroaryl group having 4 to 14 carbon atoms.

Note that, the cycloalkyl group, the arylthio group, the aralkyl group, the aralkoxy group, the aryloxy group, the aryl group, the heteroaryl group, and the cycloalkylthio group may be unsubstituted. However, when the above-mentioned groups are substituted, 1 to 8 hydrogen atoms, especially preferably 1 to 4 hydrogen atoms in a ring-forming group are preferably substituted by a substituent selected from a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 atoms, a cyano group, a nitro group, or a halogen atom. Specific examples of these substituents are the same groups as described above.

### <Photochromic compound represented by Formula (5)>

A particularly suitable chromene compound includes a compound represented by Formula (5) below:

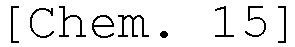

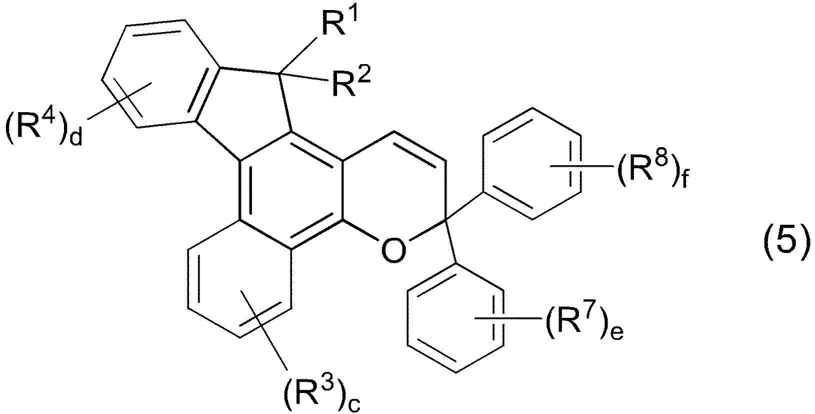

In Formula (5), R¹, R², R³, R⁴, c, and d are the same as in Formula (4).

### <R⁷ and R⁸>

R⁷ and R⁸ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, an optionally substituted arylthio group having 6 to 10 carbon atoms, or L¹-R⁴⁰⁰.

e denotes a number of R⁷ and an integer of 0 to 5. When e is 2 or more, R⁷s may be the same as or different from each other.

When e is 2 to 5 and adjacent R⁷s are present, two adjacent R⁷s may be taken together with a carbon atom to which R⁷s are attached to form a ring optionally including at least one atom selected from the group consisting of an oxygen atom, a sulfur atom, a carbon atom, and a nitrogen atom and the ring is optionally substituted. Note that, the ring can also simultaneously have two or more atoms selected from the group consisting of an oxygen atom, a sulfur atom, a carbon atom, or a nitrogen atom.

f denotes a number of R⁸ and an integer of 0 to 5. When f is 2 or more, R⁸s may be the same as or different from each other.

When f is 2 to 5 and adjacent R⁸s are present, two adjacent R⁸s may be taken together with a carbon atom to which R⁸s are attached to form a ring optionally including at least one atom selected from the group consisting of an oxygen atom, a sulfur atom, a carbon atom, and a nitrogen atom and the ring is optionally substituted. Note that, the ring can also simultaneously have two or more atoms selected from the group consisting of an oxygen atom, a sulfur atom, a carbon atom, or a nitrogen atom.

For R⁷ and R⁸, each independently, adjacent two may be taken together to form a ring group optionally including at least one selected from the group consisting of an oxygen atom, a sulfur atom, a carbon atom, and a nitrogen atom. This ring group is not particularly limited, but is preferably a ring having 5 to 8 atoms including a carbon atom to which R⁷ and R⁸ are attached. The ring may have a substituent. The substituent may be a substituent selected from a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a substituted amino group, a heterocyclic group having 3 to 8 carbon atoms, a cyano group, a nitro group, or a halogen atom. Specific examples of these substituents are the same groups as described above. For the ring, it is preferred that a plurality of the rings be taken together to form the ring represented by Formula (X4).

### <Specific example of suitable photochromic compound>

Specific examples of a particularly suitable photochromic compound include photochromic compounds represented by the following formulae:

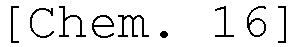

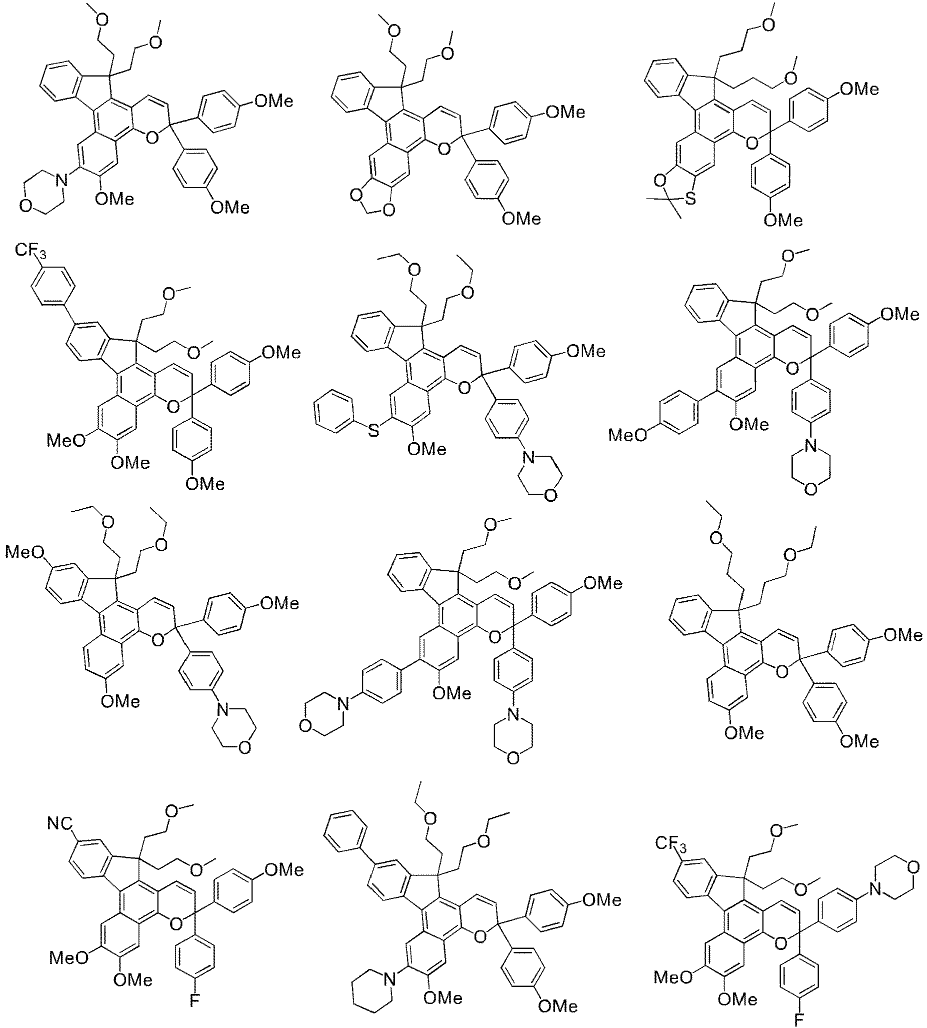

### [Naphthol derivative]

A naphthol derivative according to the embodiment has a backbone represented by Formula (6a) below. The naphthol derivative can be used as an intermediate for synthesizing a photochromic compound having a backbone represented by Formula (3) above.

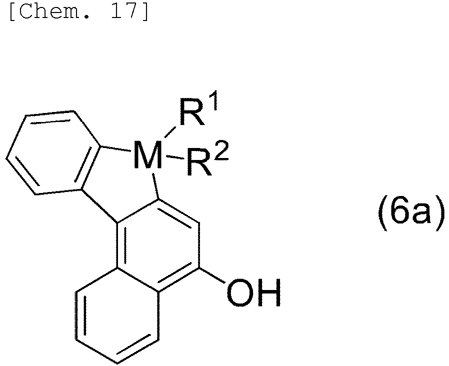

In Formula (6a), M, R¹, and R² are each independently the same as in Formula (3) above.

### <Naphthol derivative represented by Formula (6)>

A naphthol derivative represented by Formula (6) can be used as an intermediate for synthesizing a naphthol derivative represented by Formula (4) or (5) above.

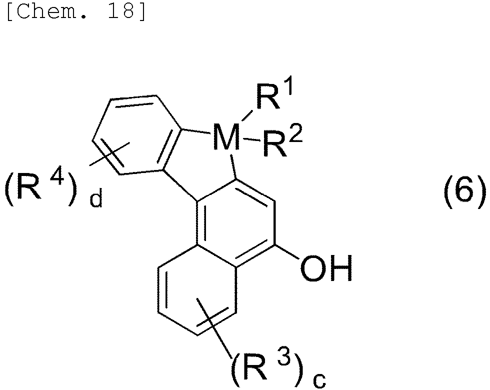

In Formula (6), M, R¹, and R² are each independently the same as in Formula (1) above. R³, R⁴, c, and d are each independently the same as in Formula (4) above.

### <Specific examples of naphthol derivative>

Specific examples of a naphthol derivative according to the embodiment include compounds represented by the following formulae:

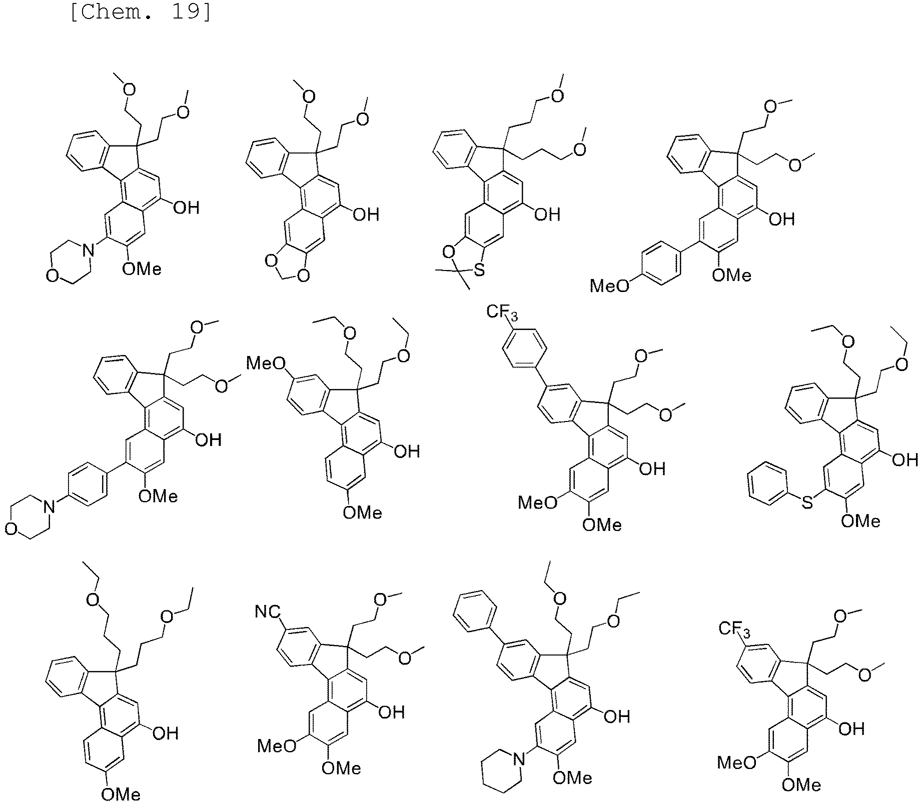

### [Method for producing photochromic compound]

A photochromic compound according to the embodiment may be produced by any synthetic method. Representative examples of a method for producing a photochromic compound will be described, but the method is not limited thereto. Note that, in the following description, a symbol in each formula has the same meaning as described for any of the above-mentioned formulae, unless otherwise noted.

A photochromic compound may be produced by a method in which the naphthol derivative represented by Formula (6) above and
a propargyl alcohol compound represented by Formula (7) below: 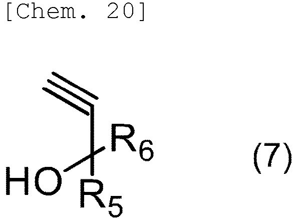 are reacted in the presence of an acid catalyst.

A reaction ratio of a naphthol compound to the propargyl alcohol compound is preferably selected from a range of 1:10 to 10:1 (molar ratio). For example, sulfuric acid, benzene sulfonic acid, p-toluenesulfonic acid, acidic alumina, or the like are used as the acid catalyst. The acid catalyst is preferably used in a range of 0.1 to 10 parts by weight per 100 parts by weight of a total of the naphthol compound and the propargyl alcohol compound. A reaction temperature is preferably 0 to 200°C. A solvent is preferably a non-protic organic solvent such as N-methylpyrrolidone, dimethylformamide, tetrahydrofuran, benzene, toluene, or the like. A method for purifying a product obtained from such a reaction is not particularly limited. For example, the product can be purified by silica gel column purification and then recrystallization.

### <Method for synthesizing naphthol derivative>

A naphthol derivative can be synthesized based on any of reaction methods described in, for example, Non-Patent Document 1, Non-Patent Document 2, or Patent Document 3.

A method for synthesizing the naphthol compound represented by Formula (6) above is not particularly limited. For example, when M is a carbon atom, the naphthol compound can be synthesized as follows.

First, a benzene compound represented by Formula (8) below is reacted with an acid chloride represented by Formula (9) below to obtain a compound represented by Formula (10) below.

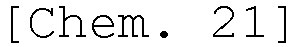

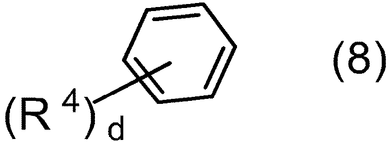

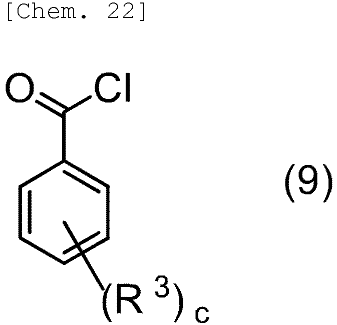

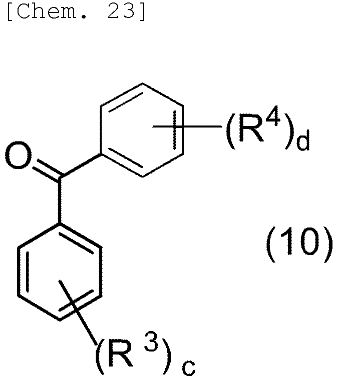

Note that, in Formula (10) above, R3, R⁴, c, and d are the same as in Formula (4) above.

Furthermore, the compound (10) is subjected to a Stobbe reaction, a cyclization reaction, a hydrolysis reaction using an alkali or acid, benzyl protection, or debenzylation by a hydrolysis reaction using an alkali or an acid to obtain a carboxylic acid of which hydroxyl group is protected with benzyl (Bn) as represented by Formula (11) below.

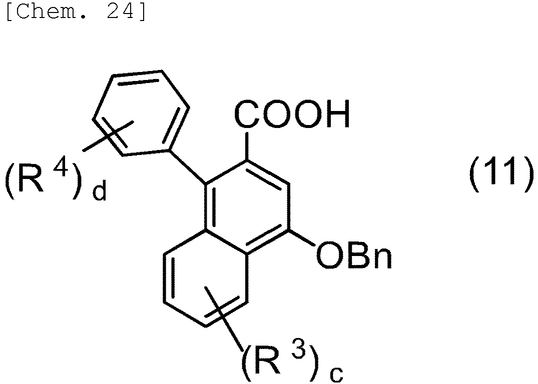

The thus-benzyl-protected carboxylic acid represented by Formula (11) above is then converted to an amine by Curtius rearrangement, Hofmann rearrangement, Lessen rearrangement, or the like, and a diazonium salt is prepared from the amine. The diazonium salt is converted to a halide such as a bromide or an iodide by a Sandmeyer reaction, or the like to obtain a halide represented by Formula (12) below in which Hal represents a halogen.

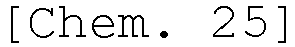

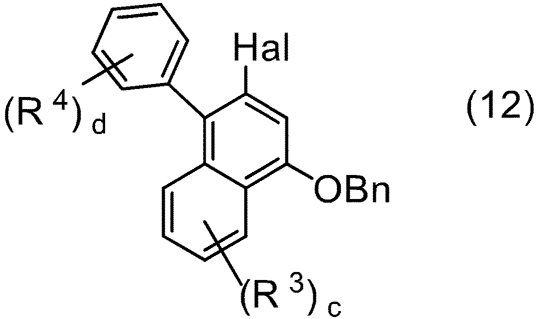

The thus-obtained halide is reacted with magnesium, lithium, or the like to prepare an organometallic reagent. This organometallic reagent is reacted with a ketone represented by Formula (13) below in which R¹ and R² are the same as in Formula (4) above in an organic solvent at -100 to 70°C to obtain a compound represented by Formula (14) below.

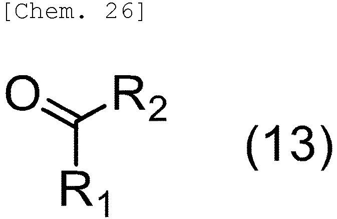

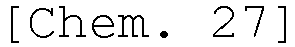

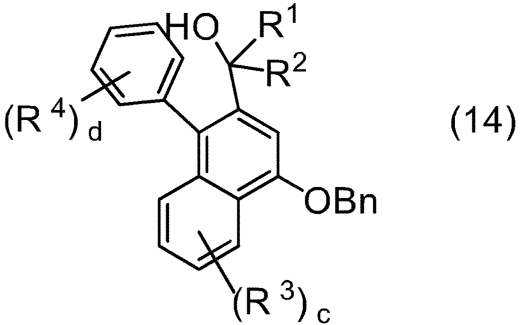

The thus-obtained compound (14) is debenzylated and then reacted under a neutral to acidic condition at 10 to 120°C for 10 minutes to 2 hours to thereby convert alcohol into its spiro form. Thus, a desired naphthol derivative represented by Formula (6) above can be synthesized. In such a reaction, a reaction ratio of the organometallic reagent to the ketone represented by Formula (13) may be selected from a wide range, but preferably from a range of 1:10 to 10:1 (molar ratio). A reaction temperature is preferably -100 to 70°C. A non-protic organic solvent such as diethyl ether, tetrahydrofuran, benzene, or toluene is preferably used as the solvent. The conversion of the alcohol into its spiro form under a neutral to an acidic condition is preferably performed in the presence of an acid catalyst. For example, acetic acid, hydrochloric acid, sulfuric acid, benzene sulfonic acid, p-toluenesulfonic acid, acidic alumina, or the like is used as the acid catalyst. Such an acid catalyst is suitably used in a range of 0.1 to 10 parts by weight per 100 parts by weight of the alcohol. The alcohol is preferably converted into its spiro form in the presence of a solvent such as tetrahydrofuran, benzene, or toluene.

The above-mentioned method is suitably used for synthesizing a photochromic compound in which R¹ and R² are different from each other. The above-mentioned method is also suitably used when R¹ and R² are the same as each other, but another method can also be used. Another method alternative to the above-mentioned method will be described below.

First, a carboxylic acid compound represented by Formula (15) below, which is obtained by subjecting the compound of Formula (10) to a Stobbe reaction, a cyclization reaction, or a hydrolysis reaction using an alkali or acid, is converted into its spiro form under a neutral to acidic condition and reduced using hydrogen or hydrazine to obtain a naphthol derivative represented by Formula (16) below.

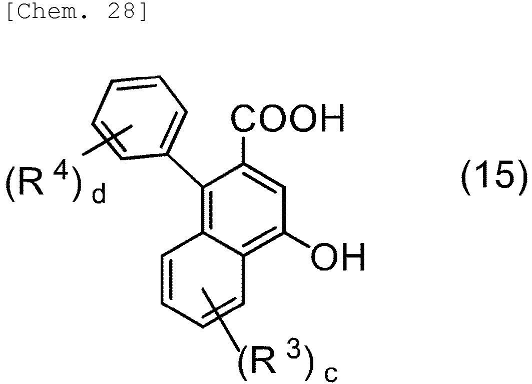

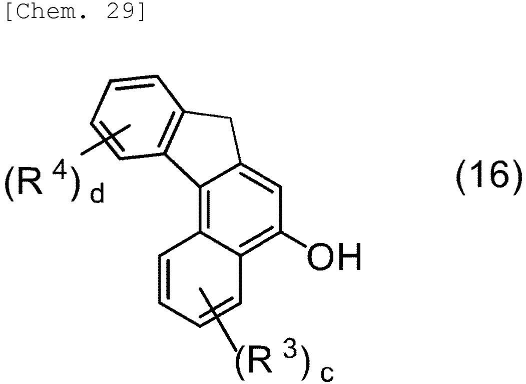

A hydroxyl group in the thus-obtained naphthol derivative is protected by benzyl protection, acetal protection, silyl protection, or the like to obtain a compound of Formula (17) below in which OPro denotes a protected hydroxyl group.

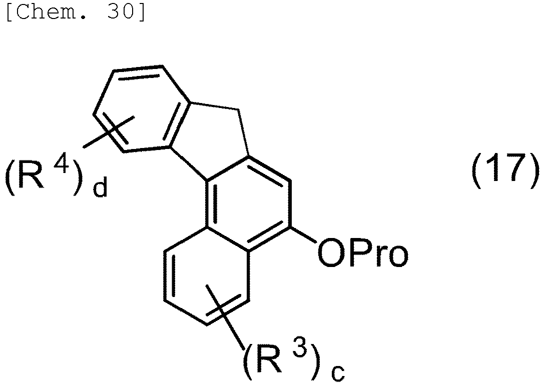

The compound can be reacted with a halide of R¹ under a basic condition, followed by deprotection to obtain a compound represented by Formula (18) below, which is a compound represented by Formula (6) above in which R¹ = R².

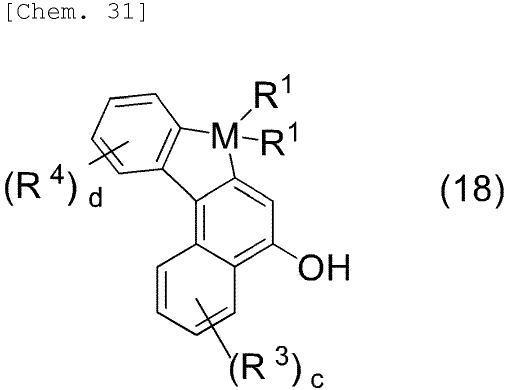

Another method alternative to the above-mentioned method, the compound represented by Formula (17) can also be synthesized by intramolecular cyclization of a halogen compound of Formula (13) above in the presence of a palladium catalyst.

### <Method for synthesizing naphthol derivative including Si or Ge>

One exemplary method for producing a naphthol derivative represented by Formula (6) in which M is Si or Ge will be described.

First, the halide represented by Formula (12) is reacted with magnesium, lithium, or the like to prepare an organometallic reagent. This organometallic reagent is reacted with a monohalide represented by Formula (19) below in which R¹ and R² are the same as in Formula (4) above in an organic solvent at -100 to 70°C to obtain a compound represented by Formula (20) below.

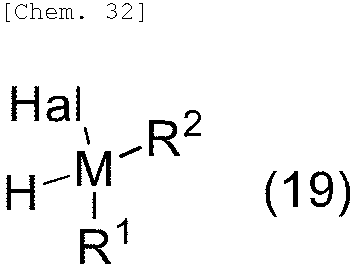

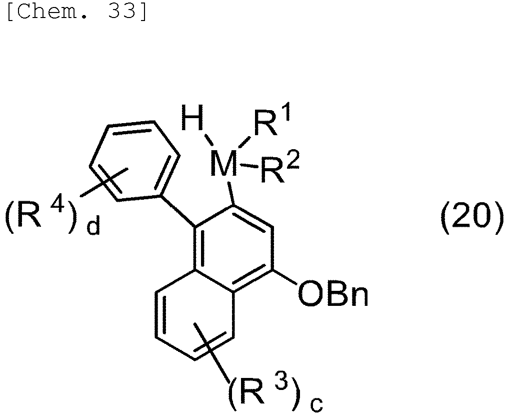

The thus-obtained compound (20) is cyclized to obtain a cyclized form represented by Formula (21) below with reference to any of reaction methods described in Non-Patent Document 3, Non-Patent Document 4, Patent Document 4, or the like.

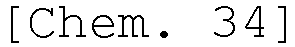

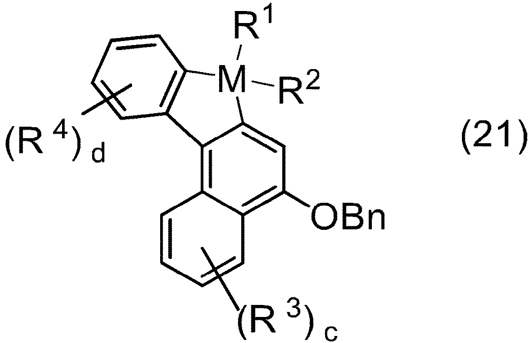

The thus-obtained cyclized form can be debenzylated to obtain the naphthol derivative of Formula (6).

### <Identification of photochromic compound>

A photochromic compound according to the embodiment is, for example, a solid or viscous liquid at room temperature and normal pressure. A photochromic compound may be isolated from this solid or liquid by separation operations such as thin layer chromatography, silica gel column chromatography, high performance liquid chromatography, gas chromatography, or the like. The absence of a byproduct such as a raw material compound or a coloring matter other than the photochromic compound is confirmed.

The thus-obtained photochromic compound is characterized by proton nuclear magnetic resonance spectroscopy (¹H-NMR) and a peak based on an aromatic proton and an alkene proton appears around δ: 5.0 to 9.0 ppm and a peak based on protons of an alkyl group and an alkylene group appears around δ: 1.0 to 4.0 ppm. By relatively comparing spectral intensities of the peaks, a number of protons on each attached group can be determined. This allows identification of a backbone, a substituent, or the like that the photochromic compound has.

When the photochromic compound is included in a cured product such as a resin, the photochromic compound can be isolated by dissolving the resin and using the above-mentioned separation operations.

### <Photochromic composition>

A photochromic compound according to the embodiment can be dissolved in a common organic solvent such as toluene, chloroform, or tetrahydrofuran. When a photochromic compound having a backbone represented by Formula (1) is dissolved in such a solvent, a clear, colorless solution is obtained. This solution exhibits good photochromic behavior, that is, when the solution is irradiated with sunlight or ultraviolet light, it quickly develops a color and when the sunlight or the like is blocked, it quickly reversibly returns to its original colorless state.

The photochromic compound according to the embodiment can be used in combination with a photochromic compound having another structure, depending on the intended use. For example, it can be used in combination with another photochromic compound in order to obtain various color tones required for a photochromic lens. A photochromic compound to be combined may be any known compound without any limitation. Examples thereof include indenonaphthopyran, naphthopyran, spirooxazine, spiropyran, fulgide, fulgimide, or diarylethene. Among them, an indenonaphthopyran compound is particularly preferred since it can maintain a uniform color tone upon color development and fading, can suppress a color shift upon color development associated with degradation of a photochromic property, and can reduce initial coloration. It is preferred to use a plurality of photochromic compounds to adjust a color tone since they can achieve both a color density under a high temperature and a fast color fading rate and also give excellent durability.

When a photochromic composition including the photochromic compound according to the embodiment and another photochromic compound is produced, a blending ratio of the photochromic compounds is appropriately determined in accordance with a desired color tone.

### <Photochromic curable composition>

A curable composition according to the embodiment includes a photochromic compound according to the embodiment; and at least one selected from the group consisting of a radical polymerizable monomer, a cationic polymerizable monomer, a compound having a polymerization reactive group, and a (thio)urethane(urea) polymer. Here, the (thio)urethane(urea) polymer includes at least one selected from the group consisting of a urethane polymer, a thiourethane polymer, a urethaneurea polymer, and a thiourethaneurea polymer.

The photochromic compound according to the embodiment and the photochromic composition are preferably used in combination with a polymerizable compound as a photochromic curable composition.

For the photochromic curable composition, although it all depends on a color developing intensity of a photochromic compound, a lens material selected, and a thickness of a lens, the photochromic compound (or photochromic composition) is preferably used in an amount of 0.001 to 10 parts by mass relative to 100 parts by mass of the polymerizable compound. An optimal amount to be incorporated depends on application for which the photochromic curable composition is used. For example, the case where the photochromic curable composition is used as a thin-film optical article or as a thick-film optical article will be described below.

### (Use as thin-film optical article)

For example, when a thin film of 10 um or more and less than 1000 µm, e.g., about 100 um (polymer film made by polymerization of the photochromic curable composition) is formed from the photochromic curable composition, 0.001 to 10 parts by mass of a photochromic compound (or photochromic composition) relative to 100 parts by mass of another polymerizable monomer may be used to adjust a color tone.

### (Use as thick film optical article)

In the case of a thick cured product (polymeric molded product made by polymerization of the photochromic curable composition), e.g., a cured product having a thickness of 1 mm or more, 0.001 to 1 part by mass of a photochromic compound (or photochromic composition) of the present invention relative to 100 parts by mass of the thick cured product or another polymerizable monomer that gives the thick cured product may be used to adjust a color tone.

### <Polymerizable compound>

As mentioned above, a photochromic compound is preferably used as a photochromic curable composition in combination with a polymerizable compound. Examples of the polymerizable compound may include a urethane- or urea-based polymerizable compound that can form a urethane bond or a urea bond, a compound having a polymerization reactive group, a radical polymerizable compound, an epoxy-based polymerizable compound, or the like. These polymerizable compounds are not particularly limited, but, for example, the polymerizable compound described in Patent Document 5 may be suitably used.

Among them, the below-mentioned polymerizable compounds are particularly suitably used.

### <Compound having polymerization reactive group>

An example of a compound having a polymerization reactive group includes a compound having an iso(thio)cyanate group. An iso(thio)cyanate compound is a compound having an isocyanate group or an isothiocyanate group and may include both an isocyanate group and an isothiocyanate group. This compound is suitable for use in combination with an active hydrogen-containing compound as described below. Examples of such an iso(thio)cyanate compound include, but are not limited to, the below-mentioned compounds.

### (Polyiso(thio)cyanate)

Polyiso(thio)cyanate is a compound having at least two or more iso(thio)cyanate groups per molecule. Examples of the polyiso(thio)cyanate include an aromatic polyiso(thio)cyanate having an aromatic ring such as m-xylene diisocyanate or 4,4'-diphenylmethane diisocyanate; or an aliphatic polyiso(thio)cyanate such as norbornane diisocyanate or dicyclohexylmethane-4,4'-diisocyanate.

### (Active hydrogen-containing compound)

An active hydrogen-containing compound is preferably a compound having a hydroxyl group and/or a thiol group and particularly preferably a polyfunctional compound having two or more active hydrogens per molecule, but is not limited thereto. Specific examples of the active hydrogen-containing compound may include a polyfunctional thiol compound such as pentaerythritol tetrakis(3-mercaptopropionate) or 4-mercaptomethyl-3,6-dithia-octanedithiol; or a polyfunctional alcohol such as trimethylolpropane or pentaerythritol.

### (Radical polymerizable compound)

A radical polymerizable compound includes a polyfunctional radical polymerizable compound and a monofunctional radical polymerizable compound. Each of these can be used alone or a plurality of them can be used in combination. Examples of a radical polymerizable substituent include a group having an unsaturated double bond, i.e., a vinyl group, a styryl group, a (meth)acryl group, an allyl group, or the like.

The polyfunctional radical polymerizable compound is a compound having two or more radical polymerizable substituents in a molecule. This polyfunctional radical polymerizable compound includes a first polyfunctional radical polymerizable compound having 2 to 10 radical polymerizable substituents and a second polyfunctional radical polymerizable compound having more than 10 radical polymerizable substituents.

The first radical polymerizable compound is not particularly limited, but more preferably has 2 to 6 radical polymerizable substituents. Specific examples thereof are as follows.

### (Polyfunctional (meth)acrylic acid ester compound)

Ethylene glycol di(meth)acrylate, diethylene glycol di(meth)acrylate, triethylene glycol di(meth)acrylate, tetraethylene glycol di(meth)acrylate, ethylene glycol bisglycidyl(meth)acrylate, bisphenol A di(meth)acrylate, 2,2-bis(4-(meth)acryloyloxyethoxyphenyl)propane, 2,2-bis(3,5-dibromo-4 -(meth)acryloyl, oxyethoxyphenyl)propane.

### (Polyfunctional allyl compound)

Diallyl phthalate, diallyl terephthalate, diallyl isophthalate, diallyl tartrate, diallyl epoxysuccinate, diallyl fumarate, diallyl chlorendate, diallyl hexaphthalate, diallyl carbonate, allyl diglycol carbonate, trimethylolpropane triallyl carbonate.

### (Polyfunctional thio(meth)acrylic acid ester compound)

1,2-bis(methacryloylthio)ethane, bis(2-acryloylthioethyl) ether, 1,4-bis(methacryloylthiomethyl)benzene.

### (Vinyl Compound)

### Divinylbenzene.

Examples of the second polyfunctional radical polymerizable compound having more than 10 radical polymerizable substituents include a compound having a relatively large molecular weight such as a silsesquioxane compound having a radical polymerizable substituent or a polyrotaxane compound having a radical polymerizable substituent.

The monofunctional radical polymerizable compound is a compound having one radical polymerizable substituent in a molecule, and specific examples thereof include, but are not limited to, the below-mentioned compounds.

### (Unsaturated carboxylic acid)

Acrylic acid, methacrylic acid, maleic anhydride.

### ((Meth)acrylic acid ester)

Methyl (meth)acrylate, benzyl methacrylate, phenyl methacrylate.

2-Hydroxyethyl methacrylate, glycidyl (meth)acrylate, β-methyl glycidyl (meth)acrylate, bisphenol A-monoglycidyl ether methacrylate, 4-glycidyloxy methacrylate, 3-(glycidyl-2-oxyethoxy)-2-hydroxy, propyl methacrylate, 3-(glycidyloxy-1-isopropyloxy)-2-hydroxypropyl acrylate, 3-glycidyloxy-2-hydroxypropyloxy)-2-hydroxypropyl acrylate.

### (Fumaric acid ester)

### Diethyl fumarate, diphenyl fumarate.

### (Thio(meth)acrylic acid)

Methyl thioacrylate, benzyl thioacrylate, benzyl thiomethacrylate.

### (Vinyl Compound)

Styrene, chlorostyrene, tylstyrene, vinylnaphthalene, α-methylstyrene dimer, bromostyrene.

The radical polymerizable compound may be used alone or a mixture of a plurality of types of them may also be used. In this case, preferably 80 to 100 parts by mass of the polyfunctional radical polymerizable compound and 0 to 20 parts by mass of the monofunctional radical polymerizable compound, and more preferably 90 to 100 parts by mass of the polyfunctional radical polymerizable compound and 0 to 10 parts by mass of the monofunctional radical polymerizable compound are used per 100 parts by mass of a total of the radical polymerizable compounds. Furthermore, preferably 80 to 100 parts by mass of the first polyfunctional radical polymerizable compound, 0 to 20 parts by mass of the second radical polymerizable compound, and 0 to 20 parts by mass of the monofunctional radical polymerizable compound, and further preferably 85 to 100 parts by mass of the first polyfunctional radical polymerizable compound, 0 to 10 parts by mass of the second polyfunctional radical polymerizable compound, and 0 to 10 parts by mass of the monofunctional radical polymerizable compound are used per 100 parts by mass of a total of the radical polymerizable compounds.

### (Various compounding agents)

Various known compounding agents may be incorporated into a curable composition as long as the effect is not impaired. Examples of the compounding agent include, for example, a releasing agent, a UV absorber, an IR absorber, a UV stabilizer, an antioxidant, an anti-coloring agent, an antistatic agent, a fluorescent dye, a dye, a pigment, a perfume, and various other stabilizers. A solvent or a leveling agent can also be incorporated. A thiol such as t-dodecyl mercaptan can be incorporated as a polymerization regulator.

Among the above-mentioned compounding agents, the UV stabilizer is suitably used from the viewpoint of improving durability of a photochromic moiety. A hindered amine light stabilizer, a hindered phenol antioxidant, a sulfur antioxidant, or the like are known as such a UV stabilizer. A particularly suitable UV stabilizer is as follows:

Bis(1,2,2,6,6-pentamethyl-4-piperidyl)sebacate; ADK STAB LA-52, LA-57, LA-62, LA-63, LA-67, LA-77, LA-82, LA-87 (manufactured by ADEKA CORPORATION); 2,6-di-tert-butyl-4-methyl-phenol, ethylene bis(oxyethylene) bis[3-(5-tert-butyl-4-hydroxy-m-tolyl)propionate]; IRGANOX 1010, 1035, 1075, 1098, 1135, 1141, 1222, 1330, 1425, 1520, 259, 3114, 3790, 5057, 565 (manufactured by BASF Japan). An amount of such a UV stabilizer to be used is not particularly limited unless the effect is impaired, but is usually in a range of 0.001 to 10 parts by mass, especially 0.01 to 1 part by mass per 100 parts by mass of a photochromic curable composition.

A UV absorber may also be used in addition to the UV stabilizer. Known UV absorbers such as a benzophenone compound, a benzotriazole compound, a cyanoacrylate compound, a triazine compound, or a benzoate compound can be used as the UV absorber, with a cyanoacrylate compound or a benzophenone compound being particularly preferred. The above-mentioned UV stabilizer is preferably used in a range of 0.001 to 5 parts by mass relative to 100 parts by mass of a photochromic curable composition including a photochromic compound and a polymerizable compound.

### <Method for using photochromic curable composition; optical article>

A photochromic curable composition is cured to obtain a photochromic cured product. Polymerization curing for producing the photochromic cured product is performed by radical polymerization, ring-opening polymerization, anionic polymerization, or condensation polymerization using irradiation with an active energy ray such as an ultraviolet ray, an α-ray, a β-ray, or a γ-ray; heat; or a combination thereof. In other words, an appropriate polymerization method may be employed depending on a type of a polymerizable compound or a polymerization accelerator and a form of a photochromic cured product to be formed.

When a curable composition containing a polymerizable compound is thermally polymerized, a temperature affects a property of the resulting photochromic cured product.

Such a temperature condition cannot be generally limited since it is affected by a type or amount of a thermal polymerization initiator or a type of polymerizable compound. However, in general, a method in which polymerization is started at a relatively low temperature, followed by heating slowly is suitable. Polymerization time also varies depending on various factors as is the case with the temperature. Therefore, although it is suitable to determine an optimum time in advance in accordance with these conditions, in general, the conditions are preferably selected so that polymerization is completed in 2 to 48 hours. When obtaining a photochromic laminated sheet, polymerization is performed at a temperature at which a reaction between polymerizable functional groups proceeds, and optimal temperature and time are preferably determined to achieve a desired molecular weight.

When a curable composition is photo-polymerized, among the polymerization conditions, especially UV intensity affects a property of the resulting photochromic cured product. Such an irradiation condition is affected by a type or amount of a photoinitiator or a type of a polymerizable monomer, so it cannot be generally limited, but in general, the conditions are preferably selected so as to give photoirradiation with 50 to 500 mW/cm² of UV light at a wavelength of 365 nm for 0.5 to 5 minutes.

### [Optical article]

A photochromic compound according to the embodiments can be widely used as a photochromic material, and can be used, for example, as various memory materials alternative to a silver halide photosensitive material, a reprographic material, a photosensitive material for printing, a memory material for a cathode ray tube, a photosensitive material for a laser, a photosensitive material for holography, and various other memory materials. The photochromic material can also be used as a material for a photochromic lens material, an optical filter material, a display material, a light meter, and a decoration material.

The photochromic compound according to the embodiment is particularly suitable for a photochromic lens application. A photochromic lens is suitable as a lens for eyeglasses such as sunglasses. Any known method for producing a photochromic lens can be employed, as long as the method provides uniform photochromic performance.

In the case where a photochromic property is developed by a kneading method, a photochromic cured product in a form of an optical material such as a lens may be obtained by injecting the above-mentioned curable composition between glass molds held with an elastomeric gasket or a spacer and then heating the curable composition in an air furnace or cast-polymerizing the curable composition by irradiation with an active energy ray such as ultraviolet ray depending on a type of a polymerizable compound or a polymerization accelerator.

In the case where a photochromic property is developed by a lamination method, a photochromic layer made of a photochromic cured product is formed on a surface of an optical substrate by dissolving a curable composition in an organic solvent as appropriate to prepare a coating solution; coating a surface of an optical substrate such as a lens substrate with the coating solution by spin coating, dipping, or the like; drying to remove the organic solvent; and then polymerization-curing the curable composition with UV irradiation, heating, or the like in an inert gas such as nitrogen (coating method).

Furthermore, a photochromic layer made of a photochromic cured product can also be formed on a surface of an optical substrate by casting polymerization using an inner mold in which an optical substrate such as a lens substrate is placed face-to-face in a glass mold so that a predetermined void is formed, the curable composition is injected into this void, and the curable composition is polymerization-cured in this state by UV irradiation, heating, or the like (casting polymerization method).

In the case where a photochromic layer is formed on a surface of an optical substrate by the lamination method (coating method and casting polymerization method) as described above, the surface of the optical substrate may be chemically treated with an alkali solution, an acid solution, or the like, or physically treated with corona discharge, plasma discharge, polishing, or the like in advance in order to enhance adhesion between the photochromic layer and the optical substrate. Of course, it is also possible to provide a transparent adhesive resin layer on the surface of the optical substrate.

In addition, when a photochromic property is developed by a binder method, a photochromic sheet is made by sheet-molding using a curable composition, sandwiched between two transparent sheets (optical sheets), and polymerization-cured as described above to obtain a photochromic laminate with a photochromic layer as an adhesive layer.

In this case, coating with a coating solution in which a curable composition is dissolved in an organic solvent can also be employed to produce a photochromic sheet.

The thus-produced photochromic laminate is, for example, mounted in a mold and then a thermoplastic resin (e.g., polycarbonate) for an optical substrate such as a lens is injection-molded thereon to obtain an optical substrate such as a lens with a photochromic property in a predetermined shape.

The photochromic laminate can also be adhered to a surface of an optical substrate with, for example, an adhesive to obtain a photochromic lens.

Note that, when a photochromic laminate is produced as described above, it is preferred to use, as a polymerizable compound, a urethane- or urea-based polymerizable compound, especially a urethane-based polymerizable compound in order to adjust so as to form polyurethane from the viewpoint of high adhesion especially to an optical substrate.

The above-mentioned curable composition can develop a photochromic property with an excellent color developing density at a high temperature.

A photochromic layer or a photochromic cured product formed from a curable composition can be subjected to post-processing such as staining using a dye such as a disperse dye; formation of a hard coat film using a silane coupling agent or a hard coat agent containing as a main component a sol of silicon, zirconium, antimony, aluminum, tin, tungsten, or the like; formation of a thin film by vapor deposition of a metal oxide such as SiO₂, TiO₂, ZrO₂, or the like; antireflection treatment by a thin film coated with an organic polymer; or antistatic treatment depending on its application.

### EXAMPLES

### (Example 1)

### First step

First, 20.0 g (43.1 mmol) of an iodine compound represented by Formula (22) below, 1.0 g (4.3 mmol) of palladium acetate, 42.3 g (431.0 mmol) of potassium acetate, 38.8 g (387.9 mmol) of potassium bicarbonate, 74.9 g (431.0 mmol) of dibromomethane, 260 mL of N,N-dimethylformamide (DMF), 86 mL of N,N-dimethylacetamide (DMAc), 4.2 mL of isopropyl alcohol (IPA), and 43 mL of water were added, heated, and then reacted at 80°C. After consumption of the iodine compound was confirmed, the resultant was allowed to cool to room temperature and then partitioned between 100 mL of water and 300 mL of ethyl acetate. A solvent was removed from the resulting organic layer, followed by purification by chromatography on silica gel to obtain a compound represented by Formula (23) below at a yield of 62%.

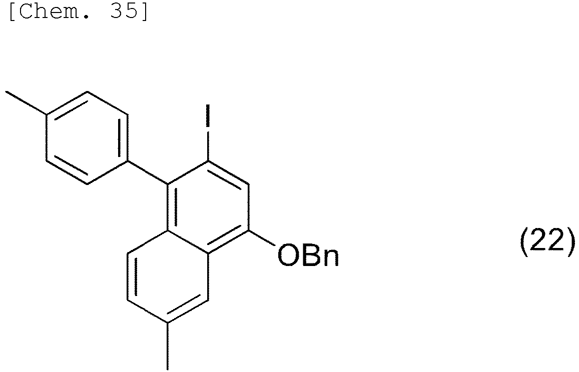

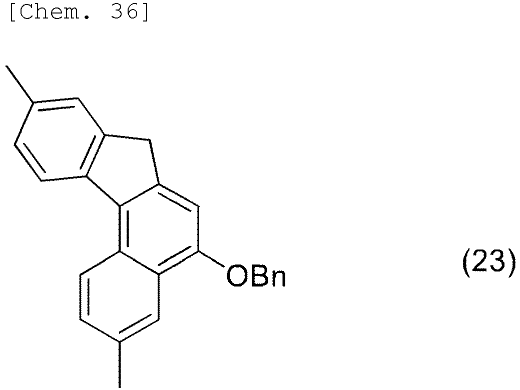

### Second step

First, 1.0 g (2.9 mmol) of the compound represented by Formula (23), 1.0 g (8.6 mmol) of potassium tertiary butoxide (tBuOK), and 50 mL of tetrahydrofuran (THF) were added and cooled to 0 to 5°C. Then, 1.3 g (8.6 mmol) of 1-bromo-2-methoxyethane diluted with 20 mL of THF was added dropwise thereto over 15 minutes. After completion of the addition, the resultant was stirred at room temperature. After consumption of the raw materials was confirmed, the resultant was cooled on ice and partitioned between 10 mL of 2% hydrochloric acid and 50 mL of ethyl acetate. The resulting organic layer was washed with 20 mL of water. This operation was repeated until a pH of an aqueous layer reached 6 to 7. After a solvent was removed from the resulting organic layer, 20 mL of THF and 0.3 g of 5% Pd/C (50% water content) were added thereto, followed by reacting under a pressure with hydrogen gas at 0.05 to 0.1 Mpa. After consumption of the raw materials was confirmed, the Pd/C was filtered off, and a solvent was removed from the resulting organic layer, followed by purification by chromatography on silica gel to obtain a naphthol derivative represented by Formula (24) below at a yield of 83%.

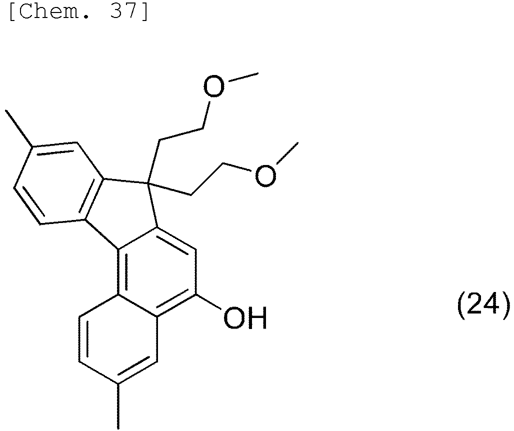

### Third step

First, 0.87 g (2.3 mmol) of the naphthol derivative represented by Formula (24) and 0.88 g (3.5 mmol) of propargyl alcohol represented by Formula (25) below were dissolved in 30 mL toluene, and 0.5 g (0.2 mmol) of pyridinium p-toluenesulfonate was added thereto and stirred at 85°C for 1 hour.

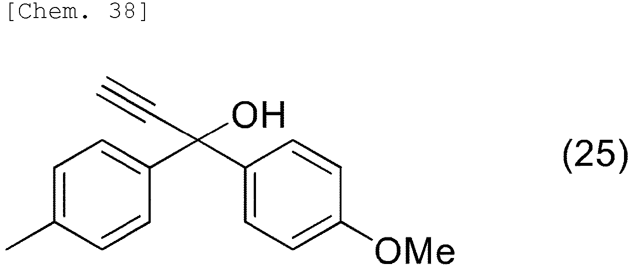

After the above reaction, a solvent was removed, followed by purification by chromatography on silica gel to obtain a photochromic compound represented by Formula (26) below at a yield of 83%.

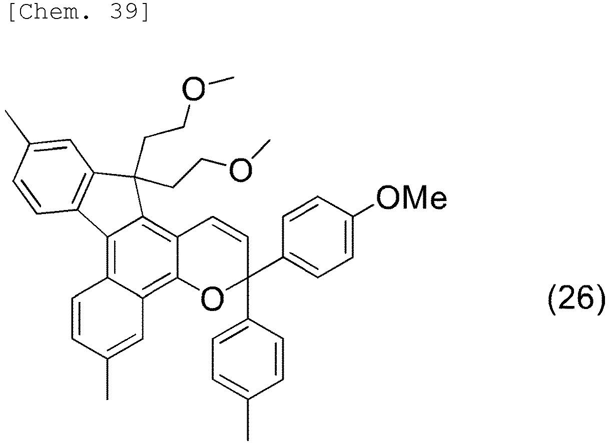

Elemental analysis values for the photochromic compound represented by Formula (26) were C, 82.56%; H, 6.95%, which are in good agreement with the calculated values for C₄₂H₄₂O₄ of C, 82.59%; H, 6.93%.

Proton nuclear magnetic resonance spectra were measured and showed a 13H peak based on a methyl group and methylene around δ: 0.5 to 3.0 ppm, a 13H peak based on a methoxy group and an ethyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 2)

### First step

Three hundred milliliters of toluene was added to 9.9 g (20.0 mmol) of an iodine compound represented by Formula (27) below, and azeotropic dehydration was performed until a water content in the toluene was 100 ppm or less. After the azeotropic dehydration, the resultant was slowly cooled to -20°C, and 15 mL of n-BuLi (1.6 mol/L hexane solution) was slowly added dropwise while maintaining -15 to -20°C. After consumption of the raw materials, 4.4 g (30.0 mmol) of 1,5-dimethoxy-3-pentanone was slowly added dropwise thereto. After the addition, the resultant was stirred at -15 to -20°C for 1 hour and then slowly warmed to room temperature. After stirring for 1 hour, the resultant was partitioned by adding 300 mL of water thereto. This operation was repeated three times, and a solvent was removed from the resulting organic layer, followed by purification by chromatography on silica gel to obtain a compound represented by Formula (28) below at a yield of 75%.

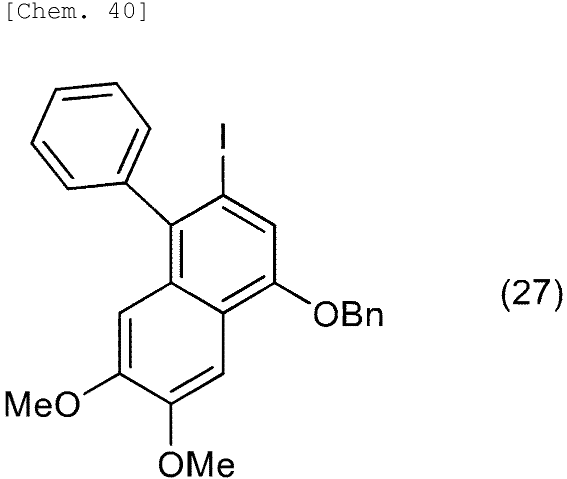

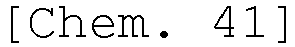

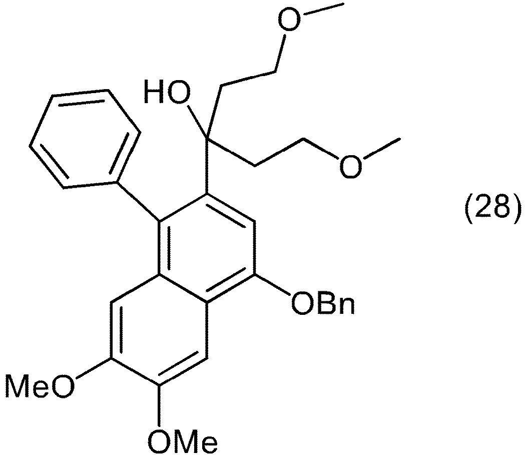

### Second step

First, 350 mL of toluene was added to 14.2 g (75.0 mmol) of p-toluenesulfonic acid monohydrate, and azeotropic dehydration was performed until a water content in the toluene was 300 ppm or less. Then, a toluene solution in which 7.7 g (15.0 mmol) of the Formula (28) was dissolved in 100 mL of toluene was added slowly while maintaining 85 to 100°C, and after the addition, the resultant was refluxed. After consumption of the raw materials was confirmed, the resultant was cooled to room temperature and partitioned by adding 500 mL of water thereto. This operation was repeated three times, and a solvent was removed from the resulting organic layer, followed by purification by chromatography on silica gel to obtain a naphthol derivative represented by Formula (29) below at a yield of 67%.

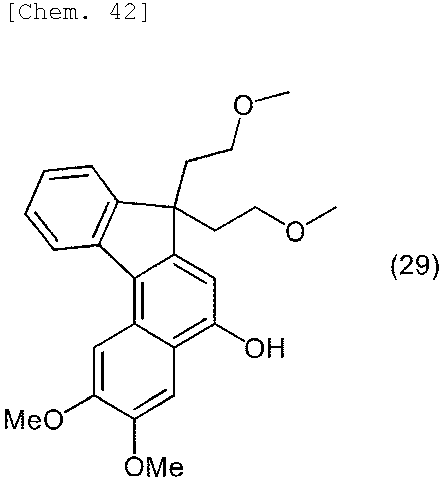

### Third step

A photochromic compound represented by Formula (31) below was obtained at a yield of 86% in the same manner as in Third step of Example 1, except that the naphthol derivative of Formula (29) was used instead of the naphthol derivative of Formula (24) and a compound of Formula (30) below was used instead of the compound of Formula (25).

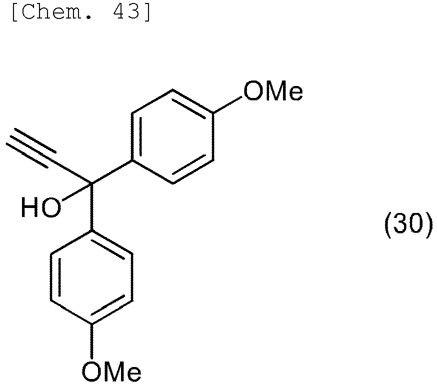

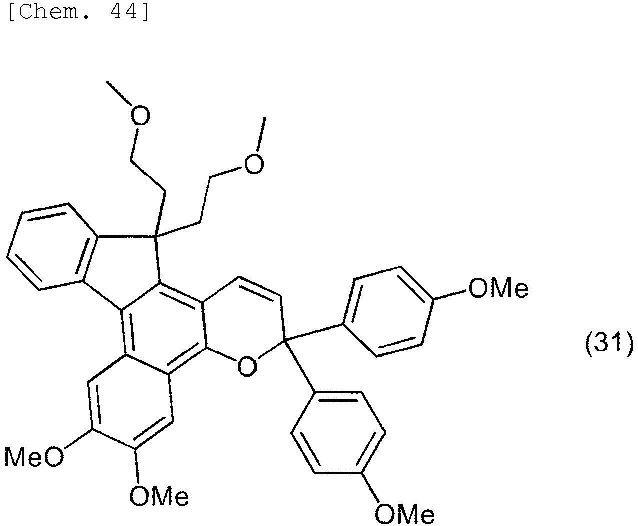

Elemental analysis values for the photochromic compound represented by Formula (31) were determined to be C, 76.59%; H, 6.42%, which are in good agreement with the calculated values for C₄₂H₄₂O₇ of C, 76.57%; H, 6.43%.

Proton nuclear magnetic resonance spectra were measured and showed a 4H peak based on methylene around δ: 0.5 to 3.0 ppm, a 22H peak based on a methoxy group and an ethyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 3)

### First step

A photochromic compound represented by Formula (33) below was obtained at a yield of 86% in the same manner as in Example 2, except that a compound represented by Formula (32) below, which was synthesized with reference to the method described in Patent Document 8, was used and 1,7-dimethoxy-4-heptanone was used instead of 1,5-dimethoxy-3-pentanone.

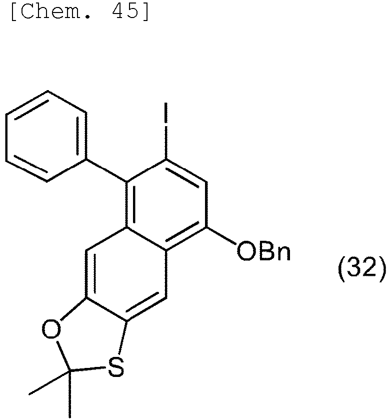

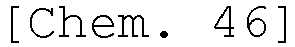

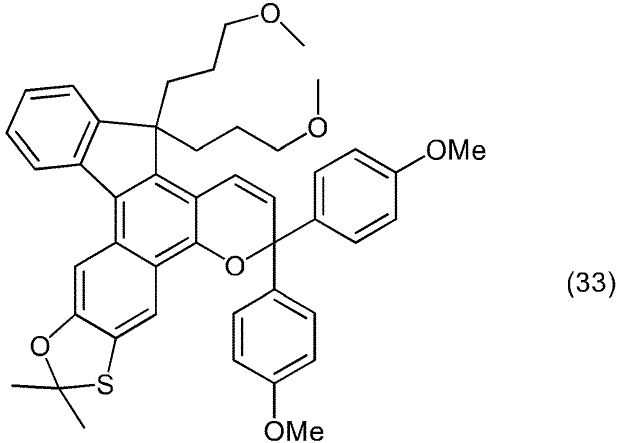

Elemental analysis values for the photochromic compound represented by Formula (33) were determined to be C, 75.59%; H, 6.50%; S, 4.47%, which are in good agreement with the calculated values for C₄₅H₄₆O₆S of C, 75.60%; H, 6.49%; S, 4.48%.

Proton nuclear magnetic resonance spectra were measured and showed a 14H peak based on a methyl group and methylene around δ: 0.5 to 3.0 ppm, a 16H peak based on a methoxy group and a propyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 4)

### First step

First, 28.7 g (62.4 mmol) of a compound represented by Formula (34) below, which was synthesized from 3,4-dimethoxy-4'-bromobenzophenone with reference to the method described in Patent Document 7, was dispersed in 100 mL of methanol. Then, 125 mL of an aqueous solution of 12.5 g (312 mmol) of sodium hydroxide was added to this solution and the resultant was refluxed for 3 hours. After the above reaction, the resultant was washed with concentrated hydrochloric acid and then water, a solvent was removed therefrom, and the resultant was purified by reslurrying with 300 mL of toluene to obtain a carboxylic acid compound represented by Formula (35) below at a yield of 90%.

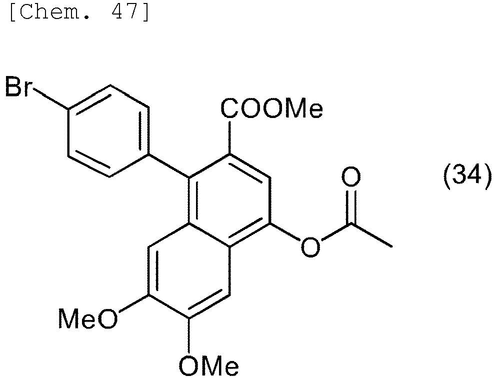

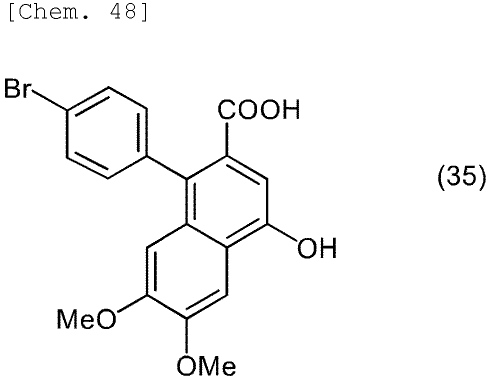

### Second step

First, 22.6 g (56.1 mmol) of the compound represented by Formula (35) and 15.7 g (123.6 mmol) of benzyl chloride were dissolved in 120 mL of DMF. Then, 19.4 g (140.6 mmol) of potassium carbonate was added to this solution, and the resultant was warmed to 80°C and stirred for 3 hours. After the above reaction, a solvent was removed by washing with water. Then, 200 mL of isopropyl alcohol was added thereto, 220 mL of an aqueous solution of 22.4 g (561.0 mmol) of sodium hydroxide was added thereto, and the resultant was refluxed for 4 hours. After the above reaction, the resultant was cooled to 0 to 5°C and partitioned by adding concentrated hydrochloric acid thereto to pH 1. A solvent was removed by washing with 500 mL of water twice. The resultant was purified by reslurrying with 300 mL of toluene to obtain a carboxylic acid compound represented by Formula (36) below at a yield of 89%.

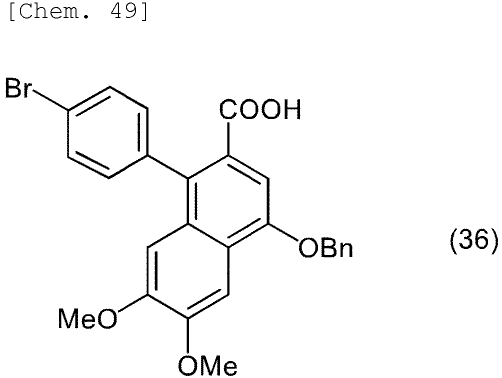

### Third step

First, 10.3 g (54.3 mmol) of p-trifluoromethylphenylboronic acid, 11.5 g (108.7 mmol) of sodium carbonate, 103.5 mL of water, 121.5 mL of 1,2-dimethoxyethane, and 12.2 mL of ethanol were added to 24.6 g (49.9 mmol) of the carboxylic acid compound represented by Formula (36) and the resultant was stirred while bubbling with nitrogen. The nitrogen bubbling was continued for about 20 minutes, and then 142.7 mg (0.1 mmol) of Pd(PPh₃)₄ was added thereto and the resultant was reacted at 75°C for 2 hours. After the above reaction, the resultant was cooled to room temperature, 650 mL of THF was added thereto, and the resultant was cooled to 0 to 5°C and partitioned by adding concentrated hydrochloric acid thereto to pH 1. A solvent was removed by washing with 500 mL of water twice. The resultant was purified by reslurrying with 300 mL of methanol to obtain a carboxylic acid compound represented by Formula (37) below at a yield of 99%.

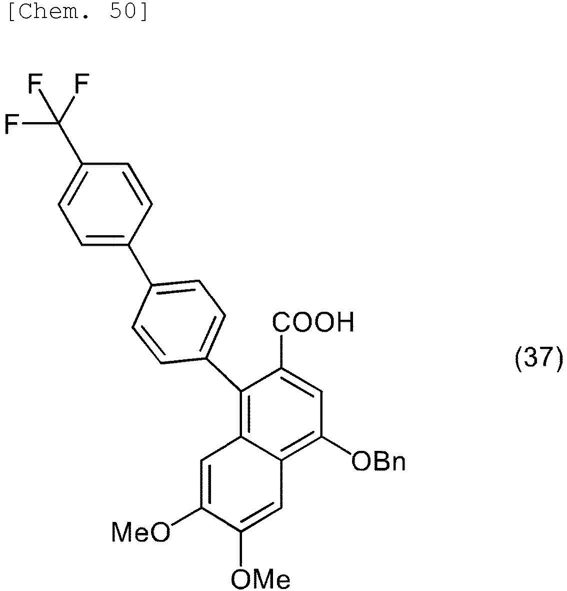

### Fourth step

First, 27.6 g (49.4 mmol) of the carboxylic acid compound represented by Formula (37) was dispersed in 400 mL of toluene. Next, 24.9 g (247.0 mmol) of triethylamine and 17.7 g (64.6 mmol) of diphenylphosphoryl azide were added to this solution and the resultant was stirred at room temperature for 2 hours. Then, 20.0 g (435.3 mmol) of ethanol was added to this solution and the resultant was reacted at 70°C for 2 hours. Five hundred milliliters of ethanol and then 30.0 g (535.7 mmol) of potassium hydroxide were added to this solution and the resultant was refluxed for 4 hours. After the above reaction, ethanol was distilled off at normal pressure, 300 mL of tetrahydrofuran was added thereto, and a solvent was removed by washing with 150 mL of water three times. Six hundred milliliters of acetonitrile and 150.0 g (247.0 mmol) of 6% hydrochloric acid aqueous solution were added thereto and the resultant was cooled to 0°C to 5°C. Then, 15.3 g (74.1 mmol) of 33% sodium nitrite aqueous solution was added to this solution and the resultant was stirred for 30 minutes. Next, 41.2 g (247.0 mmol) of 50% potassium iodide aqueous solution was added to this solution and the resultant was stirred at room temperature for 4 hours. After the above reaction, 500 mL of toluene was added thereto and the resultant was washed with 300 mL of water three times. A solvent was removed from the resulting organic layer, followed by purification by chromatography on silica gel to obtain a compound represented by Formula (38) below at a yield of 70%.

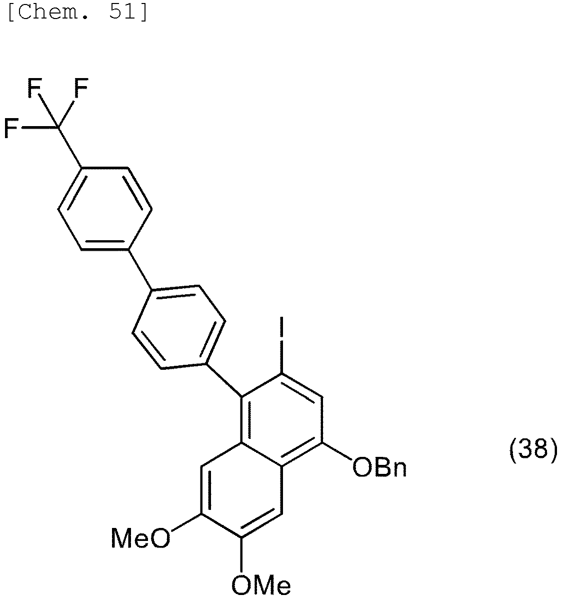

### Fifth step

A photochromic compound represented by Formula (39) below was obtained at a yield of 75% in the same manner as in Example 2, except that the compound represented by Formula (38) was used.

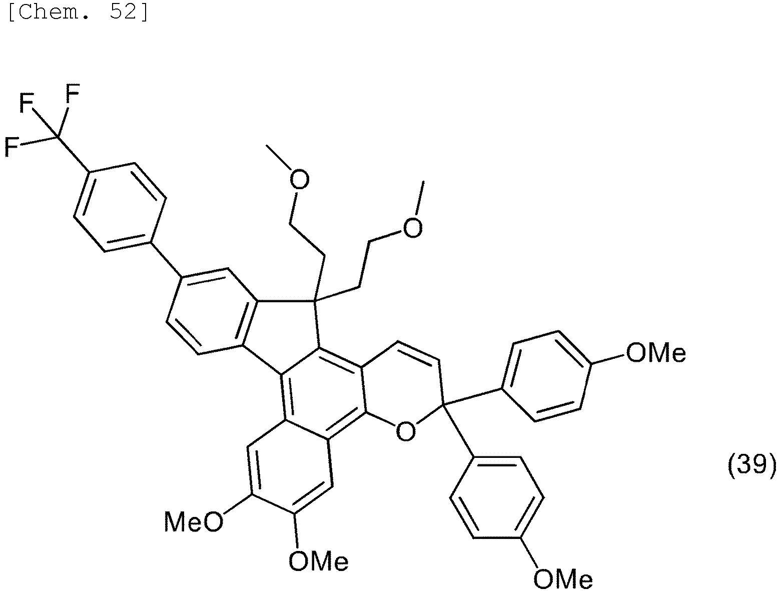

Elemental analysis values for the photochromic compound represented by Formula (39) were determined to be C, 73.27%; H, 5.68%, which are in good agreement with the calculated values for C₄₉H₄₅F₃O₇ of C, 73.30%; H, 5.65%.

Proton nuclear magnetic resonance spectra were measured and showed a 4H peak based on methylene around δ: 0.5 to 3.0 ppm, a 22H peak based on a methoxy group and an ethyloxy group around δ: 3.0 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm. ¹⁹F-nuclear magnetic resonance spectra were measured and showed a peak based on fluorine in a trifluoromethyl group around δ: -70 to -40 ppm.

### (Example 5)

### First step

A compound represented by Formula (40) below was synthesized in the same manner as in First and Second steps of Example 4, except that 3-bromo-4-methoxybenzophenone was used.

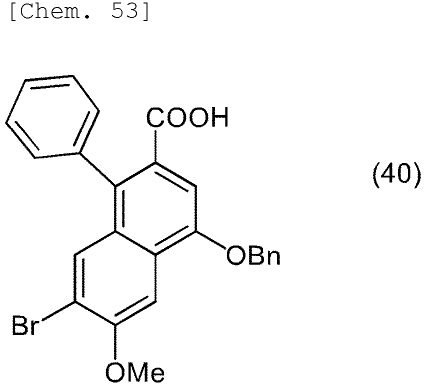

### Second step

A compound represented by Formula (41) below was synthesized in the same manner as in Third and Fourth steps of Example 4, except that 4-methoxyphenylboronic acid was used instead of p-trifluoromethylphenylboronic acid.

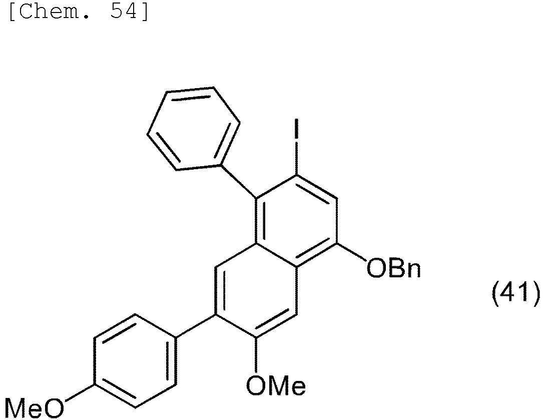

### Third step

A photochromic compound represented by Formula (43) below was obtained at a yield of 72% in the same manner, except that the compound of Formula (41) was used instead of the compound of Formula (27) and a compound of Formula (42) below was used instead of the compound of Formula (30).

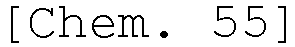

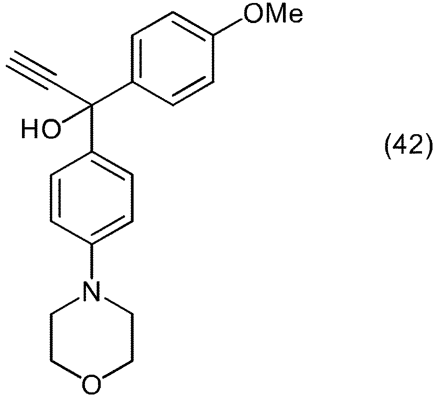

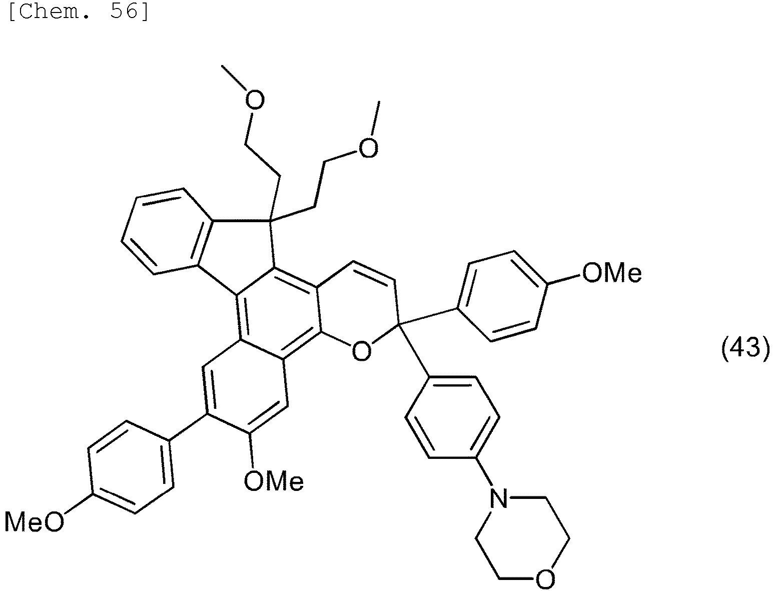

Elemental analysis values for the photochromic compound represented by Formula (43) were determined to be C, 77.56%; H, 6.50%; N, 1.76%, which are in good agreement with the calculated values for C₅₁H₅₁NO₇ of C, 77.54%; H, 6.51%; N, 1.77%.

Proton nuclear magnetic resonance spectra were measured and showed a 4H peak based on methylene around δ: 0.5 to 3.0 ppm, a 27H peak based on a methoxy group, an ethyloxy group, and a morpholino group around δ: 3.0 to 5.0 ppm, and a 20H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 70 ppm.

### (Example 6)

### First step

A naphthol derivative represented by Formula (45) below was obtained at a yield of 65% in the same manner, except that a compound represented by Formula (44) below was used instead of the compound of Formula (27) and 1,5-diethoxy-3-pentanone was used instead of 1,5-dimethoxy-3-pentanone in First step of Example 2.

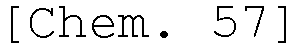

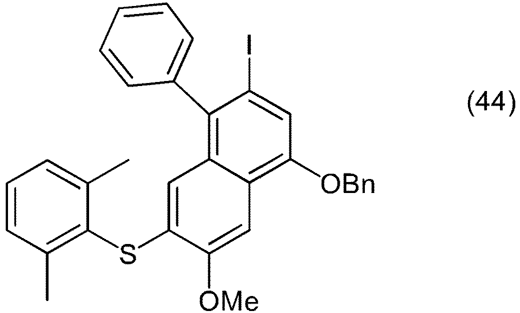

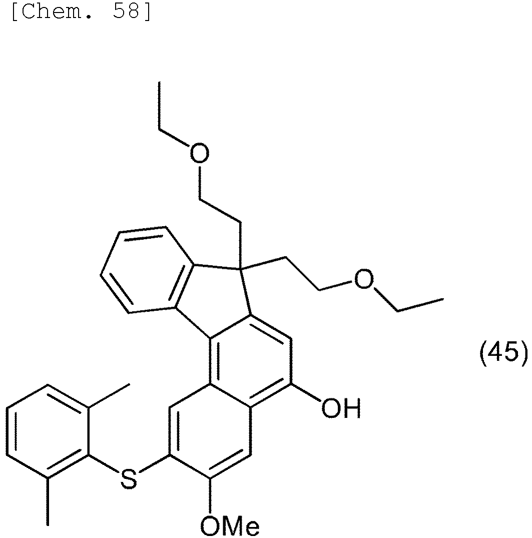

### Second step

A photochromic compound represented by Formula (46) below was obtained at a yield of 77% in the same manner, except that the naphthol derivative of Formula (45) was used for the reaction instead of the naphthol derivative of Formula (41) in Third step of Example 5.

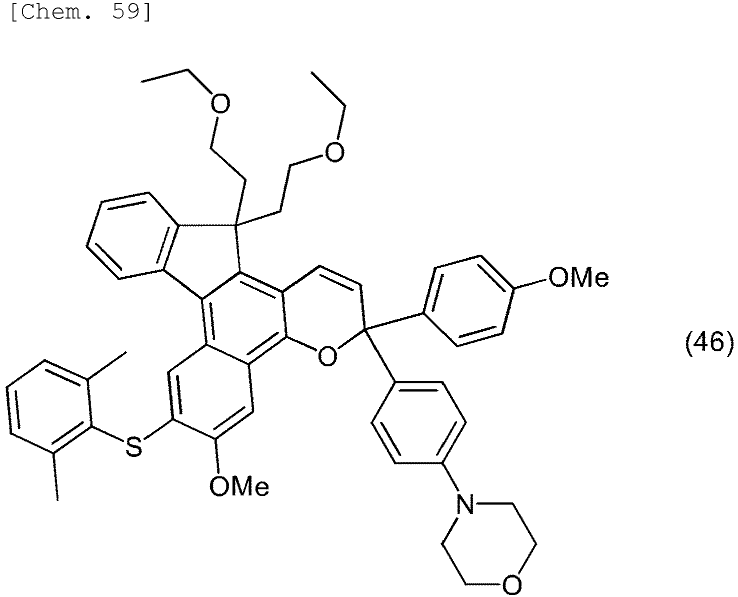

Elemental analysis values for the photochromic compound represented by Formula (46) were determined to be C, 76.48%; H, 6.74%; N, 1.67%; S, 3.77%, which are in good agreement with the calculated values for C₅₄H₅₇NO₆S of C, 76.48%; H, 6.77%; N, 1.65%; S, 3.78%.

Proton nuclear magnetic resonance spectra were measured and showed a 16H peak based on a methyl group and ethylene around δ: 0.5 to 3.0 ppm, a 22H peak based on a methoxy group, an ethyloxy group, and a morpholino group around δ: 3.0 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 70 ppm.

### (Example 7)

### First step

A photochromic compound represented by Formula (48) below was obtained at a yield of 80% in the same manner, except that a compound represented by Formula (47) below was used instead of the compound of Formula (44) in Example 6.

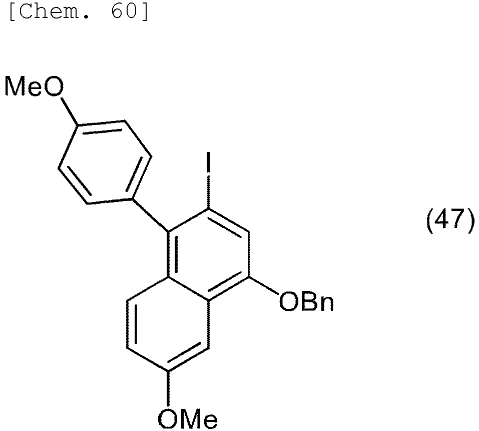

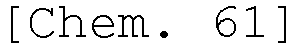

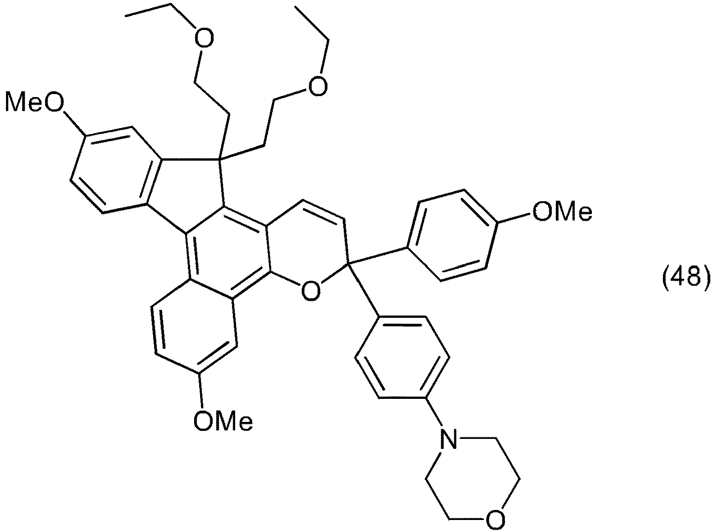

Elemental analysis values for the photochromic compound represented by Formula (48) were determined to be C, 76.10%; H, 6.91%; N, 1.90%, which are in good agreement with the calculated values for C₄₇H₅₁NO₇ of C, 76.09%; H, 6.93%; N, 1.89%.

Proton nuclear magnetic resonance spectra were measured and showed a 10H peak based on a methyl group and ethylene around δ: 0.5 to 3.0 ppm, a 25H peak based on a methoxy group, an ethyloxy group, and a morpholino group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 70 ppm.

### (Example 8)

### First step

A naphthol derivative represented by Formula (50) below was obtained at a yield of 65% in the same manner, except that a compound represented by Formula (49) below was used instead of the compound of Formula (27) and 5-methoxy-3-pentanone was used instead of 1,5-dimethoxy-3-pentanone in First step of Example 2.

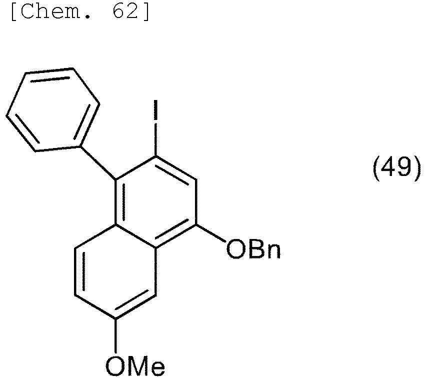

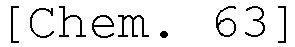

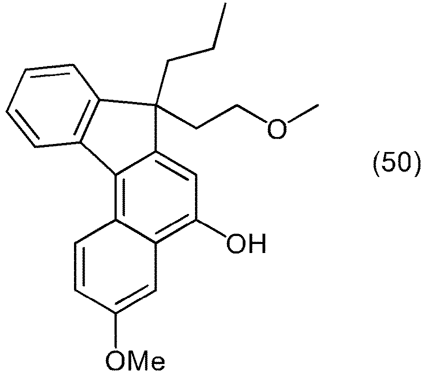

### Second step

A photochromic compound represented by Formula (52) below was obtained at a yield of 69% in the same manner, except that a compound represented by Formula (51) below was used instead of the compound of Formula (25) and the naphthol derivative of Formula (50) was used instead of the naphthol derivative of Formula (24) below in Third step of Example 1.

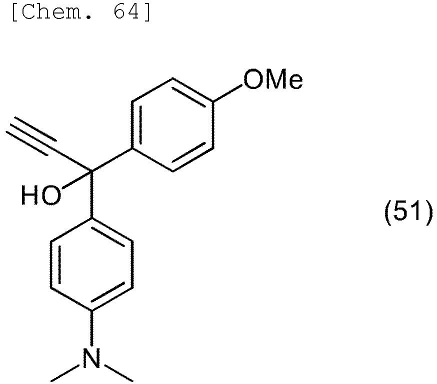

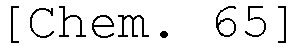

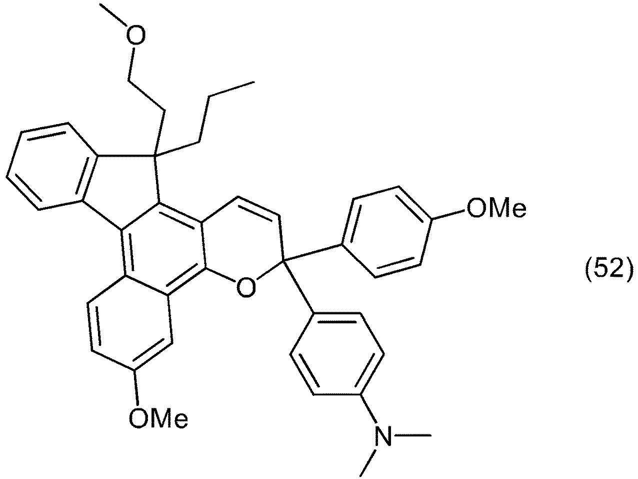

Elemental analysis values for the photochromic compound represented by Formula (52) were determined to be C, 80.61%; H, 6.91%; N, 2.25%, which are in good agreement with the calculated values for C₄₂H₄₃NO₄ of C, 80.61%; H, 6.93%; N, 2.24%.

Proton nuclear magnetic resonance spectra were measured and showed a 9H peak based on a propyl group and ethylene around δ: 0.5 to 3.0 ppm, a 17H peak based on a methoxy group, an ethyloxy group, and a dimethylamino group around δ: 3.0 to 5.0 ppm, and a 17H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 70 ppm.

### (Example 9)

### First step

A naphthol derivative represented by Formula (53) below was obtained at a yield of 54% in the same manner, except that 4-morpholinophenylboronic acid was used instead of 4-methoxyphenylboronic acid, and 1,3-dimethylthio-2-propanone was used instead of 1,5-dimethoxy-3-heptanone in Example 5.

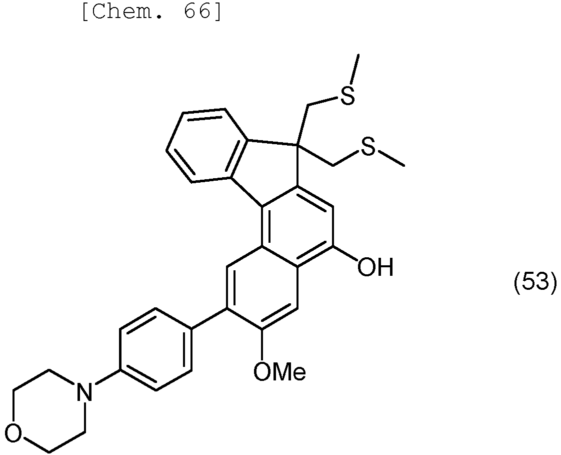

### Second step

A photochromic compound represented by Formula (55) below was obtained at a yield of 60% in the same manner, except that a compound represented by Formula (54) below was used instead of the compound of Formula (25) and the naphthol derivative of Formula (53) was used instead of the naphthol derivative of Formula (24) in Third step of Example 1.

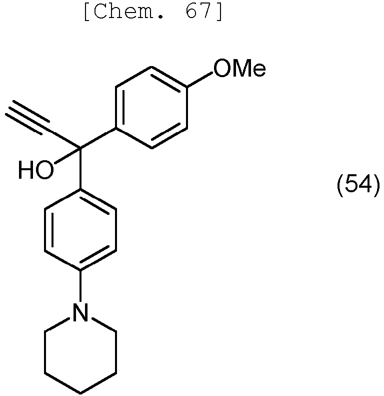

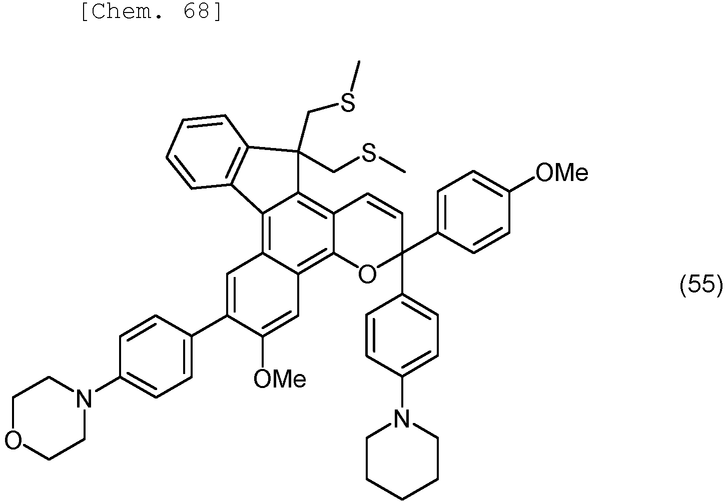

Elemental analysis values for the photochromic compound represented by Formula (55) were determined to be C, 75.16%; H, 6.44%; N, 3.30%; S, 7.56%, which are in good agreement with the calculated values for C₅₃H₅₄N₂O₄S₂ of C, 75.14%; H, 6.43%; N, 3.31%; S, 7.57%.

Proton nuclear magnetic resonance spectra were measured and showed a 6H peak based on a piperidino group and ethylene around δ: 0.5 to 2.0 ppm, a 28H peak based on a methoxy group, a methylthio group, and a piperidino group around δ: 2.0 to 5.0 ppm, and a 20H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 70 ppm.

### (Example 10)

### First step

First, 100 mL of toluene was added to 9.3 g (20.0 mmol) of the compound represented by Formula (40), 6.9 g (80.0 mmol) of morpholine, and 9.6 g (100.0 mmol) of sodium tertiary butoxide and the resultant was subject to nitrogen bubbling for 20 minutes. Then, 0.46 g (0.5 mmol) of tris(dibenzylideneacetone)dipalladium(0) and 0.95 g (2.0 mmol) of 2-dicyclohexylphosphino-triisopropylbiphenyl was added thereto and the resultant was reacted at 80°C for 4 hours. After consumption of the raw materials was confirmed, the resultant was cooled at 0 to 5°C, and 10% hydrochloric acid was added thereto to pH 6 to 7. The resultant was partitioned and washed with 100 mL of water three times. A solvent was removed from the resulting organic layer and the resultant was purified by reslurrying with 150 mL of acetonitrile to obtain a compound represented by Formula (56) below at a yield of 89%.

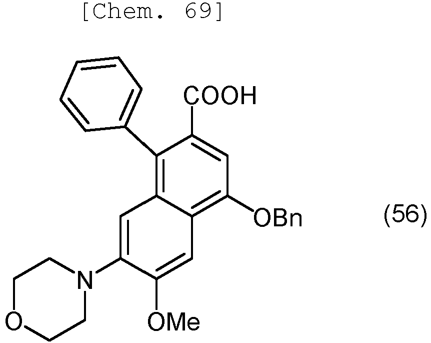

### Second step

A photochromic compound represented by Formula (57) below was obtained at a yield of 72% in the same manner as in Fourth and Fifth steps of Example 4, except that the compound of Formula (56) was used instead of the compound of Formula (37).

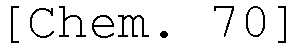

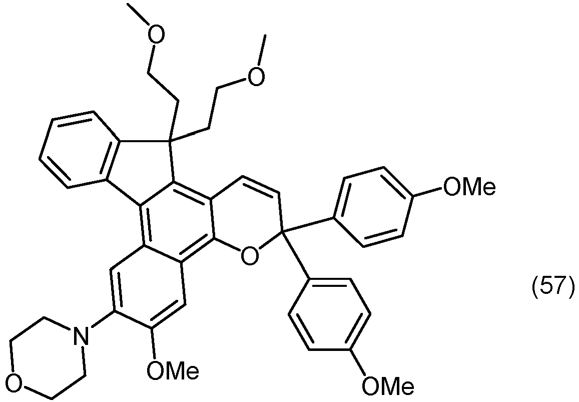

Elemental analysis values for the photochromic compound represented by Formula (57) were determined to be C, 75.69%; H, 6.66%; N, 1.97%, which are in good agreement with the calculated values for C₄₅H₄₇NO₇ of C, 75.71%; H, 6.64%; N, 1.96%.

Proton nuclear magnetic resonance spectra were measured and showed a 4H peak based on ethylene around δ: 0.5 to 2.0 ppm, a 27H peak based on a methoxy group, an ethoxy group, and a morpholino group around δ: 2.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 70 ppm.

### (Example 11)

### First step

A naphthol derivative represented by Formula (58) below was obtained at a yield of 52% in the same manner, except that the compound of Formula (22) was used instead of the compound of Formula (27) and dimethoxyacetone was used instead of 1,5-dimethoxy-3-pentanone in First and Second steps of Example 2.

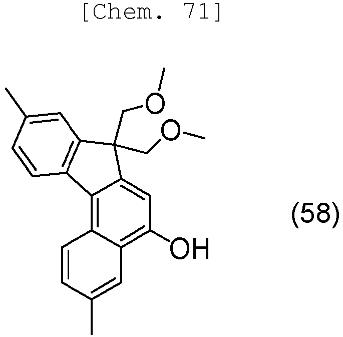

### Second step

A photochromic compound represented by Formula (59) below was obtained at a yield of 62% in the same manner, except that the naphthol derivative of Formula (58) was used instead of the naphthol derivative of Formula (24) in Third step of Example 1.

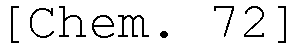

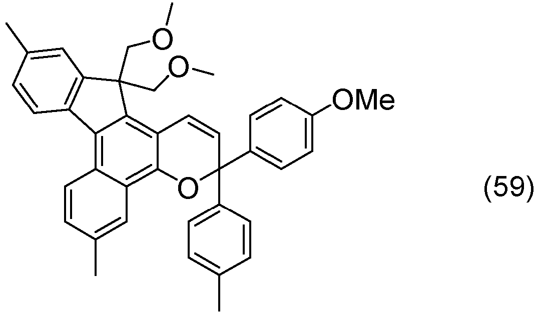

Elemental analysis values for the photochromic compound represented by Formula (59) were determined to be C, 82.44%; H, 6.58%, which are in good agreement with the calculated values for C₄₀H₃₈O₄ of C, 82.45%; H, 6.57%.

Proton nuclear magnetic resonance spectra were measured and showed a 9H peak based on a methyl group around δ: 0.5 to 3.0 ppm, a 13H peak based on a methoxy group and a methyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Analytical result for naphthol derivative)

Table 1 summarizes analytical results of the naphthol derivatives used in Examples 1 to 11.

### [Table 1]

**Table 1**

| Example | Calculated value | | | | Determined value | | | | 1H-NMR |
|---|---|---|---|---|---|---|---|---|---|
| | C | H | N | S | C | H | N | S | |
| 1 | 79.76 | 7.50 | - | - | 79.80 | 7.48 | - | - | δ0.5-5.0ppm 20H |
| | | | | | | | | | δ5.0-9.0ppm 8H |
| 2 | 73.51 | 6.91 | - | - | 73.47 | 6.93 | - | - | δ0.5-5.0ppm 20H |
| | | | | | | | | | δ5.0-9.0ppm 8H |
| 3 | 72.38 | 6.94 | - | 6.90 | 72.39 | 6.96 | - | 6.92 | δ0.5-5.0ppm 24H |
| | | | | | | | | | δ5.0-9.0ppm 8H |
| 4 | 69.55 | 5.65 | - | - | 69.50 | 5.69 | - | - | δ0.5-5.0ppm 20H |
| | | | | | | | | | δ5.0-9.0ppm 11H |
| 5 | 76.84 | 6.66 | - | - | 76.86 | 6.65 | - | - | δ0.5-5.0ppm 20H |
| | | | | | | | | | δ5.0-9.0ppm 12H |
| 6 | 75.24 | 7.06 | - | 5.91 | 75.25 | 7.10 | - | 5.89 | δ0.5-5.0ppm 27H |
| | | | | | | | | | δ5.0-9.0ppm 11H |
| 7 | 74.29 | 7.39 | - | - | 74.32 | 7.41 | - | - | δ0.5-5.0ppm 24H |
| | | | | | | | | | δ5.0-9.0ppm 8H |
| 8 | 79.53 | 7.23 | - | - | 79.57 | 7.21 | - | - | δ0.5-5.0ppm 17H |
| | | | | | | | | | δ5.0-9.0ppm 9H |
| 9 | 70.69 | 6.12 | 2.58 | 11.79 | 70.63 | 6.14 | 2.59 | 11.81 | δ0.5-5.0ppm 21H |
| | | | | | | | | | δ5.0-9.0ppm 12H |
| 10 | 72.55 | 7.18 | 3.02 | - | 72.55 | 7.21 | 3.01 | - | δ0.5-5.0ppm 25H |
| | | | | | | | | | δ5.0-9.0ppm 8H |
| 11 | 79.28 | 6.94 | - | - | 79.26 | 6.97 | - | - | δ0.5-5.0ppm 16H |
| | | | | | | | | | δ5.0-9.0ppm 8H |

### (Evaluation of photochromic plastic lens made by coating method for physical property)

### (Example 12)

### (Preparation of curable composition)

First, the photochromic compound obtained in Example 1, a photoinitiator, and a polymerizable compound were mixed to obtain a curable composition.

The polymerizable compound was a combination of the following radical polymerizable monomers:
Polyethylene glycol dimethacrylate (average molecular weight: 736): 42 parts by mass
Polyethylene glycol dimethacrylate (average molecular weight: 536): 12 parts by mass
Trimethylolpropane methacrylate: 38 parts by mass
γ-Methacryloyloxypropyltrimethoxysilane: 2 parts by mass Glycidyl methacrylate: 1 part by mass.

Note that, in the curable composition, the photochromic compound was added in an amount of 0.27 mmol taking a total amount of the radical polymerizable monomers as 100 g.

The following additives were used:

Phenylbis(2,4,6-trimethylbenzoyl)phosphine oxide (photoinitiator: Omnirad 819): 0.3 parts by mass Ethylene bis(oxyethylene) bis[3-(5-tert-butyl-4-hydroxy-m-tolyl) propionate] (stabilizer, Irganox 245): 1 part by mass Bis(1,2,2,6,6-pentamethyl-4-piperidyl)sebacate: 3 parts by mass
Leveling agent (L7001) manufactured by DuPont Toray Specialty Materials K.K.: 0.1 parts by mass.

Note that, the above-mentioned additives are described with blended ratios taking a total of the radical polymerizable monomers as 100 parts by mass.

### (Production of optical article)

Using this curable composition, a photochromic laminate was obtained by a lamination method including polymerization as follows.

First, a thiourethane plastic lens with a center thickness of 2 mm and a refractive index of 1.60 was prepared as an optical substrate. Note that, the thiourethane plastic lens had been alkaline-etched in a 10% sodium hydroxide aqueous solution at 50°C for 5 minutes in advance, and then thoroughly washed with distilled water.

A spin coater (1H-DX2, manufactured by MIKASA CO., LTD) was used to coat a surface of the above-mentioned plastic lens with a moisture-curing primer (product name: TR-SC-P, manufactured by Tokuyama Corporation) at a rotation speed of 70 rpm for 15 seconds, followed by at 1000 rpm for 10 seconds. Then, about 2 g of the photochromic curable composition obtained as above was spin-coated at a rotation speed of 60 rpm for 40 seconds, followed by at 600 rpm for 10 to 20 seconds so as to achieve a photochromic coating layer with a film thickness of 40 µm.

The lens of which surface had been coated with the photochromic curable composition (photochromic coating layer) was irradiated with light for 90 seconds in a nitrogen gas atmosphere using a metal halide lamp with an output of 200 mW/cm² to cure the coating. The resultant was then further heated at 110°C for 1 hour to produce a photochromic laminate with a photochromic layer.

### (Examples 13 to 22)

Photochromic laminates were produced using the photochromic compounds obtained in Examples 2 to 11 (compounds according to Formula (31), Formula (33), Formula (39), Formula (43), Formula (46), Formula (48), Formula (52), Formula (55), Formula (57), and Formula (59)) in the same manner as in Example 12.

### (Comparative Examples 1 to 3)

Photochromic laminates were obtained in the same manner as in Example 12 using photochromic compounds represented by Formulae (A) to (C) below.

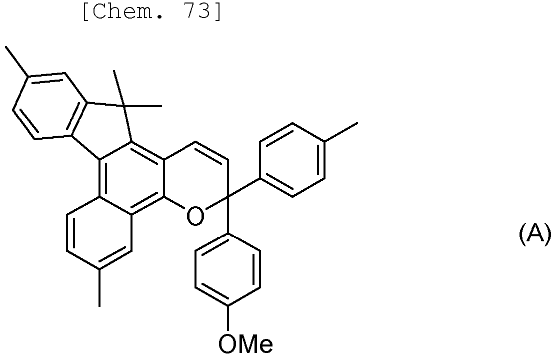

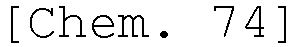

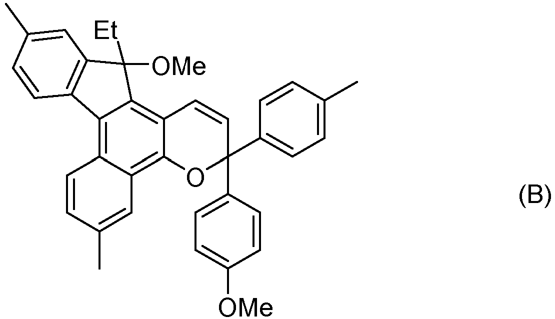

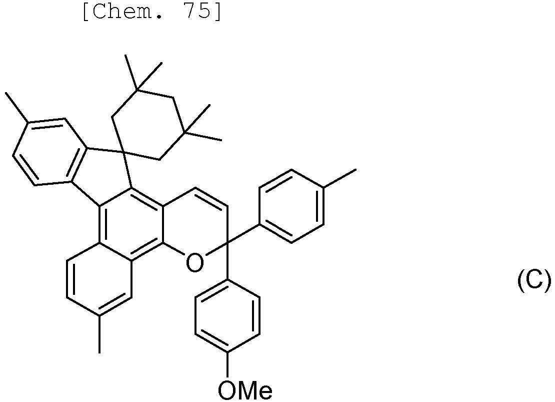

### <Evaluation method>

The resulting photochromic laminates were evaluated by the below-mentioned methods.

### (1) Photochromic property

[1] Maximum absorption wavelength (λmax): This is the maximum absorption wavelength after color development determined by a spectrophotometer manufactured by Otsuka Electronics Co., Ltd. (instantaneous multichannel photo detector MCPD3000) and was used as an index of a color tone upon color development.
[2] Color developing density at 23°C (A₂₃):
   This is a difference between an absorbance {ε(240)} after irradiation with light at 23°C for 240 seconds and an absorbance ε(0) when not irradiated with light at the above-mentioned maximum absorption wavelength and was used as an index of a color developing density. The higher this value is, the better photochromic property is.
[3] Color developing density at 35°C (A₃₅):
   This is a difference between an absorbance {ε(180)} after irradiation with light at 35°C for 240 seconds and an absorbance ε(0) when not irradiated with light at a first and second absorption wavelengths, and was used as an index of a color developing density. The higher this value is, the better photochromic property is.
[4] Rate of temperature change:
   Color developing density at 23°C (A₂₃) / Color developing density at 35°C (A₃₅) x 100 (%). When this value is high, a difference in a color developing density under high and low temperatures is small, that is, temperature dependence is low.
[5] Color fading half-life at 23°C [τ1/2 (sec).)]:
   This is a period of time taken by an absorbance at the above-mentioned maximum absorption wavelength of a sample to decrease to 1/2 of {ε(240)-ε(0)} when irradiation is stopped after irradiation with light at 23°C for 240 seconds, and was used as an index of a color fading rate. The shorter this period of time is, the faster the color fading rate is.
[6] Residual ratio (A₅₀/A₀ x 100) :
   The resulting photochromic plastic lens was subjected to accelerated degradation for 50 hours using a xenon weather meter X25 manufactured by Suga Test Instruments Co., Ltd. Color developing densities were then evaluated as described above before and after a test, and a color developing density before the test (A₀) and a color developing density after the test (A₅₀) were measured. A ratio (A₅₀/A₀) was determined as a residual ratio and was used as an index of color development durability. The higher the residual ratio is, the more durable the color development is.

The results of Examples 12 to 22 are summarized in Table 2. The results of Comparative examples 1 to 3 are summarized in Table 3.

### [Table 2]

**Table 2**

| | Compound No. | Maximum absorption wavelength (nm) | Color development density at 23°C(-) | Color development density at 35°C(-) | Color fading half-life at 23°C (sec) | Residual ratio (%) |
|---|---|---|---|---|---|---|
| Example 12 | Formula (26) | 435 | 0.55 | 0.35 | 62 | 83 |
| | | 557 | 0.95 | 0.61 | 62 | 83 |
| Example 13 | Formula (31) | 454 | 0.94 | 0.53 | 49 | 86 |
| | | 571 | 0.56 | 0.32 | 50 | 86 |
| Example 14 | Formula (33) | 460 | 0.97 | 0.54 | 43 | 85 |
| | | 573 | 0.57 | 0.32 | 43 | 85 |
| Example 15 | Formula (39) | 456 | 1.05 | 0.61 | 35 | 83 |
| | | 577 | 0.66 | 0.40 | 35 | 83 |
| Example 16 | Formula (43) | 470 | 0.62 | 0.38 | 33 | 81 |
| | | 596 | 0.76 | 0.46 | 33 | 81 |
| Example 17 | Formula (46) | 483 | 0.68 | 0.38 | 30 | 84 |
| | | 597 | 0.68 | 0.38 | 30 | 84 |
| Example 18 | Formula (48) | 458 | 0.52 | 0.34 | 51 | 81 |
| | | 608 | 0.98 | 0.64 | 51 | 81 |
| Example 19 | Formula (52) | 489 | 0.35 | 0.18 | 20 | 75 |
| | | 609 | 0.65 | 0.34 | 20 | 75 |
| Example 20 | Formula (55) | 504 | 0.73 | 0.44 | 46 | 79 |
| | | 606 | 0.85 | 0.53 | 46 | 79 |
| Example 21 | Formula (57) | 469 | 1.29 | 0.82 | 63 | 86 |
| | | 568 | 0.62 | 0.39 | 63 | 86 |
| Example 22 | Formula (59) | 433 | 0.56 | 0.38 | 105 | 81 |
| | | 549 | 0.99 | 0.69 | 105 | 81 |

### [Table 3]

**Table 3**

| | Compound No. | Maximum absorption wavelength (nm) | Color development density at 23°C(-) | Color development density at 35°C(-) | Color fading half-life at 23°C (sec) | Residual ratio (%) |
|---|---|---|---|---|---|---|
| Comparative Example 1 | Formula (A) | 435 | 0.60 | 0.43 | 162 | 79 |
| | | 557 | 1.06 | 0.77 | 162 | 79 |
| Comparative Example 2 | Formula (B) | 438 | 0.48 | 0.29 | 51 | 59 |
| | | 571 | 0.92 | 0.54 | 51 | 59 |
| Comparative Example 3 | Formula (C) | 423 | 0.43 | 0.25 | 45 | 76 |
| | | 551 | 0.86 | 0.51 | 45 | 76 |

FIG. 1 is a graph showing an example of a relationship between a color fading half-life at 23°C and a color developing density at 35°C of photochromic laminates according to Examples and Comparative Examples. The photochromic laminates according to Example 12, Example 22, and Comparative Examples 1 to 3 were used for the plot in FIG. 1.

As can be clearly seen from FIG. 1, the compounds of Examples 12 and 22 have improved temperature dependence and color fading rate compared to Comparative Examples 1 to 3. Furthermore, the results in Tables 1 and 2 clearly show that durability is also improved.

The only difference between the compounds of Examples 12 and 22 and the compound of Comparative Examples 1 to 3 is a substituent attached to a carbon atom at position 13 of indenonaphthopyran. It is clear that the photochromic compounds according to the embodiment are photochromic compounds with superior temperature dependence, color fading rate, and durability to those of conventional photochromic compounds.

### (Example 23)

### First step

A photochromic compound represented by Formula (60) below was obtained at a yield of 76% in the same manner, except that 1-bromo-3-methoxypropane was used instead of 1-bromo-2-methoxyethane in Second step of Example 1.

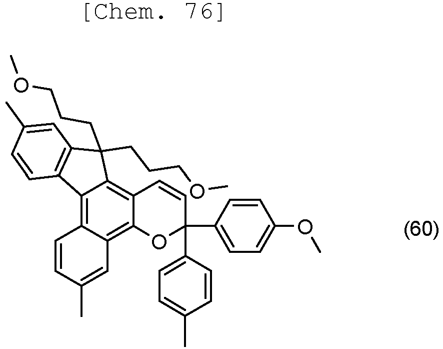

Elemental analysis values for the photochromic compound represented by Formula (60) were determined to be C, 82.69%; H, 7.24%, which are in good agreement with the calculated values for C₄₄H₄₆O₄ of C, 82.72%; H, 7.26%. Proton nuclear magnetic resonance spectra were measured and showed a 17H peak based on a methyl group and a propyleneoxy group around δ: 0.5 to 3.0 ppm, a 13H peak based on a methoxy group and a propyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 24)

### First step

A photochromic compound represented by Formula (61) below was obtained at a yield of 80% in the same manner, except that 1-bromo-4-methoxybutane was used instead of 1-bromo-2-methoxyethane in Second step of Example 1.

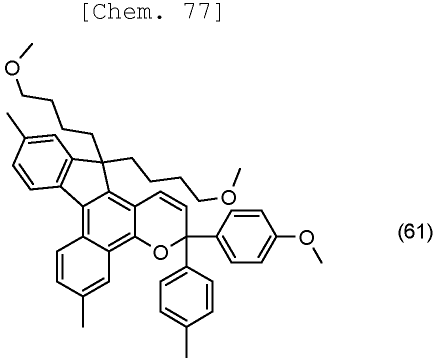

Elemental analysis values for the photochromic compound represented by Formula (61) were determined to be C, 82.88%; H, 7.53%, which are in good agreement with the calculated values for C₄₆H₅₀O₄ of C, 82.85%; H, 7.56%.

Proton nuclear magnetic resonance spectra were measured and showed a 21H peak based on a methyl group and a butyleneoxy group around δ: 0.5 to 3.0 ppm, a 13H peak based on a methoxy group and a butyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 25)

### First step

A photochromic compound represented by Formula (62) below was obtained at a yield of 71% in the same manner, except that 1-(2-bromoethoxy)butane was used instead of 1-bromo-2-methoxyethane in Second step of Example 1.

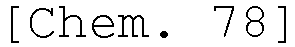

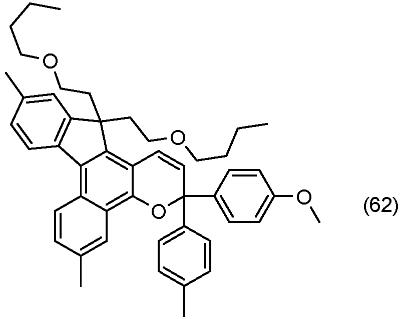

Elemental analysis values for the photochromic compound represented by Formula (62) were determined to be C, 83.00%; H, 7.81%, which are in good agreement with the calculated values for C₄₈H₅₄O₄ of C, 82.96%; H, 7.83%.

Proton nuclear magnetic resonance spectra were measured and showed a 27H peak based on a methyl group, an ethyleneoxy group, and a butyleneoxy group around δ: 0.5 to 3.0 ppm, an 11H peak based on a methoxy group, an ethyleneoxy group, and a butyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 26)

### First step

A photochromic compound represented by Formula (63) below was obtained at a yield of 75% in the same manner, except that 1-(2-bromoethoxy)hexane was used instead of 1-bromo-2-methoxyethane in Second step of Example 1.

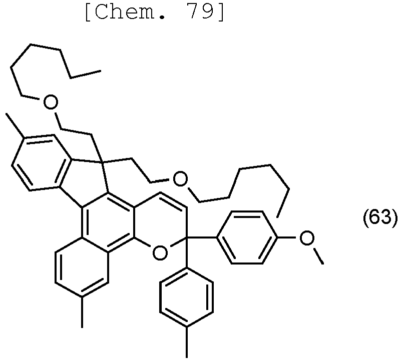

Elemental analysis values for the photochromic compound represented by Formula (63) were determined to be C, 83.17%; H, 8.28%, which are in good agreement with the calculated values for C₅₂H₆₂O₄ of C, 83.16%; H, 8.32%.

Proton nuclear magnetic resonance spectra were measured and showed a 35H peak based on a methyl group, an ethyleneoxy group, and a hexyleneoxy group around δ: 0.5 to 3.0 ppm, an 11H peak based on a methoxy group, an ethyleneoxy group, and a hexyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 27)

### First step

A photochromic compound represented by Formula (64) below was obtained at a yield of 72% in the same manner, except that 1-bromo-2-(2-methoxyethoxy)ethane was used instead of 1-bromo-2-methoxyethane in Second step of Example 1.

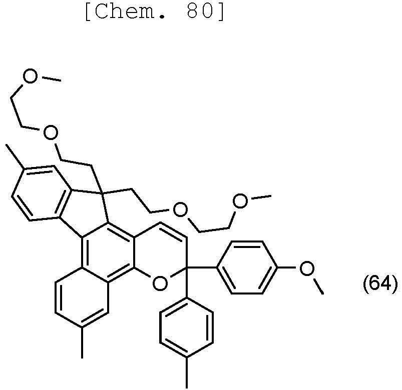

Elemental analysis values for the photochromic compound represented by Formula (64) were determined to be C, 79.08%; H, 7.18%, which are in good agreement with the calculated values for C₄₆H₅₀O₆ of C, 79.05%; H, 7.21%.

Proton nuclear magnetic resonance spectra were measured and showed a 13H peak based on a methyl group and an ethyleneoxy group around δ: 0.5 to 3.0 ppm, a 21H peak based on a methoxy group and an ethyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 28)

### First step

A photochromic compound represented by Formula (65) below was obtained at a yield of 51% in the same manner, except that 1-(2-bromoethoxy)dodecane was used instead of 1-bromo-2-methoxyethane in Second step of Example 1.

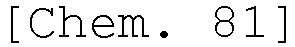

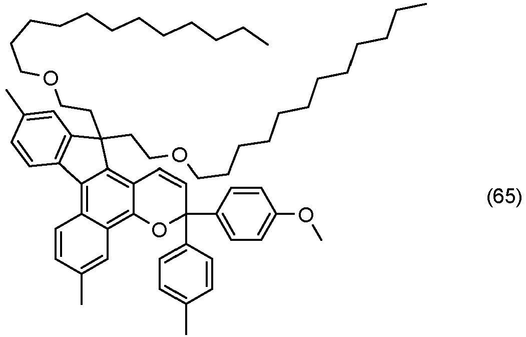

Elemental analysis values for the photochromic compound represented by Formula (65) were determined to be C, 83.57%; H, 9.45%, which are in good agreement with the calculated values for C₆₄H₈₆O₄ of C, 83.61%; H, 9.43%.

Proton nuclear magnetic resonance spectra were measured and showed a 59H peak based on a methyl group, an ethyleneoxy group, and a dodecyloxy group around δ: 0.5 to 3.0 ppm, an 11H peak based on a methoxy group, an ethyleneoxy group, and a dodecyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 28)

### First step

A photochromic compound represented by Formula (66) below was obtained at a yield of 65% in the same manner, except that 1-bromo-3-(methylthio)propane was used instead of 1-bromo-2-methoxyethane in Second step of Example 1.

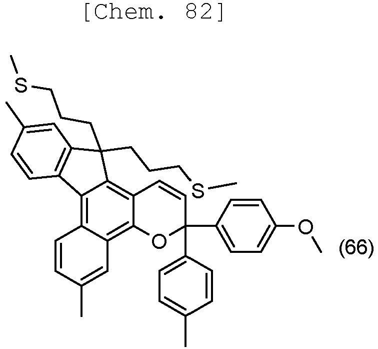

Elemental analysis values for the photochromic compound represented by Formula (66) were determined to be C, 78.70%; H, 6.97%; S, 9.53%, which are in good agreement with the calculated values for C₄₄H₄₆O₂S₂ of C, 78.76%; H, 6.91%; S, 9.56%.

Proton nuclear magnetic resonance spectra were measured and showed a 27H peak based on a methyl group and a propylenemethylthio group around δ: 0.5 to 3.0 ppm, a 3H peak based on a methoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 29)

### First step

A photochromic compound represented by Formula (67) below was obtained at a yield of 73% in the same manner, except that 1-bromo-3-(2-methoxyethoxy)propane was used instead of 1-bromo-2-methoxyethane in Second step of Example 1.

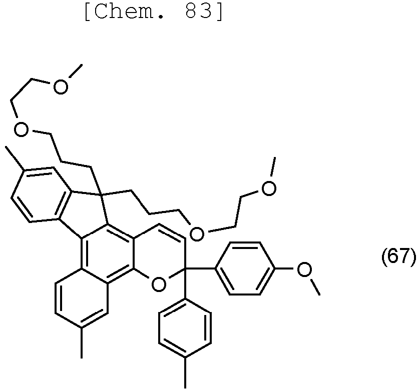

Elemental analysis values for the photochromic compound represented by Formula (67) were determined to be C, 79.28%; H, 7.53%, which are in good agreement with the calculated values for C₄₈H₅₄O₆ of C, 79.31%; H, 7.49%.

Proton nuclear magnetic resonance spectra were measured and showed a 17H peak based on a methyl group and a propyleneoxy group around δ: 0.5 to 3.0 ppm, a 21H peak based on a methoxy group, a propyleneoxy group, and an ethoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 30)

### First step

A carboxylic acid compound represented by Formula (68) below was synthesized from 4,4'-dimethylbenzophenone with reference to the method described in Patent Document 7.

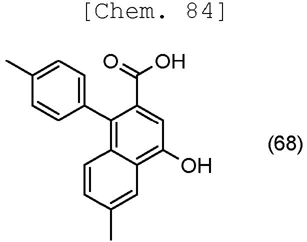

Then, 95.4 g (500 mmol) of p-toluenesulfonic acid monohydrate and 1000 mL of toluene were added to 29.2 g (100 mmol) of the resulting carboxylic acid, followed by reacting while azeotropic dehydration was performed. After consumption of the raw materials was confirmed, the resultant was cooled to room temperature and the resulting solid was filtered to obtain a carbonyl compound represented by Formula (69) below at a yield of 87%.

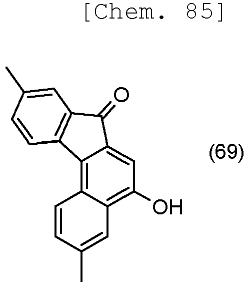

### Second step

The carbonyl compound represented by Formula (69) was reacted to obtain a naphthol derivative represented by Formula (70) below at a yield of 80% with reference to the method described in Patent Document 10.

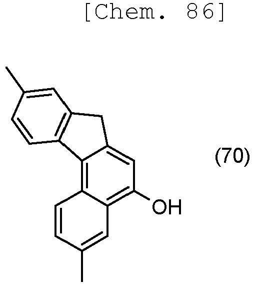

### Third step

A photochromic compound represented by Formula (71) below was obtained at a yield of 87% in the same manner, except that the naphthol derivative represented by Formula (70) was used for the reaction instead of the naphthol derivative of Formula (24) in Third step of Example 1.

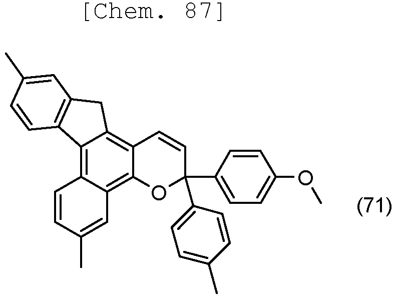

### Fourth step

A photochromic compound represented by Formula (72) below was obtained at a yield of 67% in the same manner, except that the photochromic compound of Formula (71) was used instead of the compound of Formula (23) and 1-bromo-4-(ethoxymethoxy)butane was used instead of 1-bromo-2-methoxyethane in Second step of Example 1.

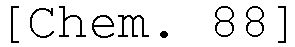

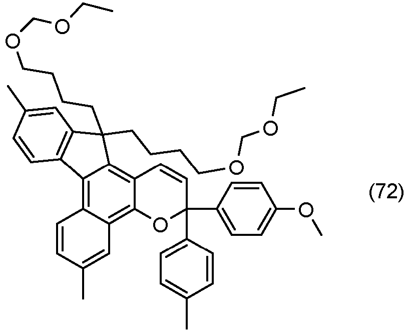

Elemental analysis values for the photochromic compound represented by Formula (72) were determined to be C, 79.56%; H, 7.77%, which are in good agreement with the calculated values for C₅₀H₅₈O₆ of C, 79.54%; H, 7.74%.

Proton nuclear magnetic resonance spectra were measured and showed a 27H peak based on a methyl group, a butyleneoxy group, and an ethyl group around δ: 0.5 to 3.0 ppm, a 15H peak based on a methoxy group, a butyleneoxy group, and a methylenedioxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 31)

### First step

A photochromic compound represented by Formula (73) below was obtained at a yield of 35% in the same manner, except that 3-(dimethylamino)propyl-4-methylbenzenesulfonate was used for the reaction instead of 1-bromo-4-(ethoxymethoxy)butane in Fourth step of Example 30.

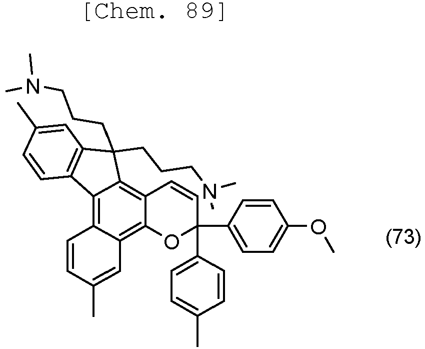

Elemental analysis values for the photochromic compound represented by Formula (73) were determined to be C, 83.05%; H, 7.91%; N, 4.22%, which are in good agreement with the calculated values for C₄₆H₅₂N₂O₂ of C, 83.09%; H, 7.88%; N, 4.21%.

Proton nuclear magnetic resonance spectra were measured and showed a 33H peak based on a methyl group and a dimethylaminopropylene group around δ: 0.5 to 3.0 ppm, a 3H peak based on a methoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 32)

### First step

A photochromic compound represented by Formula (74) below was obtained at a yield of 87% in the same manner, except that the propargyl alcohol of Formula (30) was used for the reaction instead of the propargyl alcohol of Formula (25) in Third step of Example 1.

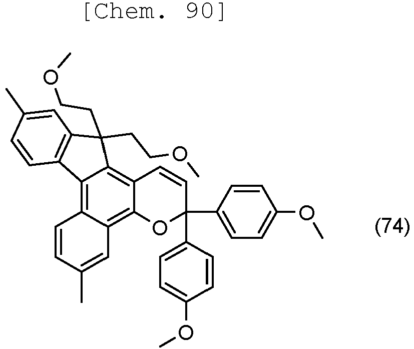

Elemental analysis values for the photochromic compound represented by Formula (74) were determined to be C, 80.52%; H, 6.73%, which are in good agreement with the calculated values for C₄₂H₄₂O₅ of C, 80.48%; H, 6.75%.

Proton nuclear magnetic resonance spectra were measured and showed a 10H peak based on a methyl group and an ethyloxy group around δ: 0.5 to 3.0 ppm, a 16H peak based on a methoxy group and an ethyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 33)

### First step

A naphthol derivative represented by Formula (75) below was obtained at a yield of 32% in the same manner, except that 4-methyl-4'-methoxybenzophenone was used for the reaction instead of 4,4'-dimethylbenzophenone in First step of Example 30.

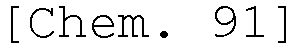

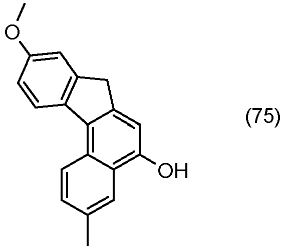

### Second step

A photochromic compound represented by Formula (76) below was obtained at a yield of 83% in the same manner, except that the propargyl alcohol of Formula (30) was used for the reaction instead of the propargyl alcohol of Formula (25) in Third step of Example 30.

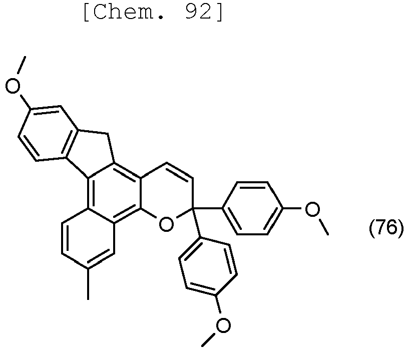

### Third step

A photochromic compound represented by Formula (77) below was obtained at a yield of 81% in the same manner, except that the photochromic compound represented by Formula (76) was used instead of the compound of Formula (23) in Second step of Example 1.

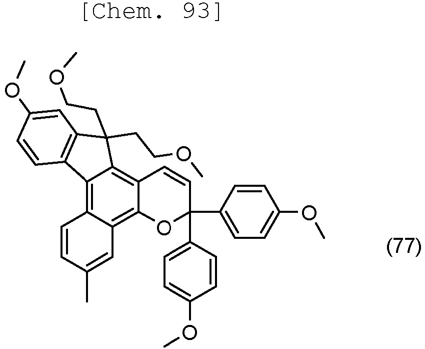

Elemental analysis values for the photochromic compound represented by Formula (77) were determined to be C, 78.52%; H, 6.62%, which are in good agreement with the calculated values for C₄₂H₄₂O₆ of C, 78.48%; H, 6.59%.

Proton nuclear magnetic resonance spectra were measured and showed a 7H peak based on a methyl group and an ethyloxy group around δ: 0.5 to 3.0 ppm, a 19H peak based on a methoxy group and an ethyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 34)

A photochromic compound represented by Formula (79) below was obtained at a yield of 76% in the same manner, except that the other isomer of the resulting naphthol derivative as represented by Formula (78) below was used for the reaction in First step of Example 33.

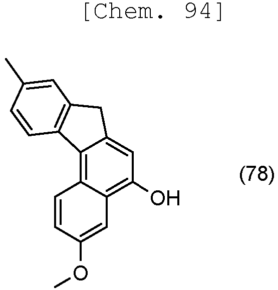

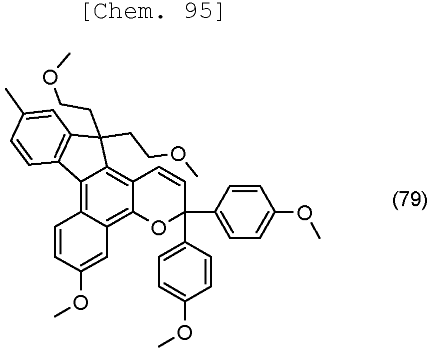

Elemental analysis values for the photochromic compound represented by Formula (79) were determined to be C, 78.46%; H, 6.55%, which are in good agreement with the calculated values for C₄₂H₄₂O₆ of C, 78.48%; H, 6.59%.

Proton nuclear magnetic resonance spectra were measured and showed a 7H peak based on a methyl group and an ethyloxy group around δ: 0.5 to 3.0 ppm, a 19H peak based on a methoxy group and an ethyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 35)

### First step

A photochromic compound represented by Formula (80) below was obtained at a yield of 73% in the same manner, except that 4,4'-dimethoxybenzophenone was used for the reaction instead of 4-methyl-4'-methoxybenzophenone in First step of Example 33.

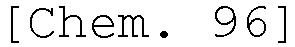

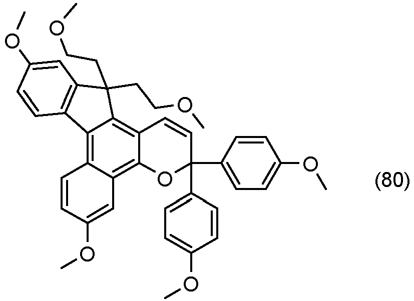

Elemental analysis values for the photochromic compound represented by Formula (80) were determined to be C, 76.60%; H, 6.41%, which are in good agreement with the calculated values for C₄₂H₄₂O₇ of C, 76.57%; H, 6.43%.

Proton nuclear magnetic resonance spectra were measured and showed a 4H peak based on an ethyloxy group around δ: 0.5 to 3.0 ppm, a 22H peak based on a methoxy group and an ethyloxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 36)

### First step

A photochromic compound represented by Formula (82) below was obtained at a yield of 67% in the same manner, except that propargyl alcohol represented by Formula (81) below was used for the reaction instead of the propargyl alcohol of Formula (30) in First step of Example 35.

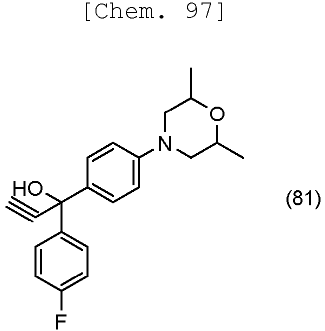

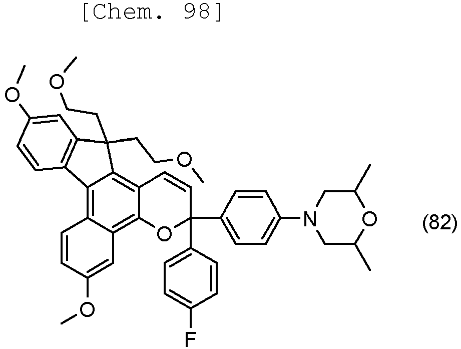

Elemental analysis values for the photochromic compound represented by Formula (82) were determined to be C, 75.64%; H, 6.67%; N, 1.91%, which are in good agreement with the calculated values for C₄₆H₄₈FNO₆ of C, 75.70%; H, 6.63%; N, 1.92%.

Proton nuclear magnetic resonance spectra were measured and showed a 14H peak based on a methyl group, an ethyloxy group, and a morpholino group around δ: 0.5 to 3.0 ppm, an 18H peak based on a methoxy group, an ethyloxy group, and a morpholino group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 37)

### First step

A photochromic compound represented by Formula (84) below was obtained at a yield of 73% in the same manner, except that the compound of Formula (35) was used instead of the carboxylic acid of Formula (68) and propargyl alcohol represented by Formula (83) below was used instead of the propargyl alcohol of Formula (25) in First step of Example 30.

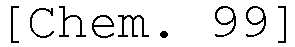

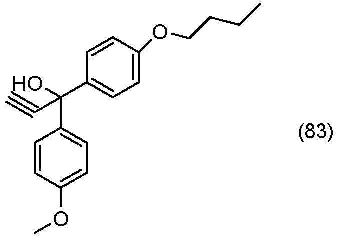

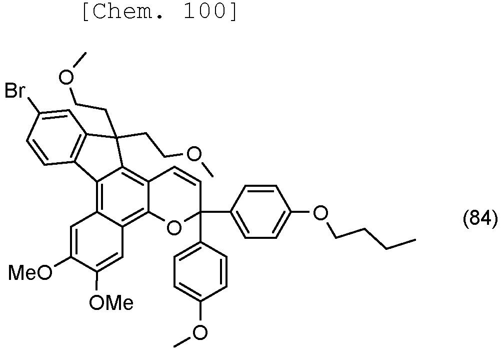

### Second step

A photochromic compound represented by Formula (85) below was obtained at a yield of 89% in the same manner, except that p-methylphenylboronic acid was used for the reaction instead of p-trifluoromethylphenylboronic acid in Third step of Example 4.

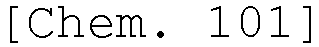

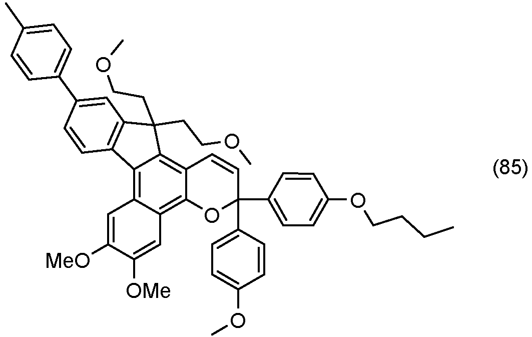

### Third step

A photochromic compound represented by Formula (86) below was obtained at a yield of 51% using the compound represented by Formula (85) below with reference to the method described in

### Patent Document 11.

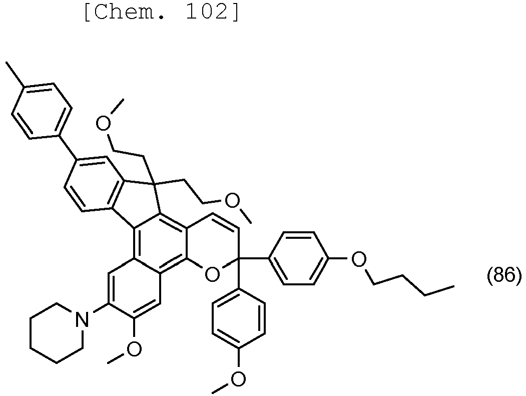

Elemental analysis values for the photochromic compound represented by Formula (86) were determined to be C, 79.71%; H, 7.24%; N, 1.63%, which are in good agreement with the calculated values for C₅₆H₆₁NO₆ of C, 79.68%; H, 7.28%; N, 1.66%.

Proton nuclear magnetic resonance spectra were measured and showed a 24H peak based on a methyl group, an ethyloxy group, a butyloxy group, and a piperidino group around δ: 0.5 to 3.0 ppm, an 18H peak based on a methoxy group, an ethyloxy group, and a butyloxy group around δ: 3.0 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### (Example 38)

### First step

A photochromic compound represented by Formula (88) below was obtained at a yield of 80% in the same manner, except that 3-bromo-4-methoxybenzophenone was used instead of 3,4-dimethoxy-4'-bromobenzophenone and propargyl alcohol represented by Formula (87) below was used instead of the propargyl alcohol of Formula (83) in First step of Example 37.

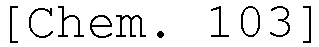

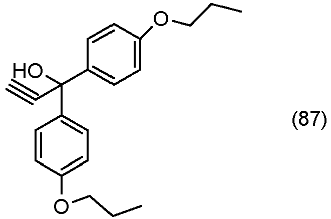

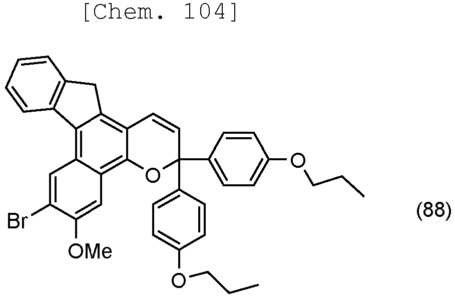

### Second step

A photochromic compound represented by Formula (89) below was obtained at a yield of 77% in the same manner, except that the photochromic compound of Formula (88) was used instead of the compound of Formula (23) and 1-bromo-4-methoxybutane was used instead of 1-bromo-2-methoxyethane in Second step of Example 1.

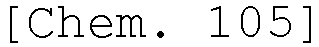

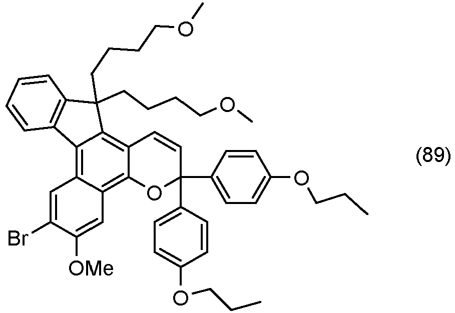

### Third step

A photochromic compound represented by Formula (90) below was obtained at a yield of 71% in the same manner, except that 4-methoxyphenylboronic acid was used for the reaction instead of p-trifluoromethylphenylboronic acid in Third step of Example 4.

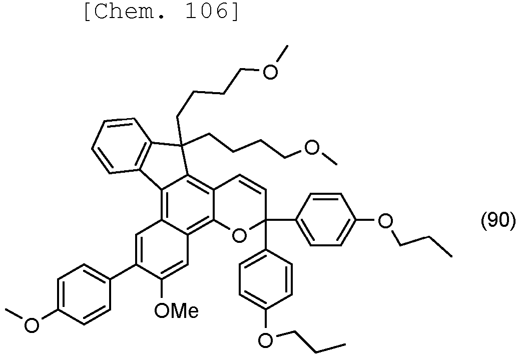

Elemental analysis values for the photochromic compound represented by Formula (90) were determined to be C, 79.42%; H, 7.42%, which are in good agreement with the calculated values for C₅₆H₆₂O₇ of C, 79.40%; H, 7.38%.

Proton nuclear magnetic resonance spectra were measured and showed a 22H peak based on a butyloxy group and a propyloxy group around δ: 0.5 to 3.0 ppm, a 20H peak based on a methoxy group, a propyloxy group, and a butyloxy group around δ: 3.0 to 5.0 ppm, and a 20H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 39

### First step

A photochromic compound represented by Formula (91) below was obtained at a yield of 77% in the same manner, except that the compound of Formula (89) was used instead of the compound of Formula (40) and diphenylamine was used instead of morpholine in First step of Example 10.

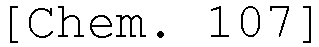

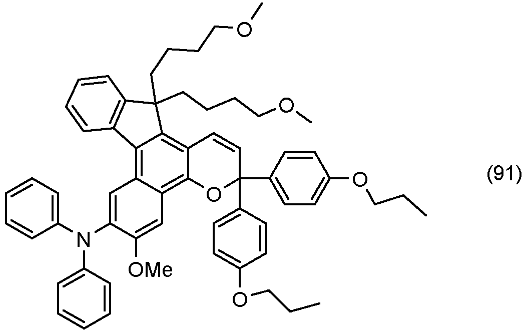

Elemental analysis values for the photochromic compound represented by Formula (91) were determined to be C, 80.62%; H, 7.19%; N, 1.58%, which are in good agreement with the calculated values for C₆₁H₆₅N0₆ of C, 80.67%; H, 7.21%; N, 1.54%.

Proton nuclear magnetic resonance spectra were measured and showed a 22H peak based on a butyloxy group and a propyloxy group around δ: 0.5 to 3.0 ppm, a 17H peak based on a methoxy group, a propyloxy group, and a butyloxy group around δ: 3.0 to 5.0 ppm, and a 26H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 40

### First step

A photochromic compound represented by Formula (93) below was obtained at a yield of 83% in the same manner, except that 3-bromo-4-methoxy-4'-methylbenzophenone was used instead of 3,4-dimethoxy-4'-bromobenzophenone and propargyl alcohol represented by Formula (92) below was used instead of the propargyl alcohol of Formula (83) in First step of Example 37.

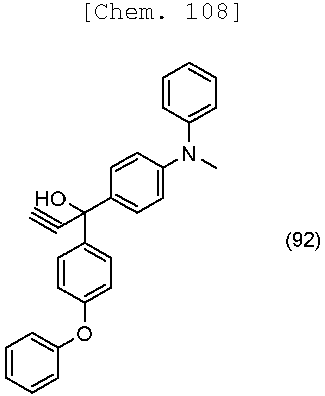

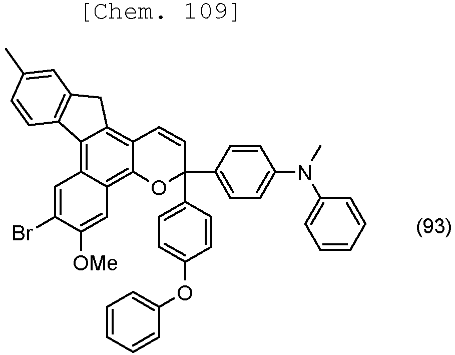

### Second step

A photochromic compound represented by Formula (94) below was obtained at a yield of 82% in the same manner, except that the photochromic compound of Formula (93) was used instead of the compound of Formula (23) and 1-bromo-3-methoxypropane was used instead of 1-bromo-2-methoxyethane in Second step of Example 1.

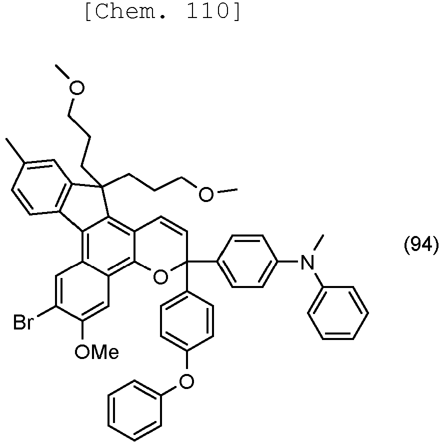

### Third step

A photochromic compound represented by Formula (95) below was obtained at a yield of 77% in the same manner, except that 4-morpholinophenylboronic acid was used for the reaction instead of p-trifluoromethylphenylboronic acid in Third step of Example 4.

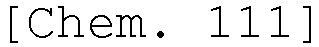

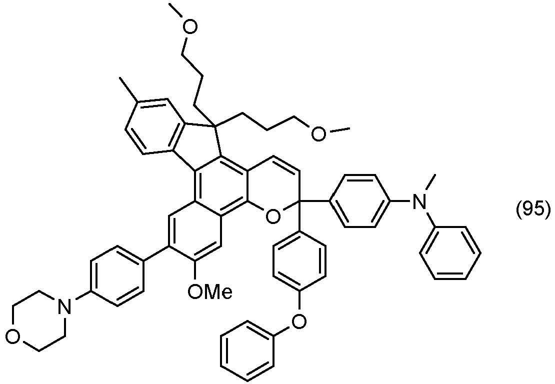

Elemental analysis values for the photochromic compound represented by Formula (95) were determined to be C, 80.51%; H, 6.70%; N, 2.87%, which are in good agreement with the calculated values for C₆₅H₆₄N₂O₆ of C, 80.55%; H, 6.66%; N, 2.89%.

Proton nuclear magnetic resonance spectra were measured and showed a 14H peak based on a propyloxy group, a methylamino group, and a methyl group around δ: 0.5 to 3.0 ppm, a 21H peak based on a methoxy group, a propyloxy group, and a morpholino group around δ: 3.0 to 5.0 ppm, and a 29H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 41

### First step

A photochromic compound represented by Formula (97) below was obtained at a yield of 72% in the same manner, except that propargyl alcohol represented by Formula (96) below was used for the reaction instead of the propargyl alcohol of Formula (87) in First step of Example 37.

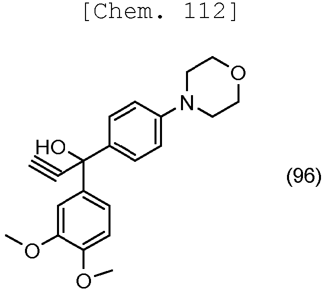

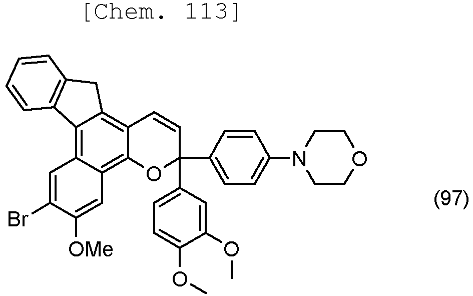

### Second step

A photochromic compound represented by Formula (98) below was obtained at a yield of 86% in the same manner, except that the photochromic compound of Formula (97) was used instead of the compound of Formula (23) and 1-bromo-3-methoxypropane was used instead of 1-bromo-2-methoxyethane in Second step of Example 1.

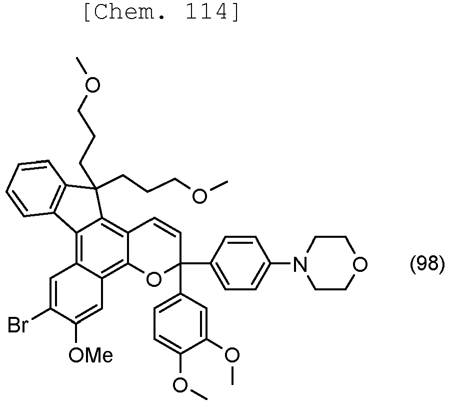

### Third step

A photochromic compound represented by Formula (99) below was obtained at a yield of 65% in the same manner, except that 4-piperidinophenylboronic acid was used for the reaction instead of p-trifluoromethylphenylboronic acid in Third step of Example 4.

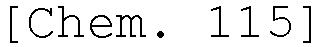

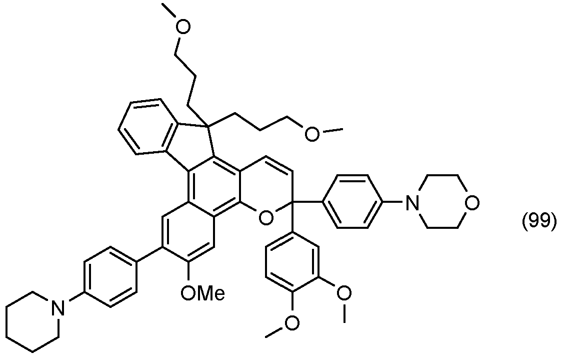

Elemental analysis values for the photochromic compound represented by Formula (99) were determined to be C, 77.33%; H, 7.18%; N, 3.08%, which are in good agreement with the calculated values for C₅₈H₆₄N₂O₇ of C, 77.30%; H, 7.16%; N, 3.11%.

Proton nuclear magnetic resonance spectra were measured and showed a 14H peak based on a propyloxy group and a piperidino group around δ: 0.5 to 3.0 ppm, a 31H peak based on a methoxy group, a propyloxy group, a morpholino group, and a piperidino group around δ: 3.0 to 5.0 ppm, and a 19H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 42

### First step

A photochromic compound represented by Formula (100) below was obtained at a yield of 69% in the same manner, except that the photochromic compound of Formula (71) was used instead of the compound of Formula (23) and 1-bromo-6-methoxyhexane was used instead of 1-bromo-2-methoxyethane in Second step of Example 1.

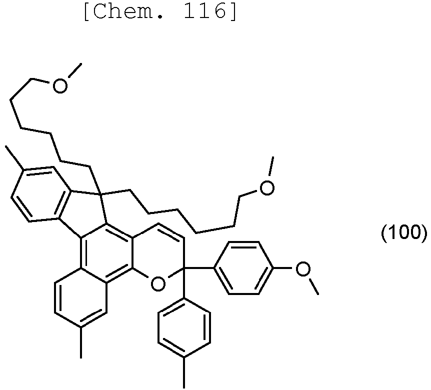

Elemental analysis values for the photochromic compound represented by Formula (100) were determined to be C, 83.02%; H, 8.11%, which are in good agreement with the calculated values for C₅₀H₅₈C₄ of C, 83.06%; H, 8.09%.

Proton nuclear magnetic resonance spectra were measured and showed a 29H peak based on a methyl group and a hexyleneoxy group around δ: 0.5 to 3.0 ppm, a 13H peak based on a methoxy group and a hexyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 43

### First step

A photochromic compound represented by Formula (101) below was obtained at a yield of 62% in the same manner, except that 13-bromo-2,5,8,11-tetraoxatridecane was used instead of 1-bromo-6-methoxyhexane in First step of Example 42.

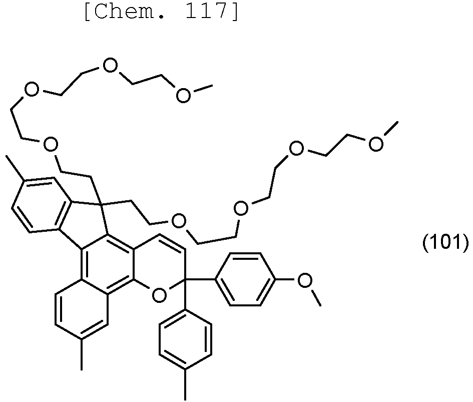

Elemental analysis values for the photochromic compound represented by Formula (101) were determined to be C, 74.07%; H, 7.63%, which are in good agreement with the calculated values for C₅₄H₆₆O₁₀ of C, 74.12%; H, 7.60%.

Proton nuclear magnetic resonance spectra were measured and showed a 13H peak based on a methyl group and an ethyloxy group around δ: 0.5 to 3.0 ppm, a 37H peak based on a methoxy group and an ethyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

### Example 44

### First step

A photochromic compound represented by Formula (102) below was obtained at a yield of 54% in the same manner, except that instead of 25-bromo-2,5,8,11,14,17,20,23-octaoxapentacosane was used instead of 1-bromo-6-methoxyhexane in First step of Example 42.

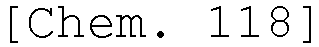

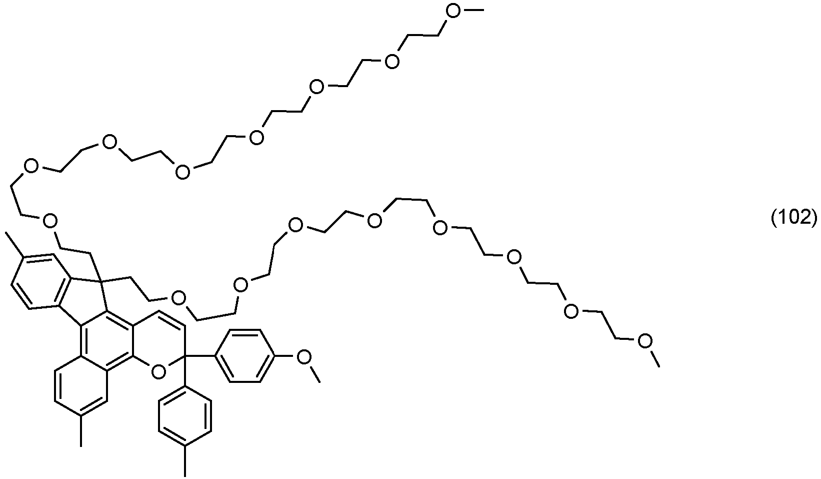

Elemental analysis values for the photochromic compound represented by Formula (102) were determined to be C, 68.46%; H, 8.09%, which are in good agreement with the calculated values for C₇₀H₉₈O₁₈ of C, 68.49%; H, 8.05%.

Proton nuclear magnetic resonance spectra were measured and showed a 13H peak based on a methyl group and an ethyloxy group around δ: 0.5 to 3.0 ppm, a 69H peak based on a methoxy group and an ethyleneoxy group around δ: 3.0 to 5.0 ppm, and a 16H peak based on an aromatic proton and an alkene proton around δ: 5.0 to 9.0 ppm.

Furthermore, ¹³C-nuclear magnetic resonance spectra were measured and showed a peak based on a carbon atom in an aromatic ring around δ: 110 to 160 ppm, a peak based on a carbon atom in an alkene around δ: 80 to 140 ppm, and a peak based on a carbon atom in an alkyl between δ: 20 to 60 ppm.

Examples 45 to 67 Photochromic laminates were produced using the photochromic compounds obtained in Examples 23 to 44 in the same manner as in Example 12.

Evaluation results are summarized in Tables 4 and 5.

### [Table 4]

**Table 4**

| | Compound No. | Maximum absorption wavelength (nm) | Color development density at 23°C(-) | Color development density at 35°C(-) | Rate of temperature change (%) | Color fading half-life at 23°C (sec) | Residual ratio (%) |
|---|---|---|---|---|---|---|---|
| Example 45 | Formula (60) | 435 | 0.62 | 0.43 | 69 | 100 | 79 |
| | | 558 | 1.05 | 0.73 | 69 | 100 | 79 |
| Example 46 | Formula (61) | 435 | 0.65 | 0.46 | 70 | 113 | 80 |
| | | 555 | 1.10 | 0.77 | 71 | 113 | 80 |
| Example 47 | Formula (62) | 434 | 0.60 | 0.41 | 68 | 85 | 78 |
| | | 558 | 1.02 | 0.70 | 68 | 85 | 79 |
| Example 48 | Formula (63) | 433 | 0.64 | 0.43 | 67 | 80 | 78 |
| | | 556 | 1.10 | 0.74 | 67 | 80 | 79 |
| Example 49 | Formula (64) | 434 | 0 . 61 | 0.41 | 67 | 80 | 80 |
| | | 555 | 1.06 | 0.71 | 67 | 81 | 80 |
| Example 50 | Formula (65) | 434 | 0.57 | 0.38 | 67 | 80 | 74 |
| | | 553 | 0 . 97 | 0.65 | 67 | 81 | 74 |
| Example 51 | Formula (66) | 435 | 0.63 | 0.44 | 69 | 113 | 70 |
| | | 557 | 1.06 | 0.74 | 69 | 113 | 70 |
| Example 52 | Formula (67) | 435 | 0.64 | 0.44 | 69 | 98 | 77 |
| | | 556 | 1.09 | 0.75 | 69 | 98 | 77 |
| Example 53 | Formula (72 | 434 | 0.77 | 0.55 | 72 | 128 | 76 |
| | | 556 | 1.28 | 0.92 | 72 | 128 | 77 |
| Example 54 | Formula (73) | 434 | 0.63 | 0.41 | 66 | 84 | 73 |
| | | 555 | 1.08 | 0.71 | 66 | 84 | 72 |
| Example 55 | Formula (74) | 438 | 0.50 | 0.29 | 59 | 40 | 89 |
| | | 563 | 0.85 | 0.50 | 59 | 40 | 89 |
| Example 56 | Formula (77) | 440 | 0.50 | 0.33 | 65 | 47 | 87 |
| | | 573 | 0.95 | 0.62 | 65 | 47 | 87 |

### [Table 5]

**Table 5**

| | Compound No. | Maximum absorption wavelength (nm) | Color development density at 23°C(-) | Color development density at 35°C(-) | Rate of temperature change (%) | Color fading half-life at 23°C (sec) | Residual ratio (%) |
|---|---|---|---|---|---|---|---|
| Example 57 | Formula (79) | 426 | 0.59 | 0.38 | 65 | 47 | 87 |
| | | 580 | 0.80 | 0.53 | 65 | 47 | 87 |
| Example 58 | Formula (80) | 430 | 0.60 | 0.40 | 66 | 62 | 84 |
| | | 595 | 0.87 | 0.58 | 67 | 63 | 84 |
| Example 59 | Formula (82) | 480 | 0.61 | 0.41 | 67 | 73 | 70 |
| | | 604 | 1.12 | 0.76 | 68 | 72 | 70 |
| Example 60 | Formula (86) | 484 | 1.49 | 1.02 | 69 | 85 | 80 |
| Example 61 | Formula (90) | 451 | 1. 10 | 0.65 | 67 | 90 | 81 |
| | | 575 | 0.85 | 0.54 | 68 | 90 | 81 |
| Example 62 | Formula (91) | 502 | 1.59 | 1.07 | 67 | 72 | 83 |
| Example 63 | Formula (95) | 502 | 1.16 | 0.82 | 70 | 101 | 73 |
| | | 604 | 1.26 | 0.89 | 70 | 101 | 73 |
| Example 64 | Formula (99) | 4 98 | 1.26 | 0.88 | 70 | 118 | 75 |
| | | 597 | 1.24 | 0.86 | 70 | 118 | 75 |
| Example 65 | Formula (100) | 433 | 0.70 | 0.48 | 70 | 133 | 78 |
| | | 560 | 1.19 | 0.83 | 70 | 133 | 78 |
| Example 66 | Formula (101) | 434 | 0.61 | 0.40 | 66 | 75 | 79 |
| | | 555 | 1.04 | 0.69 | 67 | 75 | 79 |
| Example 67 | Formula (102) | 434 | 0.57 | 0.39 | 67 | 74 | 74 |
| | | 556 | 0.98 | 0.66 | 67 | 74 | 74 |

### Example 68

### (Production of Photochromic layer (photochromic adhesive layer))

A photochromic layer was produced as follows.

(Production of terminal non-reactive urethane urea resin for photochromic layer forming composition) First, 252 parts by mass of polycarbonate diol with a number average molecular weight of 800, 100 parts by mass of isophorone diisocyanate, and 72 parts by mass of toluene were charged into a 2L 4-necked flask equipped with a stirring blade, a condense tube, a thermometer, and a nitrogen gas inlet tube and reacted under a nitrogen atmosphere at 100°C for 7 hours. Thus, a urethane prepolymer with a terminal isocyanate group was synthesized. After the reaction of the above-mentioned urethane prepolymer was completed, the resulting reaction liquid was cooled to about 0°C and dissolved into 205 parts by mass of isopropyl alcohol and 382 parts by mass of diethyl ketone, and then a temperature of the resulting liquid was kept at 0°C. Next, a mixed solution of 23 parts by mass of bis-(4-aminocyclohexyl)methane serving as a chain extender and 20 parts by mass of diethyl ketone was added dropwise thereto within 30 minutes and reacted at 0°C for 1 hour. Then, 5.7 parts by mass of 1,2,2,6,6-pentamethyl-4-aminopiperidine was added dropwise thereto and reacted at 0°C for 1 hour to obtain a solution of a terminal non-reactive urethane urea resin in diethyl ketone.

One hundred parts by mass of the resulting solution of a terminal non-reactive urethane urea resin, the photochromic compound of Formula (26) (0.27 mmol), 4 parts by mass of an isomer mixture of 4,4'-methylenebis(cyclohexylisocyanate) (polyisocyanate compound), and 0.4 parts by mass of ethylenebis(oxyethylene) bis[3-(5-tert-butyl-4-hydroxy-m-tolyl) propionate] serving as an antioxidant and 0.06 parts by mass of DOW CORNING TORAY L-7001 serving as a surfactant were added and stirred and mixed at room temperature to obtain a photochromic layer forming composition.

### (Synthesis of adhesive for adhesive layer and terminal non-reactive urethane urea resin)

A 5 L separable flask (4-necked) equipped with a stirring blade, a condense tube, a thermometer, and a nitrogen gas inlet tube was prepared and 400 parts by mass of polycarbonate diol with a number average molecular weight of 1000, 175 parts by mass of isophorone diisocyanate, and 120 parts by mass of toluene were charged into the flask and reacted under a nitrogen atmosphere at 110°C for 7 hours. Thus, a urethane prepolymer with a terminal isocyanate group was synthesized. After the reaction of the urethane prepolymer is completed, the resulting reaction liquid is cooled to about 20°C and dissolved into 2500 parts by mass of propylene glycol monomethyl ether, and then a temperature of the resulting liquid was kept at 20°C. Next, 60 parts by mass of isophoronediamine serving as a chain extender was added dropwise thereto and reacted at 20°C for 1 hour. Then, 3 parts by mass of n-butylamine was added dropwise thereto and reacted at 20°C for 1 hour to obtain a solution of a terminal non-reactive urethane urea resin in propylene glycol-monomethyl ether.

Then, 0.2 parts by mass of DOW CORNING TORAY L-7001 serving as a surfactant was added to 500 parts by mass of the resulting solution of a terminal non-reactive urethane urea resin and the resultant was stirred and mixed at room temperature to obtain an adhesive for an adhesive layer.

### (Production of photochromic layer)

Polycarbonate sheets each having a thickness of 400 um (first and second optical sheets; one would be an optical substrate and the other would be a layer without a photochromic compound) were coated with the adhesive for an adhesive layer using a coater (manufactured by TESTER SANGYO CO,. LTD.) at a coating rate of 0.5 m/min and dried at a drying temperature of 110°C for 3 minutes to obtain polycarbonate sheets with adhesive resin layers each having a thickness of 5 um.

Then, an OPP film (oriented polypropylene film) having a thickness of 50 um using a coater (manufactured by TESTER SANGYO CO., LTD.), was coated with the photochromic layer forming composition at a coating rate of 0.3 m/min and dried at a drying temperature of 100°C for 5 minutes. Thus, a photochromic layer was formed. Then, the photochromic layer (thickness: 40um) was placed onto the adhesive resin layer of the first optical sheet with the adhesive resin layer and laminated together.

Furthermore, a structure formed by releasing the OPP film from the thus-prepared one consisting of the first optical sheet / the adhesive resin layer / the photochromic layer / the OPP film laminated in this order was adhered to the polycarbonate sheet (second optical sheet) with the adhesive resin layer so that the photochromic layer and the adhesive resin layer on the polycarbonate sheet (second optical sheet) were bonded together. The resulting laminate was then left to stand at 40°C under vacuum for 24 hours, subjected to heat treatment at 110°C for 60 minutes and humidification treatment at 60°C and 100% RH for 24 hours, and finally left to stand at 40°C under vacuum for 24 hours to obtain a photochromic laminate. The resulting photochromic laminate was evaluated in the same manner as for Example 12.

### Examples 69 to 70

Photochromic laminates were produced using the photochromic compounds of Formula (46) and Formula (90) in the same manner as in Example 68 and evaluated in the same manner as for Example 12. The results are presented in Table 6.

### [Table 6]

**Table 6**

| | Compound No. | Maximum absorption wavelength (nm) | Color development density at 23°C(-) | Color development density at 35°C(-) | Rate of temperature change (%) | Color fading half-life at 23°C (sec) | Residual ratio (%) |
|---|---|---|---|---|---|---|---|
| Example 68 | Formula (26) | 436 | 0.57 | 0.37 | 65 | 77 | 93 |
| | | 555 | 0.92 | 0.60 | 65 | 77 | 93 |
| Example 69 | Formula (46) | 486 | 0.70 | 0.40 | 57 | 37 | 96 |
| | | 596 | 0.70 | 0.40 | 57 | 37 | 96 |
| Example 70 | Formula (90) | 451 | 1.14 | 0.78 | 69 | 110 | 94 |
| | | 575 | 0.88 | 0.61 | 69 | 110 | 94 |

Preferred embodiments of the present invention will be described in addition.
[1] A photochromic compound having a backbone represented by Formula (1) below: 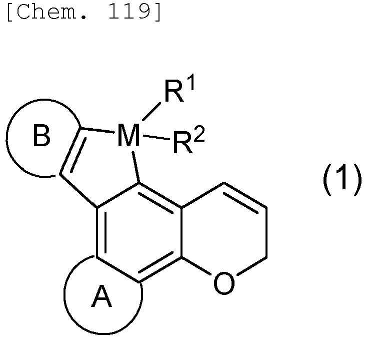 in which
   M is C, Si, or Ge;
   R¹ is a group represented by Formula (2) below;

      -Q¹-(X¹Q²)a-X²Q³ (2)
   R² is a group represented by Formula (2) above or an alkyl group optionally including a halogen atom as a substituent;
   Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings, or Ring A may not be present;
   Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
   in the Formula (2) above,
   Q¹ is an alkylene group optionally including a halogen atom as a substituent;
   Q² is an alkylene group optionally including a halogen atom as a substituent;
   Q³ is an alkyl group optionally including a halogen atom as a substituent;
   X¹ and X² are each independently O, S, NR⁷⁰⁰, PR⁷⁰¹, or P(=O);
   R⁷⁰⁰ is a hydrogen atom, an optionally substituted alkyl group, an optionally substituted cycloalkyl group, an optionally substituted aryl group, or an optionally substituted heteroaryl group;
   R⁷⁰¹ is a hydrogen atom, an optionally substituted alkyl group, an optionally substituted cycloalkyl group, an optionally substituted aryl group, or an optionally substituted heteroaryl group; and
   a is 0, or 1 or more and 10 or less.
[2] The photochromic compound according to [1], having a backbone represented by Formula (3) below: 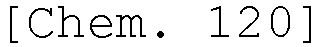 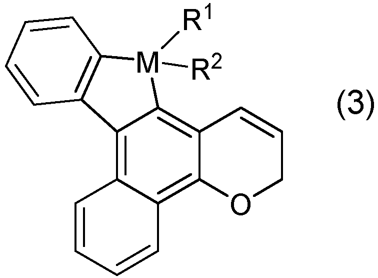 in which R¹, R², and M are each the same as in Formula (1).
[3] The photochromic compound according to [1] or [2], represented by Formula (4) below: 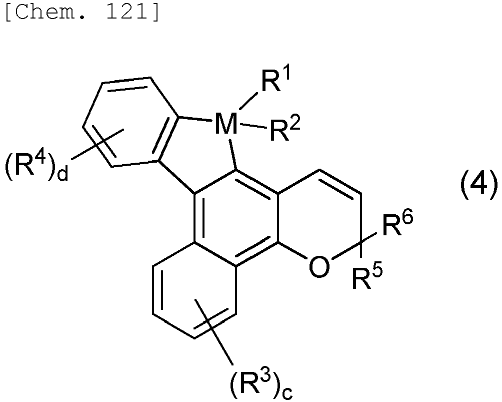
   in which R¹, R², and M are each the same as in Formula (1);
   R³ and R⁴ are each independently a group represented by Formula (2), a hydroxyl group, an alkyl group, a haloalkyl group, an optionally substituted cycloalkyl group, an alkoxy group, an amino group, a substituted amino group, an optionally substituted heterocyclic group, a cyano group, a halogen atom, an alkylthio group, an optionally substituted arylthio group,
   a nitro group, a formyl group, a hydroxycarbonyl group, an alkylcarbonyl group, an alkoxycarbonyl group, an optionally substituted aralkyl group, an optionally substituted aralkoxy group, an optionally substituted aryloxy group, an optionally substituted aryl group, an optionally substituted heteroaryl group, a thiol group, an alkoxyalkylthio group, a haloalkylthio group, an optionally substituted cycloalkylthio group, an optionally substituted silyl group, an optionally substituted oxysilyl group, a group represented by Formula (X) below, or a group represented by L¹-R⁴⁰⁰ below;
   c is an integer of 0 to 4; d is an integer of 0 to 4;
   when c is 2 to 4, a plurality of R³s may be the same as or different from each other;
   when d is 2 to 4, a plurality of R⁴s may be the same as or different from each other;
   when c is 2 to 4 and adjacent R³s are present, two adjacent R³s may be taken together with a carbon atom to which R³s are attached to form a ring optionally including at least one atom selected from the group consisting of an oxygen atom, a carbon atom, a sulfur atom, and a nitrogen atom and the ring is optionally substituted;
   when d is 2 to 4 and adjacent R⁴s are present, two adjacent R⁴s may be taken together with a carbon atom to which R⁴s are attached to form a ring optionally including at least one atom selected from the group consisting of an oxygen atom, a carbon atom, a sulfur atom, and a nitrogen atom and the ring is optionally substituted; and
   R⁵ and R⁶ are each independently an optionally substituted aryl group or an optionally substituted heteroaryl group; 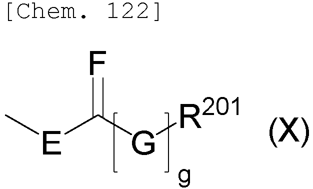
   in Formula (X),
   E is an oxygen atom or NR¹⁰¹ in which R¹⁰¹ is a hydrogen atom or an alkyl group;
   F is an oxygen atom or a sulfur atom;
   G is an oxygen atom, a sulfur atom, or NR²⁰² in which R²⁰² is a hydrogen atom, an alkyl group, a cycloalkyl group, an aryl group, or a heteroaryl group;
   g is 0 or 1;
   R²⁰¹ is a hydrogen atom, an alkyl group, a cycloalkyl group, an aryl group, or a heteroaryl group; and
   when G is an oxygen atom or a sulfur atom, R²⁰¹ is a group other than a hydrogen atom;
   in L¹-R⁴⁰⁰,
   R⁴⁰⁰ is a hydrogen atom, an alkyl group, an aryl group, a polymerizable group, a photochromic group, or a silyl group having an alkyl group, an alkoxyl group, or an aryl group as a substituent; and
   L¹ is a group represented by Formula (X2) below; 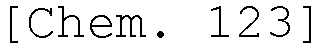 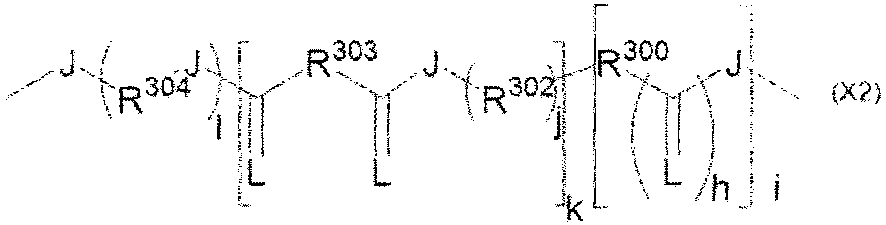
   in Formula (X2),
   J is a divalent group, each independently being directly attached, a substituted methylene group, an oxygen atom, a sulfur atom, or NR³⁰¹ in which R³⁰¹ is a hydrogen atom or an alkyl group;
   L is an oxygen atom or a sulfur atom;
   R³⁰⁰ is an alkylene group, or a silylene group having an alkyl group or an aryl group as a substituent;
   R³⁰², R³⁰³, and R³⁰⁴ are each independently an alkylene group;
   h, j, k, and l are each independently an integer of 0 or 1;
   i is an integer of 1 to 200, and a plurality of units i may be the same as or different from each other; and
   a dashed line represents an attachment to R⁴⁰⁰.
[4] The photochromic compound according to any one of [1] to [3], in which Q¹ is an alkylene group having 2 or more and 6 or less carbon atoms and optionally including a halogen atom as a substituent.
[5] The photochromic compound according to any one of [1] to [4], in which Q² is an alkylene group having 1 or more and 6 or less carbon atoms and optionally including a halogen atom as a substituent.
[6] The photochromic compound according to any one of [1] to [5], in which R² is a group represented by Formula (2).
[7] The photochromic compound according to any one of [1] to [3], in which R¹ and R² are each independently a group represented by Formula (2a) below:

   -Q¹¹-OQ¹³ (2a)

   in which
   Q¹¹ is a linear alkylene group having 1 or more and 10 or less carbon atoms; and
   Q¹³ is a linear alkyl group having 1 or more and 15 or less carbon atoms.
[8] The photochromic compound according to any one of [1] to [3], in which R¹ and R² are each independently a group represented by Formula (2b) below:

   -Q²¹-(OCH₂CH₂)ₐ₁ -OQ²³ (2b)

   in which
   Q²¹ is a linear alkylene group having 1 or more and 5 or less carbon atoms;
   Q²³ is a linear alkyl group having 1 or more and 5 or less carbon atoms; and
   a1 is 1 or more and 10 or less.
[9] The photochromic compound according to [3], represented by Formula (5) below: 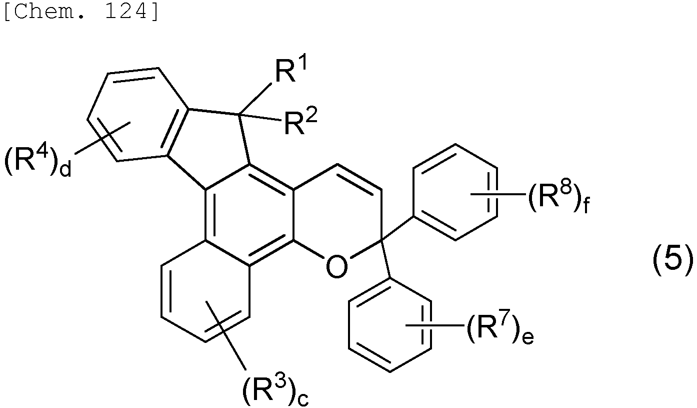 in which
   R¹, R², R3, R⁴, c, and d are each independently the same as in Formula (4);
   R⁷ and R⁸ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, an optionally substituted arylthio group having 6 to 10 carbon atoms, or the group represented by L¹-R⁴⁰⁰ ;
   e is an integer of 0 to 5;
   when e is 2 to 5, R⁷s may be the same as or different from each other;
   when adjacent R⁷s are present, two adjacent R⁷s may be taken together with a carbon atom to which R⁷s are attached to form a ring optionally including at least one atom selected from the group consisting of an oxygen atom, a sulfur atom, a carbon atom, and a nitrogen atom and the ring is optionally substituted;
   f is an integer of 0 to 5;
   when f is 2 to 5, R⁸s may be the same as or different from each other; and
   when adjacent R⁸s are present, two adjacent R⁸s may be taken together with a carbon atom to which R⁸s are attached to form a ring optionally including at least one atom selected from the group consisting of an oxygen atom, a sulfur atom, a carbon atom, and a nitrogen atom and the ring is optionally substituted.
[10] A curable composition including:
   the photochromic compound according to any one of [1] to [9]; and
   at least one selected from the group consisting of a radical polymerizable monomer, a cationic polymerizable monomer, a compound having a polymerization reactive group, and a (thio)urethane(urea) polymer.
[11] An optical article including a cured product of the curable composition according to [10].
[12] A lens including the photochromic compound according to any one of [1] to [9].
[13] Eyeglasses including the lens according to [10].
[14] A naphthol derivative having a backbone represented by Formula (6a) below: 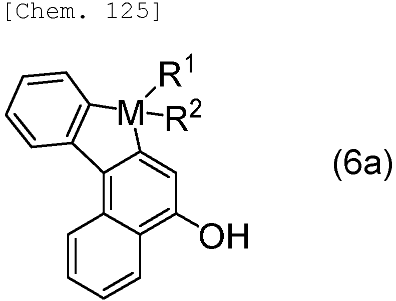 in which
   M is C, Si, or Ge;
   R¹ is a group represented by Formula (2) below;

      -Q¹-(X¹Q²)a-X²Q³ (2)
   R² is a group represented by Formula (2) above or an alkyl group optionally including a halogen atom as a substituent;
   in the Formula (2) above,
   Q¹ is an alkylene group optionally including a halogen atom as a substituent;
   Q² is an alkylene group optionally including a halogen atom as a substituent;
   Q³ is an alkyl group optionally including a halogen atom as a substituent;
   X¹ and X² are each independently O, S, NR⁷⁰⁰, PR⁷⁰¹, or P(=O); R⁷⁰⁰ is a hydrogen atom, an optionally substituted alkyl group, an optionally substituted cycloalkyl group, an optionally substituted aryl group, or an optionally substituted heteroaryl group;
   R⁷⁰¹ is a hydrogen atom, an optionally substituted alkyl group, an optionally substituted cycloalkyl group, an optionally substituted aryl group, or an optionally substituted heteroaryl group; and
   a is 0, or 1 or more and 10 or less.

## Claims

1. A photochromic compound having a backbone represented by Formula (1) below: 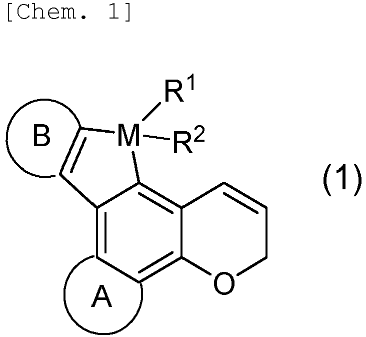 wherein
M is C, Si, or Ge;
R¹ is a group represented by Formula (2) below;
-Q¹- (X¹Q²)a-X²Q³ (2)
R² is a group represented by Formula (2) above or an alkyl group optionally comprising a halogen atom as a substituent;
Ring A is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings, or Ring A may not be present; and
Ring B is a substituted or unsubstituted aromatic hydrocarbon ring, a substituted or unsubstituted aromatic heterocyclic ring, or a substituted or unsubstituted fused polycyclic ring in which an aromatic ring or an aromatic heterocyclic ring is fused to any of the above-mentioned rings;
in the Formula (2) above,
Q¹ is an alkylene group optionally comprising a halogen atom as a substituent;
Q² is an alkylene group optionally comprising a halogen atom as a substituent;
Q³ is an alkyl group optionally comprising a halogen atom as a substituent;
X¹ and X² are each independently O, S, NR⁷⁰⁰, PR⁷⁰¹, or P(=O);
R⁷⁰⁰ is a hydrogen atom, an optionally substituted alkyl group, an optionally substituted cycloalkyl group, an optionally substituted aryl group, or an optionally substituted heteroaryl group;
R⁷⁰¹ is a hydrogen atom, an optionally substituted alkyl group, an optionally substituted cycloalkyl group, an optionally substituted aryl group, or an optionally substituted heteroaryl group; and
a is 0, or 1 or more and 10 or less.

2. The photochromic compound according to claim 1, having
a backbone represented by Formula (3) below: 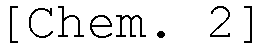 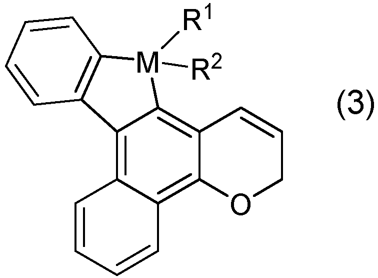
wherein R¹, R², and M are each the same as in Formula (1).

3. The photochromic compound according to claim 1 or 2,
represented by Formula (4) below: 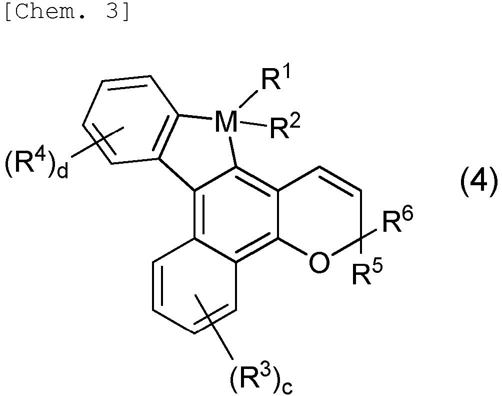
wherein R¹, R², and M are each the same as in Formula (1);
R³ and R⁴ are each independently a group represented by Formula (2), a hydroxyl group, an alkyl group, a haloalkyl group, an optionally substituted cycloalkyl group, an alkoxy group, an amino group, a substituted amino group, an optionally substituted heterocyclic group, a cyano group, a halogen atom, an alkylthio group, an optionally substituted arylthio group, a nitro group, a formyl group, a hydroxycarbonyl group, an alkylcarbonyl group, an alkoxycarbonyl group, an optionally substituted aralkyl group, an optionally substituted aralkoxy group, an optionally substituted aryloxy group, an optionally substituted aryl group, an optionally substituted heteroaryl group, a thiol group, an alkoxyalkylthio group, a haloalkylthio group, an optionally substituted cycloalkylthio group, an optionally substituted silyl group, an optionally substituted oxysilyl group, a group represented by Formula (X) below, or a group represented by L¹-R⁴⁰⁰ below;
c is an integer of 0 to 4; d is an integer of 0 to 4;
when c is 2 to 4, a plurality of R³s may be the same as or different from each other;
when d is 2 to 4, a plurality of R⁴s may be the same as or different from each other;
when c is 2 to 4 and adjacent R³s are present, two adjacent R³s may be taken together with a carbon atom to which R³s are attached to form a ring optionally comprising at least one atom selected from the group consisting of an oxygen atom, a carbon atom, a sulfur atom, and a nitrogen atom and the ring is optionally substituted;
when d is 2 to 4 and adjacent R⁴s are present, two adjacent R⁴s may be taken together with a carbon atom to which R⁴s are attached to form a ring optionally comprising at least one atom selected from the group consisting of an oxygen atom, a carbon atom, a sulfur atom, and a nitrogen atom and the ring is optionally substituted; and
R⁵ and R⁶ are each independently an optionally substituted aryl group or an optionally substituted heteroaryl group; 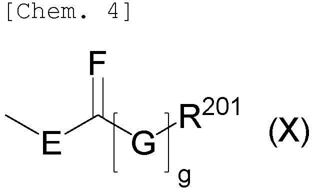
in Formula (X),
E is an oxygen atom or NR¹⁰¹ wherein R¹⁰¹ is a hydrogen atom or an alkyl group;
F is an oxygen atom or a sulfur atom;
G is an oxygen atom, a sulfur atom, or NR²⁰² wherein R²⁰² is a hydrogen atom, an alkyl group, a cycloalkyl group, an aryl group, or a heteroaryl group;
g is 0 or 1;
R²⁰¹ is a hydrogen atom, an alkyl group, a cycloalkyl group, an aryl group, or a heteroaryl group; and
when G is an oxygen atom or a sulfur atom, R²⁰¹ is a group other than a hydrogen atom;
in L¹-R⁴⁰⁰,
R⁴⁰⁰ is a hydrogen atom, an alkyl group, an aryl group, a polymerizable group, a photochromic group, or a silyl group having an alkyl group, an alkoxyl group, or an aryl group as a substituent; and
L¹ is a group represented by Formula (X2) below; 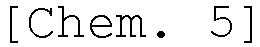 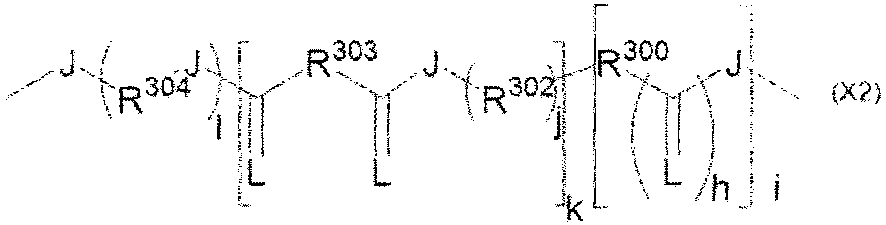
in Formula (X2),
J is a divalent group, each independently being directly attached, a substituted methylene group, an oxygen atom, a sulfur atom, or NR³⁰¹ in which R³⁰¹ is a hydrogen atom or an alkyl group;
L is an oxygen atom or a sulfur atom;
R³⁰⁰ is an alkylene group, or a silylene group having an alkyl group or an aryl group as a substituent;
R³⁰², R³⁰³, and R³⁰⁴ are each independently an alkylene group;
h, j, k, and l are each independently an integer of 0 or 1;
i is an integer of 1 to 200, and a plurality of units i may be the same as or different from each other; and
a dashed line represents a bond to R⁴⁰⁰.

4. The photochromic compound according to claim 1 or 2,
wherein Q¹ is an alkylene group having 2 or more and 6 or less carbon atoms and optionally comprising a halogen atom as a substituent.

5. The photochromic compound according to claim 1 or 2,
wherein Q² is an alkylene group having 1 or more and 6 or less carbon atoms and optionally comprising a halogen atom as a substituent.

6. The photochromic compound according to claim 1 or 2,
wherein R² is a group represented by Formula (2).

7. The photochromic compound according to claim 1 or 2,
wherein R¹ and R² are each independently a group represented by Formula (2a) below:
-Q²¹-OQ¹³ (2a)
wherein
Q¹¹ is a linear alkylene group having 1 or more and 10 or less carbon atoms; and
Q¹³ is a linear alkyl group having 1 or more and 15 or less carbon atoms.

8. The photochromic compound according to claim 1 or 2,
wherein R¹ and R² are each independently a group represented by Formula (2b) below:
-Q²¹-(OCH₂CH₂)ₐ₁-OQ²³ (2b)
wherein
Q²¹ is a linear alkylene group having 1 or more and 5 or less carbon atoms; and
Q²³ is a linear alkyl group having 1 or more and 5 or less carbon atoms; and
a1 is 1 or more and 10 or less.

9. The photochromic compound according to claim 3,
represented by Formula (5) below: 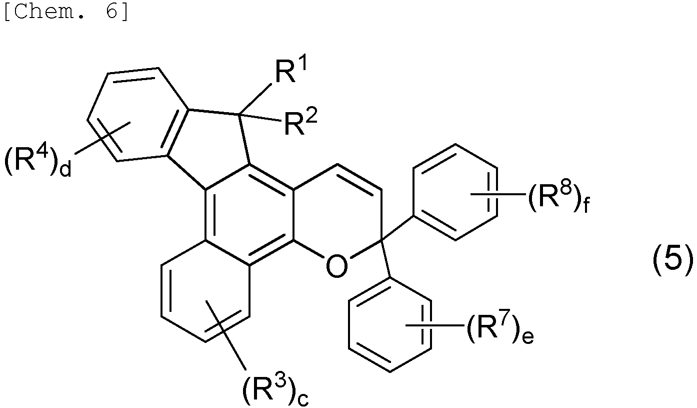
wherein
R¹, R², R³, R⁴, c, and d are each independently the same as in Formula (4);
R⁷ and R⁸ are each independently a hydroxyl group, an alkyl group having 1 to 6 carbon atoms, a haloalkyl group having 1 to 6 carbon atoms, a cycloalkyl group having 3 to 8 carbon atoms, an alkoxy group having 1 to 6 carbon atoms, an amino group, a heterocyclic group, a cyano group, a halogen atom, an alkylthio group having 1 to 6 carbon atoms, an optionally substituted arylthio group having 6 to 10 carbon atoms, or the group represented by L¹-R⁴⁰⁰;
e is an integer of 0 to 5;
when e is 2 to 5, R⁷s may be the same as or different from each other;
when adjacent R⁷s are present, two adjacent R⁷s may be taken together with a carbon atom to which R⁷s are attached to form a ring optionally comprising at least one atom selected from the group consisting of an oxygen atom, a sulfur atom, a carbon atom, and a nitrogen atom and the ring is optionally substituted;
f is an integer of 0 to 5;
when f is 2 to 5, R⁸s may be the same as or different from each other; and
when adjacent R⁸s are present, two adjacent R⁸s may be taken together with a carbon atom to which R⁸s are attached to form a ring optionally comprising at least one atom selected from the group consisting of an oxygen atom, a sulfur atom, a carbon atom, and a nitrogen atom and the ring is optionally substituted.

10. A curable composition comprising:
the photochromic compound according to claim 1 or 2; and
at least one selected from the group consisting of a radical polymerizable monomer, a cationic polymerizable monomer, a compound having a polymerization reactive group, and a (thio)urethane(urea) polymer.

11. An optical article comprising a cured product of the curable composition according to claim 10.

12. A lens comprising the photochromic compound according to claim 1 or 2.

13. Eyeglasses comprising the lens according to claim 12.

14. A naphthol derivative having a backbone represented by Formula (6a) below: 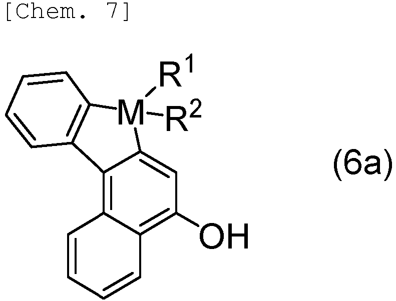 wherein
M is C, Si, or Ge;
R¹ is a group represented by Formula (2) below;
-Q¹-(X¹Q²)a-X²Q³ (2)
R² is a group represented by Formula (2) above or an alkyl group optionally comprising a halogen atom as a substituent;
in the Formula (2) above,
Q¹ is an alkylene group optionally comprising a halogen atom as a substituent;
Q² is an alkylene group optionally comprising a halogen atom as a substituent;
Q³ is an alkyl group optionally comprising a halogen atom as a substituent;
X¹ and X² are each independently O, S, NR⁷⁰⁰, PR⁷⁰¹, or P(=O);
R⁷⁰⁰ is a hydrogen atom, an optionally substituted alkyl group, an optionally substituted cycloalkyl group, an optionally substituted aryl group, or an optionally substituted heteroaryl group;
R⁷⁰¹ is a hydrogen atom, an optionally substituted alkyl group, an optionally substituted cycloalkyl group, an optionally substituted aryl group, or an optionally substituted heteroaryl group; and
a is 0, or 1 or more and 10 or less.
